# EUROPÄISCHE PATENTSCHRIFT

(11) **EP 1 303 505 B1**
(45) Veröffentlichungstag und Bekanntmachung des Hinweises auf die Patenterteilung: **03.09.2008**
(21) Anmeldenummer: 01949391.5
(22) Anmeldetag: 08.06.2001
(51) Int. Cl.: C07D 309/32, C07D 311/96, C07D 311/20, C07D 405/12, C07C 69/66, A01N 43/16

(54) **PHENYLSUBSTITUIERTE 5,6-DIHYDRO-PYRON-DERIVATE ALS PESTIZIDE UND HERBIZIDE**
PHENYL-SUBSTITUTED 5,6-DIHYDROPYRONE DERIVATIVES FOR USE AS PESTICIDES AND HERBICIDES
DERIVES DE 5,6-DIHYDRO-PYRONE SUBSTITUES PAR PHENYLE UTILISES COMME PESTICIDES ET HERBICIDES

(30) Priorität: 19.06.2000 DE 10030094
(43) Veröffentlichungstag der Anmeldung: 23.04.2003
(73) Patentinhaber: Bayer CropScience AG, 40789 Monheim (DE)
(72) Erfinder: FISCHER, Reiner, 40789 Monheim (DE); GRAFF, Alan, 51373 Leverkusen (DE); LIEB, Folker, 51375 Leverkusen (DE); ULLMANN, Astrid, 50677 Köln (DE); TRAUTWEIN, Axel, 51467 Bergisch Gladbach (DE); WISCHNAT, Ralf, 51061 Köln (DE); SCHNEIDER, Udo, 51373 Leverkusen (DE); DREWES, Mark, Wilhelm, 40764 Langenfeld (DE); ERDELEN, Christoph, 42799 Leichlingen (DE); DAHMEN, Peter, 41470 Neuss (DE); FEUCHT, Dieter, 40789 Monheim (DE); PONTZEN, Rolf, 42799 Leichlingen (DE)
(74) Vertreter: Bader, Axel Jochen
(86) Internationale Anmeldenummer: PCT/EP2001/006522
(87) Internationale Veröffentlichungsnummer: WO 2001/098288

(56) Entgegenhaltungen:
- WO-A-95/14012
- DE-A- 2 707 746
- K. LIU, L. XU: "Synthesis of 5,6-dihydro-4-hydroxy-2-pyrones via formal cycloaddition reactions" TETRAHEDRON LETTERS, Bd. 41, 2000, Seiten 3299-3302, XP004198025
- PATENT ABSTRACTS OF JAPAN vol. 018, no. 236, 6. Mai 1994 (1994-05-06) & JP 06 025210 A (SAGAMI CHEM. RESEARCH CENTER), 1. Februar 1994 (1994-02-01)

## Beschreibung

Die vorliegende Erfindung betrifft neue phenylsubstituierte 5,6-Dihydro-pyron-Derivate, mehrere Verfahren zu ihrer Herstellung und ihre Verwendung als Schädlingsbekämpfungsmittel und Herbizide.

Es ist bekannt, dass bestimmte 5,6-Dihydropyron-Derivate als Proteaseinhibitoren antivirale Eigenschaften haben: WO 95/14012. Weiterhin ist das 4-Phenyl-6-(2-phenethyl)-5,6-dihydropyron aus der Synthese von Kawalakton-Derivaten bekannt: Kappe et al.; Arch. Pharm. 309, 558-64, (1976). Außerdem sind 5,6-Dihydropyron-Derivate als Zwischenprodukte bekannt: White, J.D., Brenner, J.B., Deinsdale, M.J., J. Amer. Chem. Soc. 93, 281-2 (1971). Anwendungen im Pflanzenschutz wurden bislang nicht beschrieben.

Es wurden nun neue Verbindungen der Formel (I) gefunden 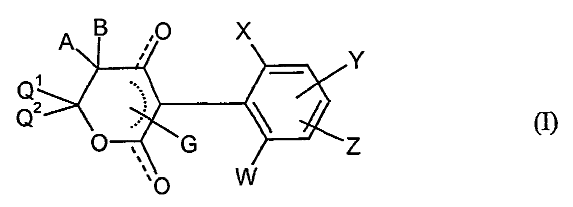 in welcher
- W: für Wasserstoff, Alkyl, Alkenyl, Alkinyl, Halogen, Halogenalkyl oder Alkoxy steht,
- X: für Halogen, Alkyl, Alkoxy, Alkenyl, Alkinyl, Halogenalkyl, Halogenalkoxy, Cyano oder jeweils gegebenenfalls substituiertes Phenyl, Phenoxy, Phenylthio, Phenylalkoxy oder Phenylalkylthio steht,
- Y: für Wasserstoff, Alkyl, Halogen, Halogenalkyl, Alkoxy, Alkenyl, Alkinyl oder gegebenenfalls substituiertes Aryl oder Hetaryl steht,
- Z: für Wasserstoff, Halogen, Alkyl, Alkoxy, Halogenalkyl, Halogenalkoxy oder Cyano steht,
- A: für eine Bindung, Wasserstoff, jeweils gegebenenfalls durch Halogen substituiertes Alkyl, Alkenyl, Alkoxyalkyl, gegebenenfalls substituiertes Cycloalkyl oder Cycloalkylalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl, Hetaryl oder Hetarylalkyl steht,
- B: für Wasserstoff oder Alkyl steht, oder
- A und B: gemeinsam mit dem Kohlenstoffatom, an das sie gebunden sind, für einen gesättigten oder ungesättigten, gegebenenfalls mindestens ein Heteroatom enthaltenden unsubstituierten oder substituierten Cyclus stehen, oder
- B und Q¹: gemeinsam für jeweils gegebenenfalls durch jeweils gegebenenfalls substituiertes Alkyl oder Alkoxy substituiertes Alkandiyl stehen, in welchem zwei nicht direkt benachbarte Kohlenstoffatome gegebenenfalls einen weiteren gegebenenfalls substituierten Cyclus bilden oder
- Q¹: für Wasserstoff, Hydroxy, Alkyl, Alkoxy, Alkoxyalkyl, Alkylacyloxy, gegebenenfalls substituiertes Cycloalkyl (worin gegebenenfalls eine Methylengruppe durch Sauerstoff oder Schwefel ersetzt ist) oder gegebenenfalls substituiertes Phenyl steht,
- Q²: für Wasserstoff oder Alkyl steht, oder
- Q¹ und Q²: gemeinsam mit dem Kohlenstoffatom, an das sie gebunden sind, für einen gegebenenfalls ein Heteroatom enthaltenden unsubstituierten oder substituierten Cyclus stehen,
- G: für Wasserstoff (a) oder für eine der Gruppen 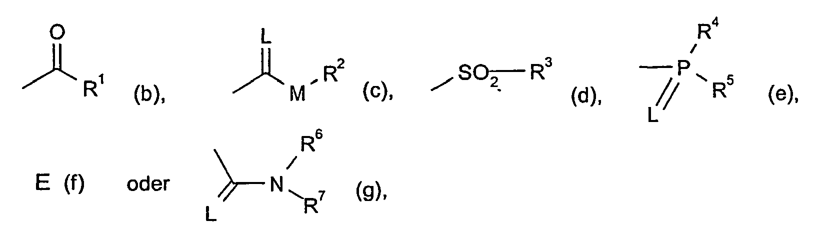 steht,
worin
E für ein Metallion oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, in welchem eine oder mehrere Methylengruppen durch Heteroatome ersetzt sein können, jeweils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio, Cycloalkylthio und für jeweils gegebenenfalls substituiertes Phenyl, Benzyl, Phenoxy oder Phenylthio stehen, und
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenen-falls substituiertes Benzyl stehen, oder gemeinsam mit dem N-Atom, an das sie gebunden sind, für einen gegebenenfalls durch Sauerstoff oder Schwefel unterbrochenen Ring stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sein können.

Die Verbindungen der Formel (I) können in Abhängigkeit von der Stellung des Substituenten G in den zwei isomeren Formen der Formeln (I-A) und (I-B) vorliegen, 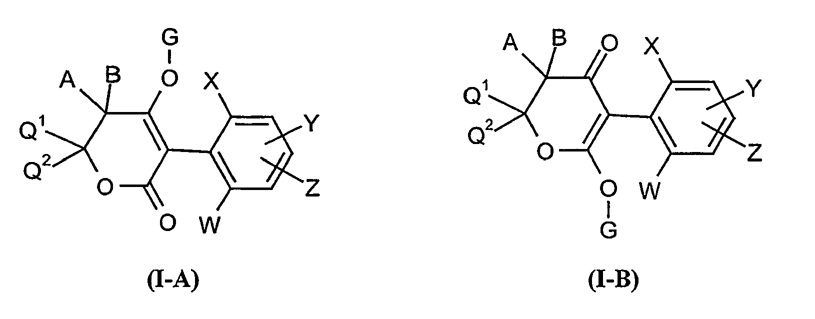 was durch die gestrichelte Linie in der Formel (I) zum Ausdruck gebracht werden soll.

Die Verbindungen der Formeln (I-A) und (I-B) können sowohl als Gemische als auch in Form ihrer reinen Isomeren vorliegen. Gemische der Verbindungen der Formeln (I-A) und (I-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-a) bis (I-g) 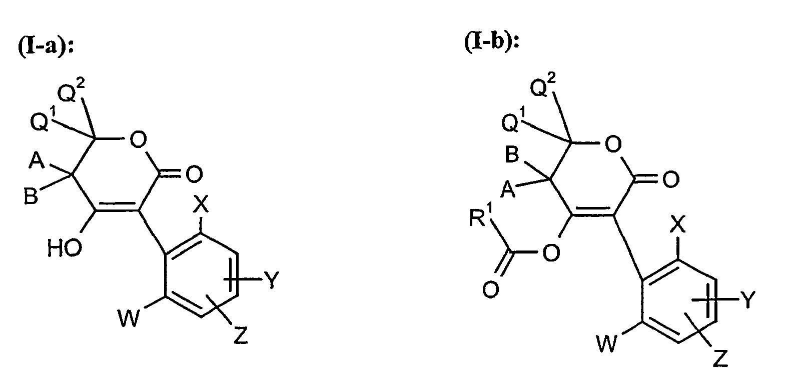 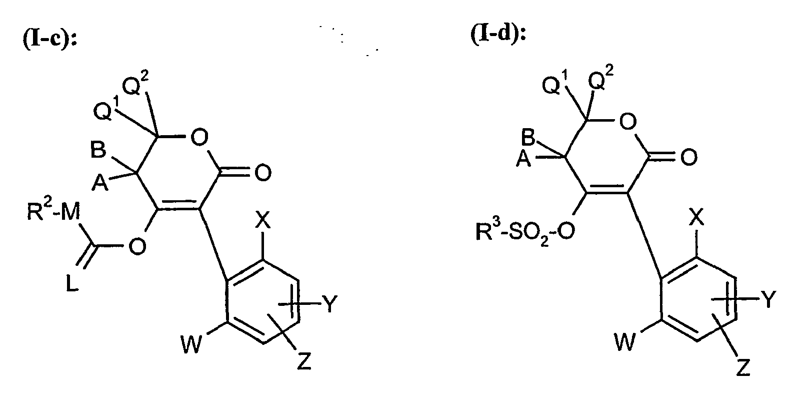 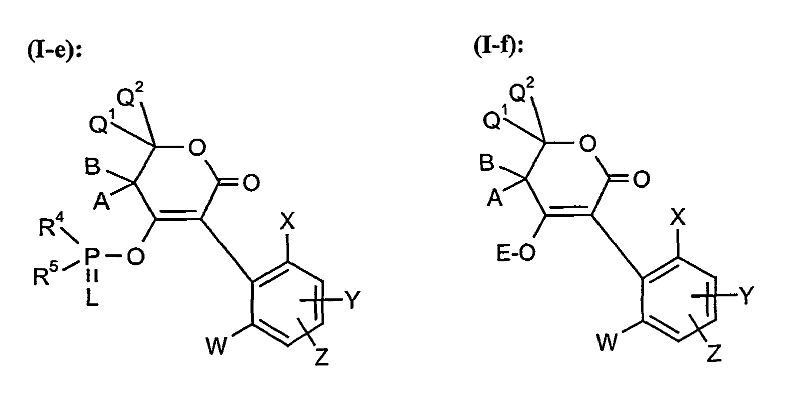 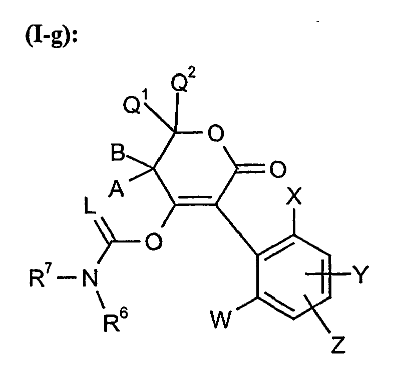 worin
A, B, E, L, M, Q¹, Q², W, X, Y, Z, R¹, R², R³, R⁴, R⁵, R⁶ und R⁷ die oben angegebenen Bedeutungen besitzen.

Weiterhin wurde gefunden, dass man die neuen Verbindungen der Formel (I) nach einem der im folgenden beschriebenen Verfahren erhält:
(A) Man erhält substituierte 5,6-Dihydropyrone der Formel (I-a) 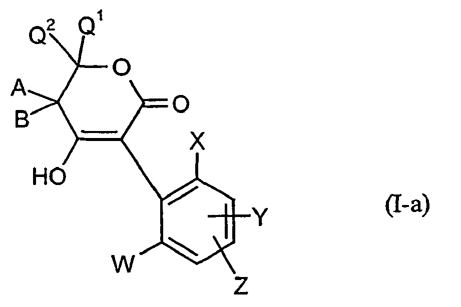 in welcher
   A, B, Q¹, Q², W, X, Y und Z die oben angegebenen Bedeutungen haben,
   wenn man
   O-Acylhydroxycarbonsäureester der Formel (II) 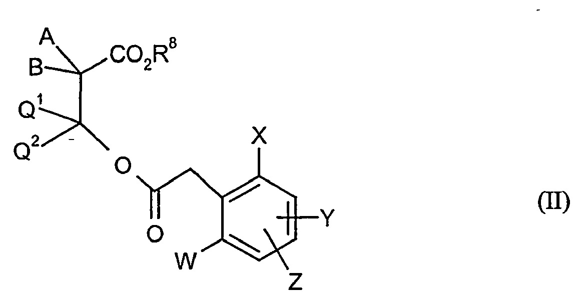 in welcher
   A, B, Q¹, Q², W, X, Y und Z die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
   Weiterhin wurde gefunden,
(B) dass man Verbindungen der oben gezeigten Formeln (I-a) bis (I-g), in welchen A, B, G, Q¹, Q², W, X, Y und Z die oben angegebenen Bedeutungen haben, erhält, wenn man Verbindungen der Formel (I-a') bis (I-g'), 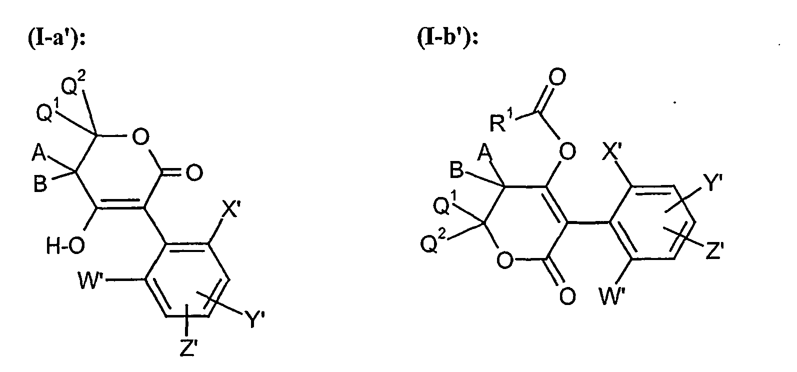 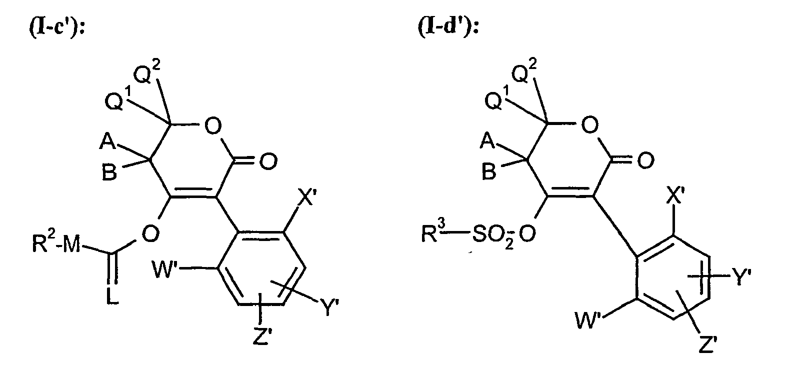 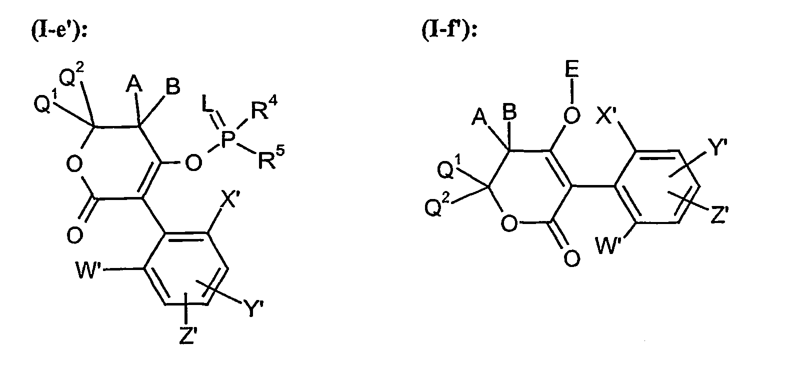 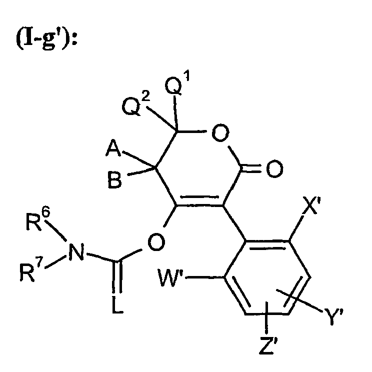 in welchen
   A, B, R¹, R², R³, R⁴, R⁵, R⁶, R⁷, E, L, M, Q¹, Q², W', X', Y' und Z' die oben angegebene Bedeutung von W, X, Y und Z haben und wobei mindestens einer der Reste
   W, X', Y' für Chlor, Brom oder Jod, bevorzugt für Brom steht,
   und Z' nicht für Brom oder Jod steht,
   α) mit Silylacetylen der Formel (III) 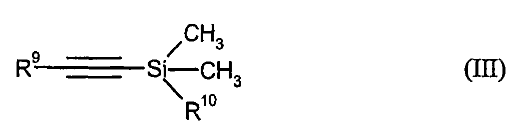 in welcher
      - R⁹: für Wasserstoff und
      - R¹⁰: für C₁-C₄-Alkyl oder Phenyl, besonders für Methyl oder tert.-Butyl steht, zunächst in Gegenwart eines Lösungsmittels, einer Base und eines Katalysators umsetzt, wobei als Katalysator insbesondere Palladium-komplexe in Frage kommen, und anschließend die Silylgruppe abspaltet,
      oder
   β) mit Vinylstannanen der Formel (IV) 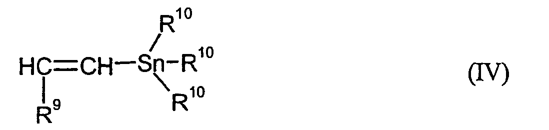 in welcher
      - R⁹: für Wasserstoff, Methyl oder Ethyl und
      - R¹⁰: für C₁-C₄-Alkyl, insbesondere für Butyl steht,
      in Gegenwart eines Lösungsmittels, gegebenenfalls in Gegenwart einer Base und in Gegenwart eines Katalysators umsetzt, wobei als Katalysator insbesondere Palladiumkomplexe in Frage kommen,
      oder
   γ) im speziellen Fall, wenn Y' für Chlor, Brom oder Jod, bevorzugt für Brom steht, und W', X' und Z' nicht für Brom oder Jod steht, mit Boronsäuren der Formel (V) 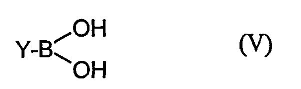 in welcher
      - Y: für gegebenenfalls substituiertes Phenyl oder Hetaryl steht,
      in Gegenwart eines Lösungsmittels, einer Base und eines Katalysators umsetzt, wobei als Katalysator insbesondere Palladiumkomplexe in Frage kommen.
   Außerdem wurde gefunden,
(C) dass man die Verbindungen der oben gezeigten Formel (I-b), in welcher A, B, Q¹, Q², R¹, W, X, Y und Z die oben angebenen Bedeutungen haben, erhält, wenn man Verbindungen der oben gezeigten Formel (I-a), in welchen A, B, Q¹, Q², W, X, Y und Z die oben angegebenen Bedeutungen haben, jeweils
   (α) mit Säurehalogeniden der Formel (VI) 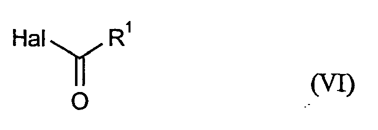 in welcher
      - R¹: die oben angegebene Bedeutung hat und
      - Hal: für Halogen (insbesondere Chlor oder Brom) steht
      oder
   (β) mit Carbonsäureanhydriden der Formel (VII)

      R¹-CO-O-CO-R¹ (VII)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(D) dass man die Verbindungen der oben gezeigten Formel (I-c), in welcher A, B, Q¹, Q², R², M, W, X, Y und Z die oben angegebenen Bedeutungen haben und L für Sauerstoff steht, erhält, wenn man Verbindungen der oben gezeigten Formel (I-a), in welcher A, B, Q¹, Q², W, X, Y und Z die oben angegebenen Bedeutungen haben, jeweils
   mit Chlorameisensäureestern oder Chlorameisensäurethioestern der Formel (VIII)

   R²-M-CO-Cl (VIII)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(E) dass man Verbindungen der oben gezeigten Formel (I-c), in welcher A, B, Q¹, Q², R², M, W, X, Y und Z die oben angegebenen Bedeutungen haben und L für Schwefel steht, erhält, wenn man Verbindungen der oben gezeigten Formel (I-a), in welcher A, B, Q¹, Q², W, X, Y und Z die oben angegebenen Bedeutungen haben, jeweils
   mit Chlormonothioameisensäureestern oder Chlordithioameisensäureestern der Formel (IX) 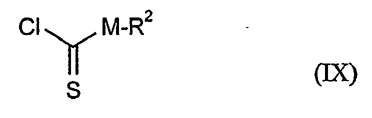 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt
   und
(F) dass man Verbindungen der oben gezeigten Formel (I-d), in welcher A, B, Q¹, Q², R³, W, X, Y und Z die oben angegebenen Bedeutungen haben, erhält, wenn man Verbindungen der oben gezeigten Formel (I-a), in welcher A, B, Q¹, Q², W, X, Y und Z die oben angegebenen Bedeutungen haben, jeweils
   mit Sulfonsäurechloriden der Formel (X)

   R³-SO₂-Cl (X)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(G) dass man Verbindungen der oben gezeigten Formel (I-e), in welcher A, B, L, Q¹, Q², R⁴, R⁵) W, X, Y und Z die oben angegebenen Bedeutungen haben, erhält, wenn man Verbindungen der oben gezeigten Formel (I-a), in welcher A, B, Q¹, Q², W, X, Y und Z die oben angegebenen Bedeutungen haben, jeweils
   mit Phosphorverbindungen der Formel (XI) 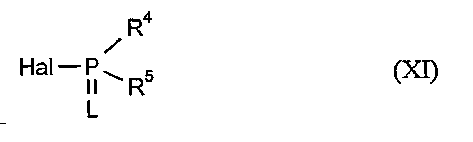 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(H) dass man Verbindungen der oben gezeigten Formel (I-f), in welcher A, B, E, Q¹, Q², W, X, Y und Z die oben angegebenen Bedeutungen haben, erhält, wenn man Verbindungen der oben gezeigten Formel (I-a), in welchen A, B, Q¹, Q², W, X, Y und Z die oben angegebenen Bedeutungen haben, jeweils
   mit Metallverbindungen oder Aminen der Formeln (XII) oder (XIII)

   Me(OR¹¹)ₜ (XII)

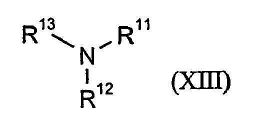

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹¹, R¹², R¹³: unabhängig voneinander für Wasserstoff oder Alkyl (bevorzugt C₁-C₈-Alkyl) stehen,
   gegebenenfalls in Gegenwart eines Verdünnungsmittels umsetzt,
(I) dass man Verbindungen der oben gezeigten Formel (I-g), in welcher A, B, L, Q¹, Q², R⁶, R⁷, W, X, Y und Z die oben angegebenen Bedeutungen haben, erhält, wenn man Verbindungen der oben gezeigten Formel (I-a), in welcher A, B, Q¹, Q², W, X, Y und Z die oben angegebenen Bedeutungen haben, jeweils
   (α) mit Isocyanaten oder Isothiocyanaten der Formel (XIV)

      R⁶-N=C=L (XIV)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   (β) mit Carbamidsäurechloriden oder Thiocarbamidsäurechloriden der Formel (XV) 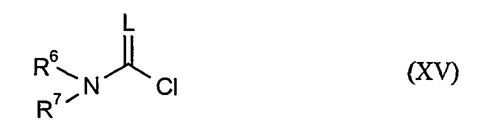 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt.
   Weiterhin wurde gefunden, dass sich 5,6-Dihydropyron-Derivate der Formel (I) herstellen lassen, indem man
J) Silylenolether der Formel 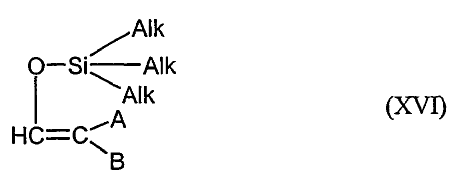 in welcher
   A und B die oben angegebenen Bedeutungen haben und
   Alk für Alkyl mit 1 bis 4 Kohlenstoffatomen steht,
   jeweils mit Keten-Derivaten der Formel 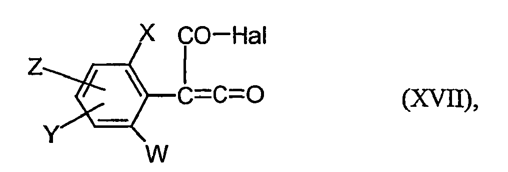 in welcher
   - W, X, Y und Z: die oben angegebenen Bedeutungen haben und
   - Hal: für Chlor oder Brom steht,
   gegebenenfalls in Gegenwart eines Verdünnungsmittels oder eines Verdünnungsmittelgemisches und gegebenenfalls in Gegenwart eines Säurebindemittels umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und auch als Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- W: steht bevorzugt für Wasserstoff, C₁-C₆-Alkyl, C₂-C₄-Alkenyl, Ethinyl, Fluor, Chlor, Brom, C₁-C₄-Halogenalkyl oder C₁-C₆-Alkoxy,
- X: steht bevorzugt für Fluor, Chlor, Brom, C₁-C₆-Alkyl, C₁-C₄-Halogenalkyl, C₁-C₆-Alkoxy, C₂-C₄-Alkenyl, Ethinyl, C₁-C₄-Halogenalkoxy, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder Benzyloxy,
- Y: steht bevorzugt für Wasserstoff, C₁-C₆-Alkyl, C₁-C₄-Halogenalkyl, Fluor, Chlor, Brom, C₁-C₆-Alkoxy, C₂-C₄-Alkenyl, Ethinyl oder für einen der Reste 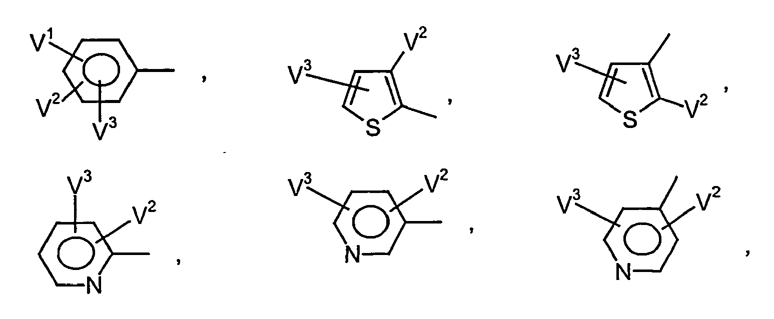
- V¹: steht bevorzugt für Wasserstoff, Halogen, C₁-C₁₂-Alkyl, C₁-C₆-Alkoxy, C₁-C₆-Alkylthio, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio,
- V²: steht bevorzugt für Wasserstoff, Fluor, Chlor, C₁-C₆-Alkyl, C₁-C₆-Alkoxy, C₁-C₄-Halogenalkyl oder C₁-C₄-Halogenalkoxy,
- V³: steht bevorzugt für Wasserstoff, Fluor, Chlor, Methyl oder Methoxy,
- Z: steht bevorzugt für Wasserstoff, Fluor, Chlor, Brom, C₁-C₆-Alkyl, C₁-C₄-Halogenalkyl, C₁-C₆-Alkoxy, C₁-C₄-Halogenalkoxy oder Cyano,
mit der ersten Maßgabe, dass W, X und Z nicht für Brom, C₂-C₄-Alkenyl und Ethinyl stehen, wenn Y für durch V¹, V² und V³ substituiertes Phenyl oder Hetaryl steht, und dass zweitens nur maximal zwei der Reste W, X und Y für C₂-C₄-Alkenyl oder Ethinyl stehen dürfen, mit der Maßgabe, dass dann keiner der anderen Reste W, X, Y und Z für Brom stehen darf,
- A: steht bevorzugt für eine Bindung, Wasserstoff oder jeweils gegebenenfalls durch Halogen substituiertes C₁-C₁₂-Alkyl, C₃-C₈-Alkenyl, C₁-C₆-Alkoxy-C₁-C₄-alkyl, jeweils gegebenenfalls durch Halogen, C₁-C₄-Alkyl oder C₁-C₄-Alkoxy substituiertes C₃-C₈-Cycloalkyl oder C₃-C₆-Cycloalkyl-C₁-C₄-alkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Benzyl, Hetaryl mit 5 bis 6 Ringatomen (beispielsweise Furanyl, Pyridyl, Imidazolyl, Triazolyl, Pyrazolyl, Pyrimidyl, Thiazolyl oder Thienyl) oder Hetaryl-C₁-C₄-alkyl mit 5 bis 6 Ringatomen (beispielsweise Pyridyl, Pyrimidyl oder Thiazolyl),
- B: steht bevorzugt für Wasserstoff oder C₁-C₆-Alkyl, oder
- A, B: und das Kohlenstoffatom an das sie gebunden sind, stehen bevorzugt für gesättigtes C₃-C₁₀-Cycloalkyl oder ungesättigtes C₅-C₁₀-Cycloalkyl, worin gegebenenfalls ein Ringglied durch Sauerstoff oder Schwefel ersetzt ist und welche gegebenenfalls einfach oder zweifach durch C₁-C₆-Alkyl, C₃-C₈-Cycloalkyl, C₁-C₆-Halogenalkyl, C₁-C₆-Alkoxy, C₁-C₆-Alkylthio, Halogen oder Phenyl substituiert sind, mit der Maßgabe, dass Q¹ und Q² keinen weiteren Cyclus bilden, oder
- B und Q¹: stehen gemeinsam bevorzugt für gegebenenfalls einfach oder zweifach, gleich oder verschieden durch C₁-C₄-Alkyl substituiertes C₃-C₆-Alkandiyl, in welchem zwei nicht direkt benachbarte Kohlenstoffatome gegebenenfalls einen weiteren 3- bis 6-gliedrigen Cyclus bilden, oder
- Q¹: steht bevorzugt für Wasserstoff, Hydroxy, C₁-C₆-Alkyl, C₁-C₆-Alkoxy, C₁-C₆-Alkoxy-C₁-C₂-alkyl, C₁-C₆-Alkylacyloxy, gegebenenfalls durch Fluor, Chlor, C₁-C₄-Alkyl, C₁-C₂-Halogenalkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl,
- Q²: steht bevorzugt für Wasserstoff oder C₁-C₄-Alkyl, oder
- Q¹ und Q²: stehen bevorzugt gemeinsam mit dem Kohlenstoffatom, an das sie gebunden sind, für gegebenenfalls durch C₁-C₆-Alkyl, C₁-C₆-Alkoxy oder C₁-C₂-Halogenalkyl substituiertes C₃-C₇-Cycloalkyl, in welchem gegebenenfalls ein Ringglied durch Sauerstoff oder Schwefel ersetzt ist, mit der Maßgabe, dass A und B keinen weiteren Cyclus bilden,
- G: steht bevorzugt für Wasserstoff (a) oder für eine der Gruppen 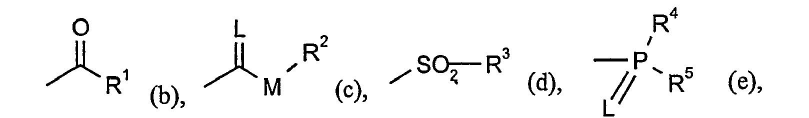 E (f) oder 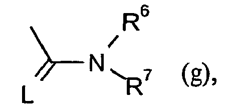 insbesondere für (a), (b) oder (c),
in welchen
E für ein Metallion oder ein Ammoniumion steht,
L für Sauerstoff oder Schwefel steht und
M für Sauerstoff oder Schwefel steht.
- R¹: steht bevorzugt für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere (bevorzugt ein oder zwei) nicht direkt benachbarte Ringglieder durch Sauerstoff und/oder Schwefel ersetzt sind, oder
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl, oder für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl, oder
für gegebenenfalls durch Halogen, C₁-C₆-Alkyl oder Trifluormethyl substituiertes 5- oder 6-gliedriges Hetaryl (beispielsweise Pyrazolyl, Thiazolyl, Pyridyl, Pyrimidyl, Furanyl oder Thienyl), oder
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5-oder 6-gliedriges Hetaryloxy-C₁-C₆-alkyl (beispielsweise Pyridyloxy-C₁-C₆-alkyl, Pyrimidyloxy-C₁-C₆-alkyl oder Thiazolyloxy-C₁-C₆-alkyl),
- R²: steht bevorzugt für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl, oder
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
- R³: steht bevorzugt für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
- R⁴ und R⁵: stehen bevorzugt unabhängig voneinander für jeweils gegebenenfalls durch Halogen substituiertes C₁-C₈-Alkyl, C₁-C₈-Alkoxy, C₁-C₈-Alkyl-amino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogen-alkylthio, C₁-C₄-Alkyl oder C₁-C₄-Halogenalkyl substituiertes Phenyl, Benzyl, Phenoxy oder Phenylthio,
- R⁶ und R⁷: stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenen-falls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest, in welchem gegebenenfalls ein Kohlenstoffatom durch Sauerstoff oder Schwefel ersetzt ist.

In den als bevorzugt genannten Restedefinitionen steht Halogen, auch als Substituent, wie z.B. in Halogenalkyl, für Fluor, Chlor, Brom und Iod, insbesondere für Fluor und Chlor.
- W: steht besonders bevorzugt für Wasserstoff, C₁-C₄-Alkyl, Chlor oder Brom,
- X: steht besonders bevorzugt für Chlor, Brom, C₁-C₄-Alkyl, C₁-C₄-Alkoxy, C₂-C₃-Alkenyl, Ethinyl, C₁-C₂-Halogenalkyl, C₁-C₂-Halogenalkoxy oder Cyano.
- Y: steht besonders bevorzugt für Wasserstoff, C₁-C₄-Alkyl, C₁-C₂-Halogenalkyl, Fluor, Chlor, Brom, C₁-C₄-Alkoxy, C₂-C₃-Alkenyl, Ethinyl, 2-Thienyl, 3-Thienyl oder für den Rest 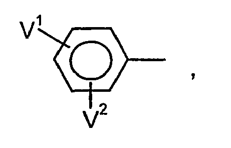
- V¹: steht besonders bevorzugt für Wasserstoff, Fluor, Chlor, Brom, C₁-C₆-Alkyl, C₁-C₄-Alkoxy, C₁-C₄-Alkylthio, C₁-C₂-Halogenalkyl, C₁-C₂-Halogenalkoxy, Nitro, Cyano oder Phenyl,
- V²: steht besonders bevorzugt für Wasserstoff, Fluor, Chlor, C₁-C₄-Alkyl, C₁-C₄-Alkoxy oder C₁-C₂-Halogenalkyl,
- Z: steht besonders bevorzugt für Wasserstoff, Fluor, Chlor, Brom, C₁-C₄-Alkyl, C₁-C₂-Halogenalkyl, C₁-C₄-Alkoxy oder C₁-C₂-Halogenalkoxy,
mit der ersten Maßgabe, dass W, X und Z nicht für Brom, C₂-C₃-Alkenyl und Ethinyl stehen, wenn Y für durch V¹ und V² substituiertes Phenyl, 2-Thienyl oder 3-Thienyl steht, und dass zweitens nur einer der Reste X und Y für C₂-C₃-Alkenyl und Ethinyl stehen darf, mit der Maßgabe, dass dann keiner der anderen Reste W, X, Y und Z für Brom stehen darf.
- A: steht besonders bevorzugt für eine Bindung, Wasserstoff, jeweils gegebenenfalls durch Fluor substituiertes C₁-C₈-Alkyl, C₁-C₄-Alkoxy-C₁-C₂-alkyl, jeweils gegebenenfalls durch Fluor, Chlor, Methyl, Ethyl oder Methoxy substituiertes C₅-C₆-Cycloalkyl oder C₃-C₆-Cycloalkyl-C₁-C₂-alkyl, in welchem gegebenenfalls ein Ringglied durch Sauerstoff oder Schwefel ersetzt ist oder jeweils gegebenenfalls durch Fluor, Chlor, Brom, C₁-C₄-Alkyl, C₁-C₂-Halogenalkyl, C₁-C₄-Alkoxy oder C₁-C₂-Halogenalkoxy substituiertes Phenyl oder Benzyl.
- B: steht besonders bevorzugt für Wasserstoff oder C₁-C₄-Alkyl, oder
- A, B: und das Kohlenstoffatom an das sie gebunden sind, stehen besonders bevorzugt für gesättigtes C₅-C₇-Cycloalkyl, worin gegebenenfalls ein Ringglied durch Sauerstoff ersetzt ist und welches gegebenenfalls einfach durch C₁-C₄-Alkyl, Trifluormethyl oder C₁-C₄-Alkoxy substituiert ist, mit der Maßgabe, dass Q¹ und Q² keinen weiteren Cyclus bilden oder
- B und Q¹: stehen gemeinsam besonders bevorzugt für gegebenenfalls einfach durch C₁-C₂-Alkyl substituiertes C₃-C₄-Alkandiyl in welchem zwei nicht direkt benachbarte Kohlenstoffatome gegebenenfalls einen weiteren fünf- bis sechsgliedrigen Cyclus bilden, oder
- Q¹: steht besonders bevorzugt für Wasserstoff, Hydroxy, C₁-C₄-Alkyl, C₁-C₄-Alkoxy, C₁-C₄-Alkoxy-C₁-C₂-alkyl, C₁-C₄-Alkylacyloxy oder gegebenenfalls durch Methyl oder Methoxy substituiertes C₃-C₆-Cycloalkyl, worin gegebenenfalls eine Methylengruppe durch Sauerstoff ersetzt ist,
- Q²: steht besonders bevorzugt für Wasserstoff, Methyl oder Ethyl, oder
- Q¹ und Q²: stehen besonders bevorzugt gemeinsam mit dem Kohlenstoff, an das sie gebunden sind, für gegebenenfalls durch C₁-C₄-Alkyl, Trifluormethyl oder C₁-C₄-Alkoxy substituiertes gesättigtes C₅-C₆-Cycloalkyl, in welchem gegebenenfalls ein Ringglied durch Sauerstoff ersetzt ist, mit der Maßgabe, dass A und B keinen weiteren Cyclus bilden,
- G: steht besonders bevorzugt für Wasserstoff (a) oder für eine der Gruppen 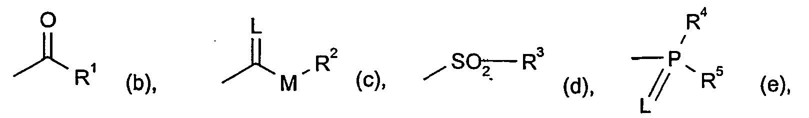 E (f) oder 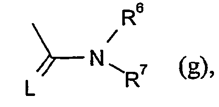 insbesondere für (a), (b) oder (c),
in welchen
E für ein Metallion oder ein Ammoniumion steht,
L für Sauerstoff oder Schwefel steht und
M für Sauerstoff oder Schwefel steht,
- R¹: steht besonders bevorzugt für jeweils gegebenenfalls durch Fluor oder Chlor substituiertes C₁-C₁₆-Alkyl, C₂-C₁₆-Alkenyl, C₁-C₄-Alkoxy-C₁-C₂-alkyl, C₁-C₄-Alkylthio-C₁-C₂-alkyl, oder gegebenenfalls durch Fluor, Chlor, C₁-C₂-Alkyl oder C₁-C₂-Alkoxy substituiertes C₃-C₇-Cycloalkyl, in welchem gegebenenfalls ein oder zwei nicht direkt benachbarte Ringglieder durch Sauerstoff und/oder Schwefel ersetzt sind, oder
für gegebenenfalls einfach bis zweifach durch Fluor, Chlor, Brom, Cyano, Nitro, C₁-C₄-Alkyl, C₁-C₄-Alkoxy, Trifluormethyl oder Trifluormethoxy substituiertes Phenyl, oder
für jeweils gegebenenfalls einfach durch Fluor, Chlor, Brom, Methyl, Ethyl oder Trifluormethyl substituiertes Pyridyl oder Thienyl,
- R²: steht besonders bevorzugt für jeweils gegebenenfalls einfach bis dreifach durch Fluor substituiertes C₁-C₁₆-Alkyl, C₂-C₁₆-Alkenyl oder C₁-C₄-Alkoxy-C₂-C₄-alkyl, oder
für gegebenenfalls einfach durch Methyl, Ethyl oder Methoxy substituiertes C₃-C₇-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl,
- R³: steht besonders bevorzugt für gegebenenfalls einfach bis fünffach durch Fluor substituiertes C₁-C₆-Alkyl oder für einfach bis zweifach gegebenenfalls durch Fluor, Chlor, Brom, C₁-C₄-Alkyl, C₁-C₄-Alkoxy, Trifluormethyl, Trifluormethoxy, Cyano oder Nitro substituiertes Phenyl,
- R⁴: steht besonders bevorzugt für C₁-C₆-Alkyl, C₁-C₆-Alkoxy, C₁-C₆-Alkyl-amino, Di-(C₁-C₆-alkyl)amino, C₁-C₆-Alkylthio, oder für jeweils gegebenenfalls einfach bis zweifach durch Fluor, Chlor, Brom, Nitro, Cyano, C₁-C₃-Alkoxy, Trifluormethoxy, C₁-C₃-Alkyl oder Trifluormethyl substituiertes Phenyl, Benzyl, Phenoxy oder Phenylthio,
- R⁵: steht besonders bevorzugt für C₁-C₄-Alkyl, C₁-C₄-Alkoxy oder C₁-C₄-Alkylthio,
- R⁶: steht besonders bevorzugt für C₁-C₆-Alkyl, C₃-C₆-Cycloalkyl, C₁-C₆-Alkoxy, C₃-C₆-Alkenyl, C₁-C₆-Alkoxy-C₁-C₆-alkyl, oder für gegebenenfalls einfach bis zweifach durch Fluor, Chlor, Brom, Trifluormethyl, C₁-C₄-Alkyl oder C₁-C₄-Alkoxy substituiertes Phenyl, oder für gegebenenfalls einfach bis zweifach durch Fluor, Chlor, Brom, Methyl, Ethyl, Trifluormethyl oder Methoxy substituiertes Benzyl,
- R⁷: steht besonders bevorzugt für Wasserstoff, C₁-C₆-Alkyl oder C₃-C₆-Alkenyl, oder
- R⁶ und R⁷: stehen besonders bevorzugt zusammen für einen gegebenenfalls einfach bis zweifach durch Methyl oder Ethyl substituierten C₄-C₅-Alkylenrest, in welchem gegebenenfalls eine Methylengruppe durch Sauerstoff oder Schwefel ersetzt ist.

In den als besonders bevorzugt genannten Restedefinitionen steht Halogen, auch als Substituent wie z.B. in Halogenalkyl, für Fluor, Chlor, Brom und Iod, besonders für Fluor und Chlor, ganz besonders für Fluor.
- W: steht ganz besonders bevorzugt für Wasserstoff, Chlor, Brom, Methyl oder Ethyl,
- X: steht ganz besonders bevorzugt für Chlor, Brom, Methyl, Ethyl, n-Propyl, Methoxy, Ethoxy, Trifluormethyl, Difluormethoxy, Trifluormethoxy oder Cyano (hervorgehoben für Chlor, Brom, Methyl, Ethyl, n-Propyl oder Trifluormethyl),
- Y: steht ganz besonders bevorzugt für Wasserstoff, Methyl, Ethyl, Propyl, iso-Propyl, Trifluormethyl, Fluor, Chlor, Brom, Methoxy oder für den Rest 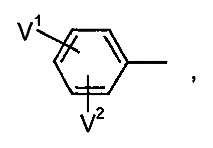
- V¹: steht ganz besonders bevorzugt für Wasserstoff, Fluor, Chlor, Brom, Methyl, Ethyl, iso-Propyl, tert-Butyl, Methoxy, Trifluormethyl oder Trifluormethoxy, Cyano oder Phenyl,
- V²: steht ganz besonders bevorzugt für Wasserstoff, Fluor, Chlor, Methyl oder Trifluormethyl,
- Z: steht ganz besonders bevorzugt für Wasserstoff, Fluor, Chlor, Brom, Methyl, Methoxy oder Trifluormethyl (hervorgehoben für Wasserstoff, Fluor, Chlor, Brom oder Methyl), mit der Maßgabe, dass W, X und Z nicht für Brom stehen, wenn Y für durch V¹ und V² substituiertes Phenyl steht,
- A: steht ganz besonders bevorzugt für eine Bindung, Wasserstoff, Methyl, Ethyl, n-Propyl, iso-Propyl, n-Butyl oder iso-Butyl,
- B: steht ganz besonders bevorzugt für Wasserstoff, Methyl oder Ethyl, oder
- A, B: und das Kohlenstoffatom, an das sie gebunden sind, stehen ganz besonders bevorzugt für gesättigtes C₅-C₆-Cycloalkyl, in welchem gegebenenfalls ein Ringglied durch Sauerstoff ersetzt ist und welches gegebenenfalls einfach durch Methyl, Ethyl, Methoxy oder Ethoxy substituiert ist, mit der Maßgabe, dass Q¹ und Q² keinen weitereren Cyclus bilden oder
- B und: Q¹ stehen gemeinsam ganz besonders bevorzugt für gegebenenfalls einfach durch Methyl substituiertes C₃-C₄-Alkandiyl, in welchem zwei nicht direkt benachbarte Kohlenstoffatome gegebenenfalls einen weiteren drei- bis sechsgliedrigen Cyclus bilden, oder
- Q¹: steht ganz besonders bevorzugt für Wasserstoff, Hydroxy, Methyl, Ethyl, n-Propyl, iso-Propyl, Methoxy, Ethoxy, Propoxy, Acetyloxy oder Propionyloxy,
- Q²: steht ganz besonders bevorzugt für Wasserstoff, Methyl oder Ethyl, oder
- Q¹ und Q²: stehen ganz besonders bevorzugt gemeinsam mit dem Kohlenstoff, an den sie gebunden sind, für gegebenenfalls durch Methyl, Ethyl, Methoxy, Ethoxy, Propoxy oder Butoxy substituiertes gesättigtes C₆-Cycloalkyl, in welchem gegebenenfalls ein Ringglied durch Sauerstoff ersetzt ist, mit der Maßgabe, dass A und B keinen weiteren Cyclus bilden,
- G: steht ganz besonders bevorzugt für Wasserstoff (a) oder für eine der Gruppen 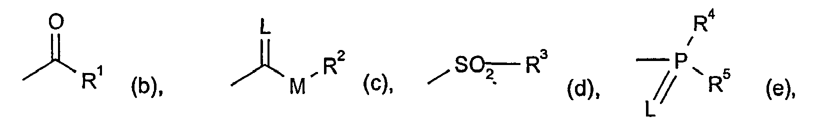 E (f) oder 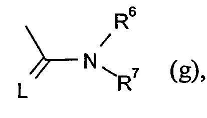 insbesondere für (a), (b) oder (c),
in welchen
E für ein Metallion oder ein Ammoniumion steht,
L für Sauerstoff (insbesonders bevorzugt für (c)) oder Schwefel steht und
M für Sauerstoff oder Schwefel steht.
- R¹: steht ganz besonders bevorzugt für jeweils gegebenenfalls durch Fluor oder Chlor substituiertes C₁-C₈-Alkyl, C₂-C₈-Alkenyl, C₁-C₂-Alkoxy-C₁-alkyl, C₁-C₂-Alkylthio-C₁-alkyl, Cyclopropyl, Cyclopentyl oder Cyclohexyl oder
für gegebenenfalls einfach durch Fluor, Chlor, Brom, Cyano, Nitro, Methyl, Ethyl, iso-Propyl, tert-Butyl, Methoxy, Trifluormethyl oder Trifluormethoxy substituiertes Phenyl, oder
für jeweils gegebenenfalls einfach durch Chlor, Brom oder Methyl substituiertes Thienyl oder Pyridyl,
- R²: steht ganz besonders bevorzugt für C₁-C₈-Alkyl, C₂-C₈-Alkenyl oder C₁-C₄-Alkoxy-C₂-alkyl, oder Cyclohexyl
oder für jeweils gegebenenfalls einfach durch Fluor, Chlor, Brom, Cyano, Nitro, Methyl, tert-Butyl, Methoxy, Trifluormethyl oder Trifluormethoxy substituiertes Phenyl oder Benzyl,
- R³: steht ganz besonders bevorzugt für Methyl, Ethyl, n-Propyl oder gegebenenfalls einfach durch Fluor, Chlor, Brom, Methyl, tert-Butyl, Methoxy, Trifluormethyl, Trifluormethoxy, Cyano oder Nitro substituiertes Phenyl,
- R⁴: steht ganz besonders bevorzugt für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls einfach durch Fluor, Chlor, Brom, Nitro, Cyano, C₁-C₂-Alkoxy, Trifluormethoxy oder C₁-C₃-Alkyl substituiertes Phenyl, Phenoxy oder Phenylthio,
- R⁵: steht ganz besonders bevorzugt für Methyl, Ethyl, Methoxy, Ethoxy, Methylthio oder Ethylthio,
- R⁶: steht ganz besonders bevorzugt für C₁-C₄-Alkyl, C₃-C₆-Cycloalkyl, C₁-C₄-Alkoxy, C₃-C₄-Alkenyl oder C₁-C₄-Alkoxy-C₁-C₄-alkyl,
- R⁷: steht ganz besonders bevorzugt für Wasserstoff, C₁-C₄-Alkyl oder C₃-C₄-Alkenyl, oder
- R⁶ und R⁷: stehen ganz besonders bevorzugt zusammen für einen C₆-Alkylenrest, in welchem gegebenenfalls eine Methylengruppe durch Sauerstoff oder Schwefel ersetzt ist.
- W: steht hervorgehoben für Wasserstoff, Methyl, Ethyl, Chlor oder Brom,
- X: steht hervorgehoben für Methyl, Ethyl, n-Propyl, Trifluormethyl oder Chlor,
- Y: steht hervorgehoben für Methyl, Trifluormethyl, Chlor, Brom, für gegebenenfalls einfach oder zweifach durch Chlor und/oder Methyl substituiertes Phenyl,
- Z: steht hervorgehoben für Wasserstoff oder Methyl, mit der Maßgabe, dass W nicht für Brom steht, wenn Y für substituiertes Phenyl steht,
- A: steht hervorgehoben für Methyl, Ethyl oder eine Bindung,
- B: steht hervorgehoben für Methyl oder Ethyl oder
- A und B: und das Kohlenstoffatom, an das sie gebunden sind stehen hervorgehoben für Cyclopropyl oder Cyclohexyl, oder
- B und Q¹: stehen gemeinsam hervorgehoben für gegebenenfalls einfach durch Methyl substituiertes C₄-Alkandiyl,
- Q¹: steht hervorgehoben für Wasserstoff, Methyl, Methoxy, Ethoxy, Propoxy, Hydroxy oder Acetyloxy,
- Q²: steht hervorgehoben für Wasserstoff oder Methyl,
- G: steht hervorgehoben für Wasserstoff (a) oder für eine der Gruppen 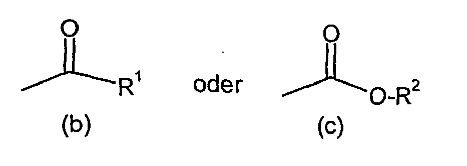 wobei
R¹ hervorgehoben für C₁-C₄-Alkyl oder für jeweils gegebenenfalls einfach durch Chlor substituiertes Phenyl oder Pyridyl steht,
R² hervorgehoben für C₁-C₄-Alkyl steht.

Die oben aufgeführten allgemeinen oder in Vorzugsbereichen aufgeführten Restedefinitionen bzw. Erläuterungen können untereinander, also auch zwischen den j eweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.
Erfindungsgemäß besonders bevorzugt werden die Verbindungen der Formel (I), in welchen eine Kombination der vorstehend als besonders bevorzugt aufgeführten Bedeutungen vorliegt.
Erfindungsgemäß ganz besonders bevorzugt werden die Verbindungen der Formel (I), in welchen eine Kombination der vorstehend als ganz besonders bevorzugt aufgeführten Bedeutungen vorliegt.
Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Verwendet man gemäß Verfahren (A) O-[(2,4-Dichlor)-phenylacetyl]-1-hydroxymethyl-cyclohexan-carbonsäureethylester als Ausgangsstoff, so kann der Verlauf des erfindungsgemäßen Verfahrens durch folgendes Reaktionsschema wiedergegeben werden:

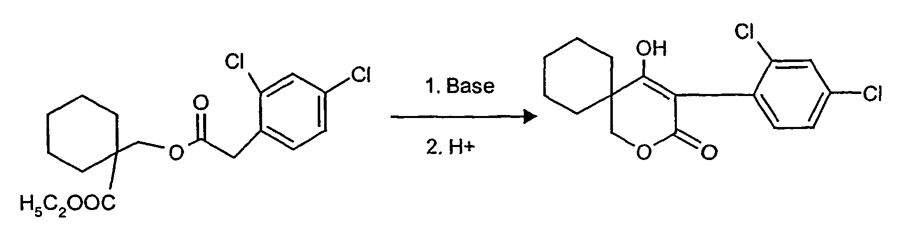

Verwendet man gemäß Verfahren (Bγ) 3-[(2-Chlor-4-brom-6-methyl)-phenyl]-5,5,6,6-tetramethyl-5,6-dihydropyron und 4-Chlorphenylboronsäure als Ausgangsprodukte, so kann der Reaktionsverlauf durch folgendes Schema wiedergegeben werden:

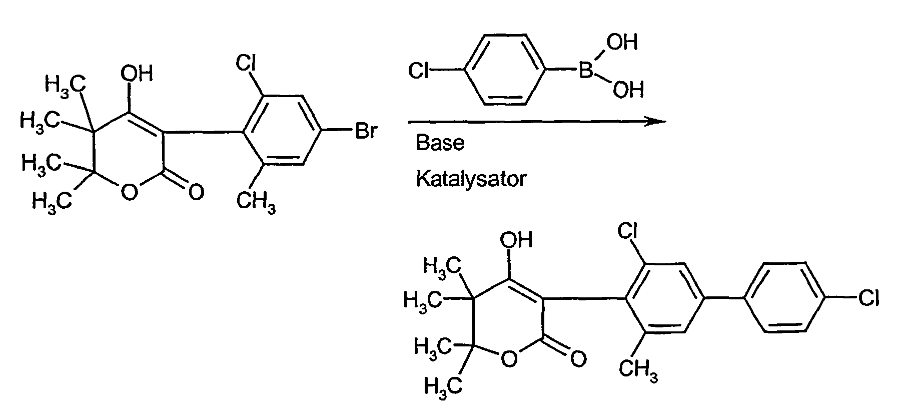

Verwendet man gemäß Verfahren (Cα) 3-[(2,4-Dichlor)-phenyl]-5,5,6,6-tetramethyl-5,6-dihydropyron und Pivaloylchlorid als Ausgangsstoffe, so kann der Verlauf des erfindungsgemäßen Verfahrens durch folgendes Reaktionsschema wiedergegeben werden:

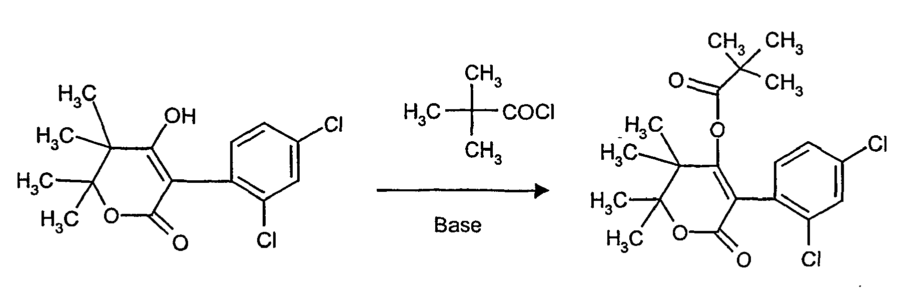

Verwendet man gemäß Verfahren (Cβ) 3-[(4-Brom-2-chlor-6-ethyl)-phenyl]-6,6-dimethyl-5,6-dihydropyron und Acetanhydrid als Ausgangsverbindungen, so kann der Verlauf des erfindungsgemäßen Verfahrens durch folgendes Reaktionsschema wiedergegeben werden:

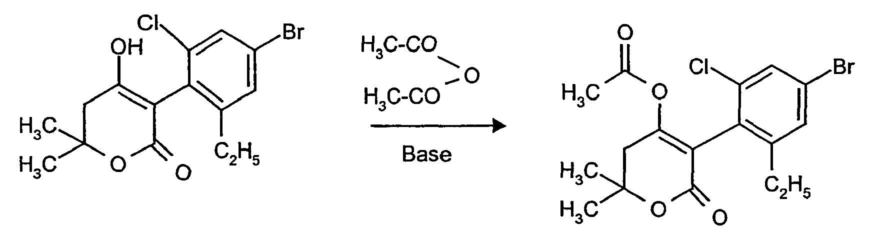

Verwendet man gemäß Verfahren (D) 3-[(2-Chlor-6-ethyl-4-phenyl)-phenyl]-5,5-dimethyl-5,6-dihydropyron und Chlorameisensäureethoxyethylester als Ausgangsverbindungen, so kann der Verlauf des erfindungsgemäßen Verfahrens durch folgendes Reaktionsschema wiedergegeben werden:

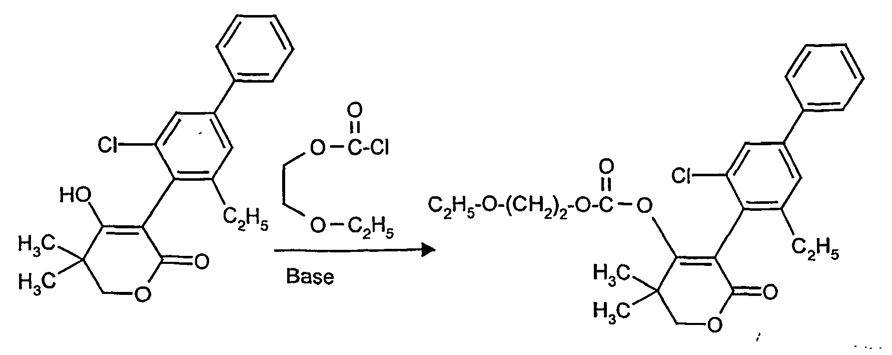

Verwendet man gemäß Verfahren (E), 3-[2,4,6-Trichlor-phenyl]-5,5,6,6-tetramethyl-5,6-dihydropyron und Chlormonothioameisensäuremethylester als Ausgangsprodukte, so kann der Reaktionsverlauf folgendermaßen wiedergegeben werden:

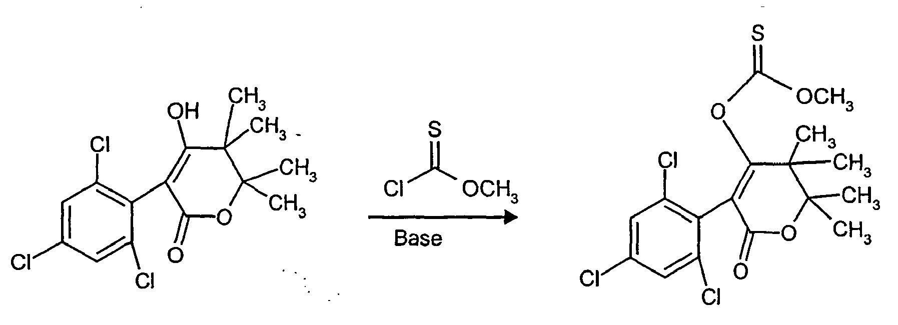

Verwendet man gemäß Verfahren (F) 3-(4-Chlor-2-methyl-phenyl)-5,5-dimethyl-6-methoxy-5,6-dihydropyron und Methansulfonsäurechlorid als Ausgangsprodukte, so kann der Reaktionsverlauf durch folgendes Reaktionsschema wiedergegeben werden:

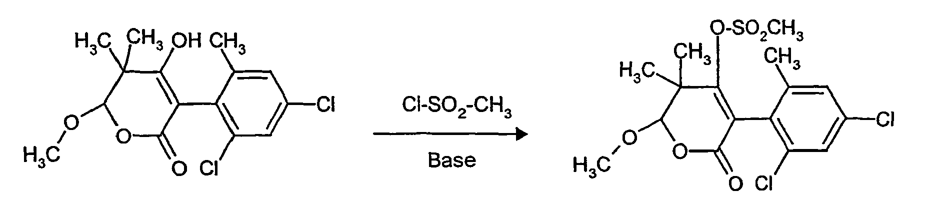

Verwendet man gemäß Verfahren (G) 2-(2-Methyl-5-brom-phenyl)-5,5,6,6-tetramethyl-5,6-dihydropyron und Methanthio-phosphonsäurechlorid-(2,2,2-trifluorethylester) als Ausgangsprodukte, so kann der Reaktionsverlauf durch folgendes Reaktionsschema wiedergegeben werden:

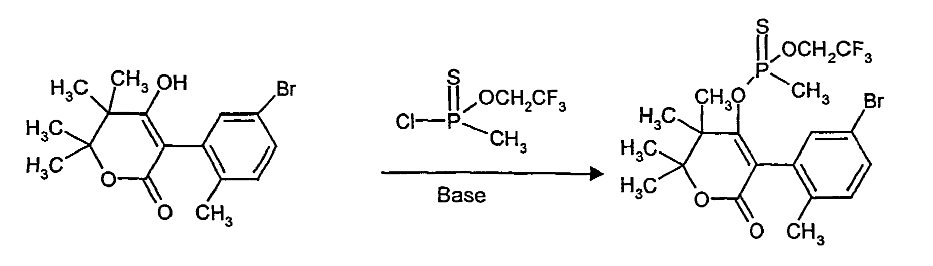

Verwendet man gemäß Verfahren (H) 3-(2,4-Dichlor-phenyl)- 5,5-pentamethylen-6-methoxy-5,6-dihydropyron und NaOH als Komponenten, so kann der Verlauf des erfindungsgemäßen Verfahrens durch folgendes Reaktionsschema wiedergegeben werden:

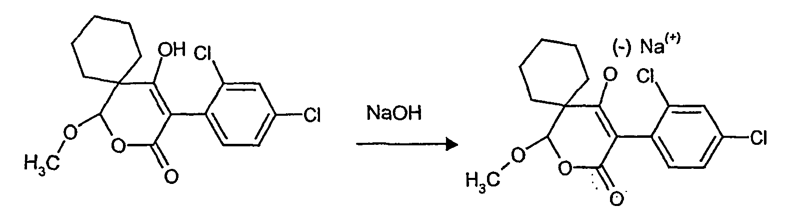

Verwendet man gemäß Verfahren (Iα) 3-(2,4-Dichlor-phenyl)-5,5,6,6-tetramethyl-5,6-dihydropyron und Ethylisocyanat als Ausgangsprodukte, so kann der Reaktionsverlauf durch folgendes Reaktionsschema wiedergegeben werden:

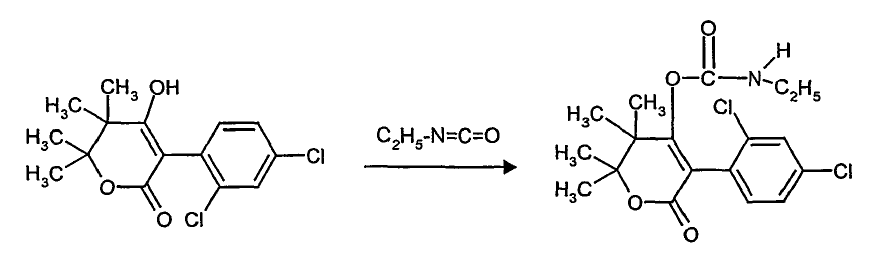

Verwendet man gemäß Verfahren (Iβ) 3-(2-Chlor-4-brom-phenyl)-5,5-dimethyl-6-methoxy-5,6-dihydropyron und Dimethylcarbamidsäurechlorid als Ausgangsprodukte, so kann der Reaktionsverlauf durch folgendes Schema wiedergegeben werden:

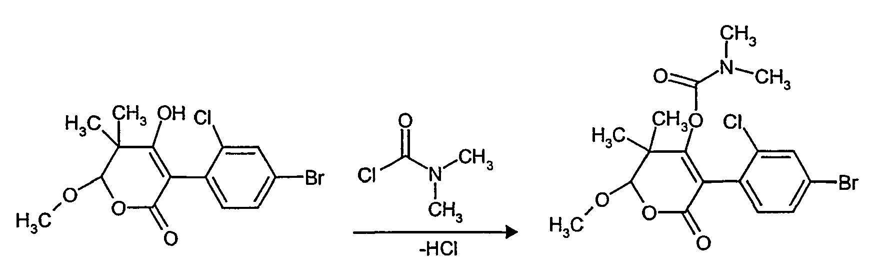

Verwendet man (Chlorcarbonyl)-2-mesitylenketen und Trimethyl-silyloxymethyliden-cyclohexan als Ausgangssubstanzen, so kann der Verlauf des erfindungsgemäßen Verfahrens (J) durch das folgende Formelschema veranschaulicht werden:

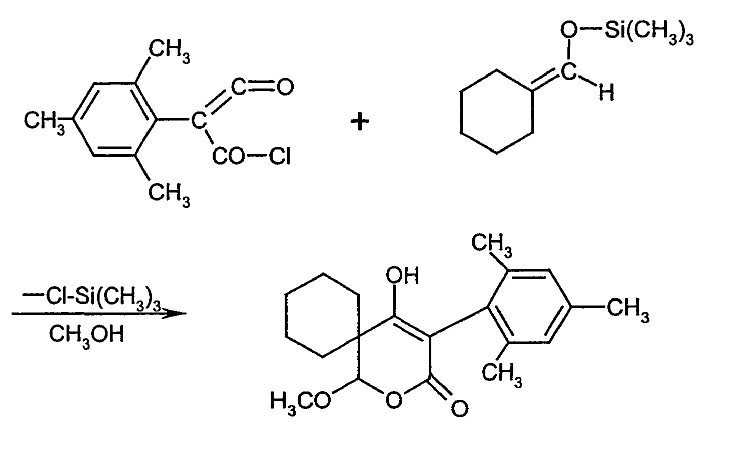

Die beim erfindungsgemäßen Verfahren (A) als Ausgangsstoffe benötigten Verbindungen der Formel (II) 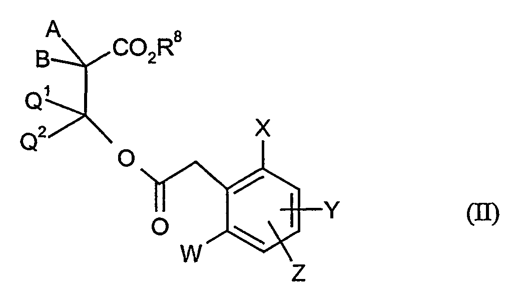 in welcher
- A, B, Q¹, Q², W X, Y, Z und R⁸: die oben angegebenen Bedeutungen haben,
sind neu.

Man erhält die Acylhydroxycarbonsäureester der Formel (II) beispielsweise, wenn man Hydroxycarbonsäureester der Formel (XVIII) 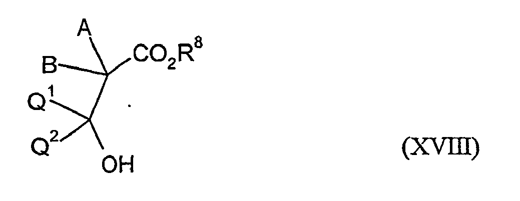 in welcher
- A, B, Q¹, Q² und R⁸: die oben angegebenen Bedeutungen haben,
mit substituierten Phenylessigsäurehalogeniden der Formel (XIX) 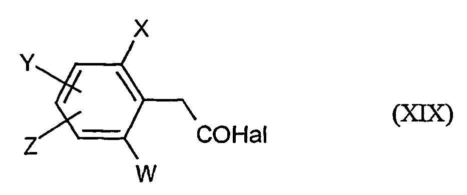 in welcher
- W, X, Y und Z: die oben angegebenen Bedeutungen haben und
- Hal: für Chlor oder Brom steht,
acyliert (s. Herstellungsbeispiele für Verbindungen der Formel (II)).

Die Verbindungen der Formel (XVIII) sind teilweise bekannt oder lassen sich nach im Prinzip bekannten Verfahren z.B. durch Reformatskij-Synthese herstellen (Organikum, VEB Deutscher Verlag der Wissenschaften, Berlin 1990, 18. Aufl., S. 501 ff.).

Die Verbindungen der Formel (XIX) sind teilweise bekannt und käuflich. Sie lassen sich nach im Prinzip bekannten Verfahren darstellen (s. z.B. H. Henecka, Houben-Weyl, Methoden der Organischen Chemie, Bd. 8, S. 467-469 (1952), WO 97/02243, WO 99/43649).

Man erhält die Verbindungen der Formel (XIX) beispielsweise, indem man substituierte Phenylessigsäuren der Formel (XX) 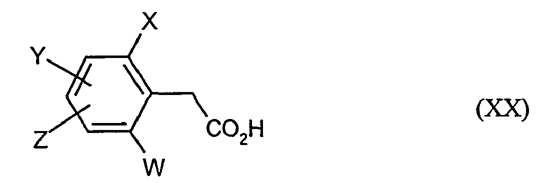 in welcher
- W, X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XX) sind teilweise käuflich, teilweise bekannt oder lassen sich nach im Prinzip bekannten Verfahren herstellen (z.B. WO 97/02243, WO 99/43649).

Die zur Durchführung des Verfahrens B(α) benötigten Silylacetylene der Formel (III) sind teilweise käuflich oder lassen sich nach allgemein bekannten Verfahren herstellen. Die zur Durchführung des Verfahrens B(β) benötigten Vinylstannane der Formel (IV) sind ebenfalls teilweise käuflich oder lassen sich nach bekannten Verfahren herstellen.

Die zur Durchführung des Verfahrens B(γ) benötigten Boronsäuren der Formel (V) 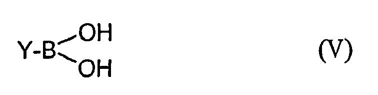 in welcher
- Y: für gegebenenfalls substituiertes Phenyl oder Hetaryl steht,
sind teilweise käuflich oder lassen sich nach allgemein bekannten Verfahren in einfacher Weise herstellen.

Die bei der Durchführung des erfindungsgemäßen Verfahrens (J) als Ausgangsstoffe benötigten Silylenolether sind durch die Formel (XVI) allgemein definiert. In dieser Formel haben A und B vorzugsweise diejenigen Bedeutungen, die bereits im Zusammenhang mit der Beschreibung der erfindungsgemäßen 5,6-Dihydro-pyrone der Formel (I) für diese Reste als bevorzugt genannt wurden. Alk steht vorzugsweise für Methyl oder Ethyl, besonders bevorzugt für Methyl.

Die Silylenolether der Formel (XVI) sind bekannt oder lassen sich nach bekannten Methoden herstellen.

Die bei der Durchführung des erfindungsgemäßen Verfahrens als Reaktionskomponenten benötigten Keten-Derivate sind durch die Formel (XIV) allgemein definiert. In dieser Formel haben W, X, Y und Z vorzugsweise diejenigen Bedeutungen, die bereits im Zusammenhang mit der Beschreibung der erfindungsgemäßen 5,6-Dihydro-pyrone der Formel (I) für diese Reste als bevorzugt genannt wurden. Hal steht auch vorzugsweise für Chlor oder Brom.

Die Keten-Derivate der Formel (XVII) sind bekannt oder lassen sich nach bekannten Verfahren herstellen (vgl. Org. Prep. Proced. Int. 7, 155-158 (1975) und DE-A 1 945 703). So erhält man Keten-Derivate der Formel (XVII), indem man substituierte Phenylmalonsäuren der Formel (XXI) 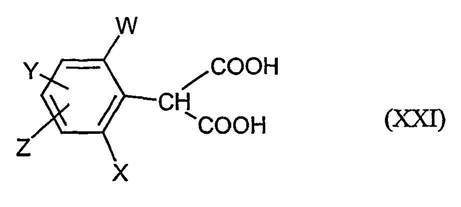 in welcher
W, X, Y und Z die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Sterylformamid oder Triphenylphosphin und gegebenenfalls in Gegenwart von Basen wie z.B. Pyridin oder Triethylamin, bei einer Temperatur zwischen -20°C und +200°C, bevorzugt zwischen 0°C und 150°C, umsetzt.

Die substituierten Phenylmalonsäuren der Formel (XXI) sind bekannt oder lassen sich nach bekannten Methoden herstellen (vgl. z.B. Organikum, VEB Deutscher Verlag der Wissenschaften, Berlin 1977, S. 517 ff). So erhält man substituierte Phenylmalonsäuren der Formel (XXI), indem man substituierte Phenylmalonsäureester der Formel 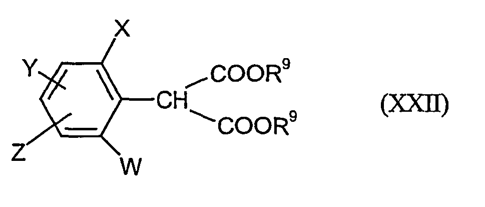 in welcher
- W, X, Y und Z: die oben angegebenen Bedeutungen haben und
- R⁹: für Alkyl mit 1 bis 4 Kohlenstoffatomen steht,
mit Alkalimetallhydroxiden, wie Natriumhydroxid oder Kaliumhydroxid, in Gegenwart eines Verdünnungsmittels, wie Wasser, bei Temperaturen zwischen 0°C und 30°C umsetzt.

In der Formel (XXII) haben W, X, Y und Z vorzugsweise diejenigen Bedeutungen, die bereits im Zusammenhang mit der Beschreibung der erfindungsgemäßen 5,6-Dihydro-pyrone der Formel (I) für diese Reste als bevorzugt genannt wurden. R⁹ steht vorzugsweise für Methyl oder Ethyl.

Die substituierten Phenylmalonsäureester der Formel (XXII) sind bekannt oder lassen sich nach bekannten Methoden herstellen (vgl. Tetrahedron Letters 27, 2763 (1986) und Organikum, VEB Deutscher Verlag der Wissenschaften, Berlin 1977, S. 587 ff.).

Die zur Durchführung der erfindungsgemäßen Verfahren (C), (D), (E), (F), (G), (H) und (I) außerdem als Ausgangsstoffe benötigten Säurehalogenide der Formel (VI), Carbonsäureanhydride der Formel (VII), Chlorameisensäureester oder Chlorameisensäurethioester der Formel (VIII), Chlormonothioameisensäureester oder Chlordithioameisensäureester der Formel (IX), Sulfonsäurechloride der Formel (X), Phosphorverbindungen der Formel (XI) und Metallhydroxide, Metallalkoxide oder Amine der Formel (XII) und (XIII) und Isocyanate der Formel (XIV) und Carbamidsäurechloride der Formel (XV) sind allgemein bekannte Verbindungen der Organischen bzw. Anorganischen Chemie.

Das Verfahren (A) ist **dadurch gekennzeichnet, dass** man Verbindungen der Formel (II), in welcher A, B, Q¹, Q², W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methylpyrrolidon, sowie Alkohole wie Methanol, Ethanol, Propanol, iso-Propanol, Butanol, iso-Butanol und tert-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa doppeltäquimolaren Mengen ein. Es ist jedoch auch möglich, die eine oder andere Komponente in einem größeren Überschuss (bis zu 3 Mol) zu verwenden.

Zur Durchführung des erfindungsgemäßen Verfahrens B (α) bis B (γ) sind Palladium(0)-Komplexe als Katalysator geeignet. Eingesetzt wird beispielsweise Tetrakis-(triphenylphosphin)palladium. Es eignen sich auch Palladium(II)-Verbindungen wie Bis(triphenylphosphin)palladium(II)chlorid.

Als Säureakzeptoren zur Durchführung des erfindungsgemäßen Verfahrens B (α) und B (γ) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Kaliumfluorid, Cäsiumfluorid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens B (γ) kommen Wasser, organische Lösungsmittel und beliebige Mischungen davon in Betracht. Beispielhaft für die Verfahren B (α) bis B (γ) seien als organische Lösungsmittel genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-tert-butyl-, Methyl-tert-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder iso-Propanol, n-, iso-, sek- oder tert-Butanol, Ethandiol, Propan-1,2-diol, Ethoxyethanol, Methoxyethanol, Diethylenglykolmonomethylether; Diethylenglykolmonoethylether; Wasser.

Die Reaktionstemperatur kann bei dem erfindungsgemäßen Verfahren (B) innerhalb eines größeren Bereiches variiert werden. Im allgemeinen arbeitet man bei Temperaturen zwischen 0°C und +180°C, bevorzugt zwischen 50°C und +150°C.

Bei der Durchführung des Verfahrens B (α) werden Silylacetylene der Formel (III) und Verbindungen der Formel (I-a') bis (I-g') im molaren Verhältnis 1:1 bis 10:1, vorzugsweise 1:1 bis 3:1 eingesetzt. Bei der Durchführung des Verfahrens B(β) werden Vinylstannane der Formel (IV) und Verbindungen der Formel (I-a') bis (I-g') im molaren Verhältnis von 1:1 bis 10:1, bevorzugt 1:1 bis 3:1 eingesetzt.

Bei der Durchführung des erfindungsgemäßen Verfahrens B (γ) werden Boronsäuren der Formel (V) und Verbindungen der Formeln (I-a') bis (I-g') im molaren Verhältnis 1:1 bis 3:1, vorzugsweise 1:1 bis 2:1 eingesetzt.

Vom Katalysator setzt man im allgemeinen 0,005 bis 0,5 mol, vorzugsweise 0,01 mol bis 0,1 mol pro Mol der Verbindungen (I-a') bis (I-g') ein. Die Base setzt man im allgemeinen als Überschuss ein.

Das Verfahren (C-α) ist **dadurch gekennzeichnet, dass** man Verbindungen der Formel (I-a) jeweils mit Carbonsäurehalogeniden der Formel (VI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (C-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (C-α) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C-α) werden die Ausgangsstoffe der Formel (I-a) und das Carbonsäurehalogenid der Formel (VI) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (C-β) ist **dadurch gekennzeichnet, dass** man Verbindungen der Formeln (I-a) mit Carbonsäureanhydriden der Formel (VII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (C-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (C-β) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C-β) werden die Ausgangsstoffe der Formel (I-a) und das Carbonsäureanhydrid der Formel (VII) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (D) ist **dadurch gekennzeichnet, dass** man Verbindungen der Formel (I-a) jeweils mit Chlorameisensäureestern oder Chlorameisensäurethiolestern der Formel (VIII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (D) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (D) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylsulfoxid und Sulfolan.

Die Reaktionstemperaturen können bei der Durchführung des erfindungsgemäßen Verfahrens (D) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (D) wird im allgemeinen unter Normaldruck durchgeführt.

Bei der Durchführung des erfindungsgemäßen Verfahrens (D) werden die Ausgangsstoffe der Formel (I-a) und der entsprechende Chlorameisensäureester bzw. Chlorameisensäurethiolester der Formel (VIII) im allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das erfindungsgemäße Verfahren (E) ist **dadurch gekennzeichnet, dass** man Verbindungen der Formel (I-a) jeweils mit Verbindungen der Formel (IX) in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Chlormonothioameisensäureester bzw. Chlordithioameisensäureester der Formel (IX) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Nitrile, Ether, Ester, Amide, Sulfone, Sulfoxide, aber auch Halogenalkane.

Vorzugsweise werden Acetonitril, Ethylacetat, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln, wie z.B. Natriumhydrid oder Kalium-tert-butylat, das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist **dadurch gekennzeichnet, dass** man Verbindungen der Formel (I-a) jeweils mit Sulfonsäurechloriden der Formel (X) gegebenenfalls in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-a) ca. 1 Mol Sulfonsäurechlorid der Formel (X) bei -20 bis 150°C, vorzugsweise bei 20 bis 70°C um.

Als gegebenenfalls zugesetzte Verdünnungsmittel kommen alle inerten polaren organischen Lösungsmittel in Frage, wie Ester, Ether, Amide, Nitrile, Sulfone, Sulfoxide oder halogenierte Kohlenwasserstoffe wie Methylenchlorid.

Vorzugsweise werden Acetonitril, Ethylacetat, Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kalium-tert-butylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist **dadurch gekennzeichnet, dass** man Verbindungen der Formel (I-a) jeweils mit Phosphorverbindungen der Formel (XI) gegebenenfalls in Gegenwart eines Verdünnungsmittels und gegebenenfalls in Gegenwart eines Säurebindemittels umsetzt.

Beim Herstellungsverfahren (G) setzt man zum Erhalt von Verbindungen der Formel (I-e) auf 1 Mol der Verbindung (I-a), 1 bis 2, vorzugsweise 1 bis 1,3 Mol der Phosphorverbindung der Formel (XI) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage, wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (H) ist **dadurch gekennzeichnet, dass** man Verbindungen der Formel (I-a) mit Metallhydroxiden bzw. Metallalkoxiden der Formel (XII) oder Aminen der Formel (XIII), gegebenenfalls in Gegenwart eines Verdünnungsmittels, umsetzt.

Als Verdünnungsmittel können bei dem erfindungsgemäßen Verfahren (H) vorzugsweise Ether, wie Tetrahydrofuran, Dioxan, Diethylether oder aber Alkohole, wie Methanol, Ethanol, Isopropanol, aber auch Wasser eingesetzt werden.

Das erfindungsgemäße Verfahren (H) wird im allgemeinen unter Normaldruck durchgeführt.

Die Reaktionstemperaturen liegen im allgemeinen zwischen -20°C und 100°C, vorzugsweise zwischen 0°C und 50°C.

Das erfindungsgemäße Verfahren (I) ist **dadurch gekennzeichnet, dass** man (I-α) Verbindungen der Formel (I-a) jeweils mit Verbindungen der Formel (XIV) gegebenenfalls in Gegenwart eines Verdünnungsmittels und gegebenenfalls in Gegenwart eines Katalysators oder (I-β) mit Verbindungen der Formel (XV) gegebenenfalls in Gegenwart eines Verdünnungsmittels und gegebenenfalls in Gegenwart eines Säurebindemittels umsetzt.

Bei Herstellungsverfahren (I-α) setzt man pro Mol Ausgangsverbindung der Formel (I-a) ca. 1 Mol Isocyanat der Formel (XIV) bei 0 bis 100°C, vorzugsweise bei 20 bis 50°C um.

Als gegebenenfalls zugesetzte Verdünnungsmittel kommen alle inerten organischen Lösungsmittel in Frage, wie Nitrile, Ester, Ether, Amide, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (I-β) setzt man pro Mol Ausgangsverbindung der Formel (I-a) ca. 1 Mol Carbamidsäurechlorid der Formel (XV) bei -20 bis 150°C, vorzugsweise bei 0 bis 70°C um.

Als gegebenenfalls zugesetzte Verdünnungsmittel kommen alle inerten polaren organischen Lösungsmittel in Frage, wie Nitrile, Ester, Ether, Amide, Sulfone, Sulfoxide oder halogenierte Kohlenwasserstoffe.

Vorzugsweise werden Acetonitril, Ethylacetat, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kalium-tert-butylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Als Verdünnungsmittel kommen bei der Durchführung des erfindungsgemäßen Verfahrens (J) alle üblichen, gegenüber den Reaktionsteilnehmern inerten organischen Solventien in Frage. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methylpyrrolidon. Als Kosolvenzien können gemäß Beispiel (I-2-a) Alkohole wie Methanol, Ethanol, Propanol, Butanol, aber auch Wasser eingesetzt werden.

Als Säureakzeptoren kommen bei der Durchführung des erfindungsgemäßen Verfahrens (J) alle üblichen Säurebindemittel in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base oder N,N-Dimethylanilin.

Die Reaktionstemperaturen können bei der Durchführung des erfindungsgemäßen Verfahrens (J) innerhalb eines größeren Bereiches variiert werden. Zweckmäßigerweise arbeitet man bei Temperaturen zwischen 0°C und 250°C, vorzugsweise zwischen 50°C und 220°C.

Bei der Durchführung des erfindungsgemäßen Verfahrens (J) arbeitet man vorzugsweise unter Atmosphärendruck.

Bei der Durchführung des erfindungsgemäßen Verfahrens (J) setzt man auf 1 Mol an Silylether der Formel (XVI) im allgemeinen eine äquimolare Menge an Keten-Derivat der Formel (XVII) und gegebenenfalls auch eine äquimolare Menge an Säureakzeptor ein. Es ist jedoch auch möglich, die eine oder andere Komponente in einem größeren Überschuß (bis zu 5 Mol) zu verwenden.

Die erfindungsgemäßen 5,6-Dihydro-pyrone der Formel (I) besitzen eine sehr gute pestizide Wirksamkeit und weisen gegenüber Kulturpflanzen eine sehr gute Verträglichkeit auf.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp.

Aus der Ordnung der Phthiraptera z.B. Pediculus humanus corporis, Haematopinus spp., Linognathus spp., Trichodectes spp., Damalinia spp.

Aus der Ordnung der Thysanoptera z.B. Hercinothrips femoralis, Thrips tabaci, Thrips palmi, Frankliniella occidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.

Aus der Ordnung der Siphonaptera z.B. Xenopsylla cheopis, Ceratophyllus spp.

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide

Aldimorph, Ampropylfos, Ampropylfos-Kalium, Andoprim, Anilazin, Azaconazol, Azoxystrobin,
Benalaxyl, Benodanil, Benomyl, Benzamacryl, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
Bis-(1-methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,
4-[3,4-Dimethoxyphenyl-3-(4-fluorphenyl)-acryloxy]-morpholin

### Bakterizide

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chlorethocarb, Chlorethoxyfos, Chlorfenapyr, Chlorfenvinphos, Chlorfluazuron, Chlormephos, Chlorpyrifos, Chlorpyrifos M, Chlorvaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Indoxacarb, Isazofos, Isofenphos, Isoxathion, Ivermectin,
Kempolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Spirodiclofen, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon, Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(phenylmethyl)-3-furanyl]-methyl-3-[(dihydro-2-oxo-3-(2H)-furanyliden)-methyl]-2,2-dimethylcyclopropancarboxylat
(3-Phenoxyphenyl)-methyl-2,2,3,3-tetramethylcyclopropancarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetyloxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid 3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure-[2-benzoyl-1-(1,1-dimethylethyl)-hydrazid
Butansäure-2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylinethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat
N-Cyanomethyl-4-trifluormethyl-nicotinamid
3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufinilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattella germanica, Supella spp.

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec., Tryptodendron spec., Apate monachus, Bostrychus capucins, Heterobostrychus brunneus, Sinoxylon spec., Dinoderus minutus;

### Hautflügler wie

Sirex juvencus, Urocerus gigas, Urocerus gigas taignus, Urocerus augur;

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

### Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls ein Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittels oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel-(gemisch) oder ein Weichmacher(gemisch) ersetzt werden. Diese Zusätze sollen einer Verflüchtigung der Wirkstoffe sowie einer Kristallisation bzw. einem Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide, wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octyl-isothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer, sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, daß die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen, wie z.B. in Bis-(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wis-mutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridin-thiol-1-oxid, Bisdimethyldithiocarbamoylzinkethylenbisthiocarbamat, Zinkoxid, Kupfer(I)-ethylen-bisdithiocarbamat, Kupferthiocyanat, Kupfemaphthenat und Tributylzinnhalogeniden, verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

### Algizide, wie

2-tert-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

### Fungizide, wie

Benzo[b]thiophencarbonsäurecyclohexylamid-S,S-dioxid, Dichlofluanid, Fluorfolpet, 3-Iod-2-propinyl-butylcarbamat, Tolylfluanid, und Azole, wie Azaconazole, Cyproconazole, Epoxyconazole, Hexaconazole, Metconazole, Propiconazole und Tebuconazole;
Molluskizide, wie
Fentinacetat, Metaldehyd, Methiocarb, Niclosamid, Thiodicarb und Trimethacarb;
oder herkömmliche Antifouling-Wirkstoffe, wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile, wie z.B. in Unterer, Chem. Ind. 1985, 37, 730-732 und Williams, Antifouling Marine Coatings, Noyes, Park Ridge,1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga camaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködem oder Köderstationen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakterielle Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die sowohl durch konventionelle Züchtung, durch Mutagenese oder durch rekombinante DNA-Techniken erhalten worden sind. Dies können Sorten, Bio- und Genotyen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen. Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch systemische Akquirierte Resistenz (SAR), Systemin, Phytoalexive, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GAR® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der Formel (I) behandelt werden. Die bei den Wirkstoffen oben angegebenen Vorzugsbereiche gelten auch für die Behandlung dieser Pflanzen. Besonders hervorgehoben sei die Pflanzenbehandlung mit den im vorliegenden Text speziell aufgeführten Mischungen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, BAS-662H, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlormethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, - methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel I-a-1

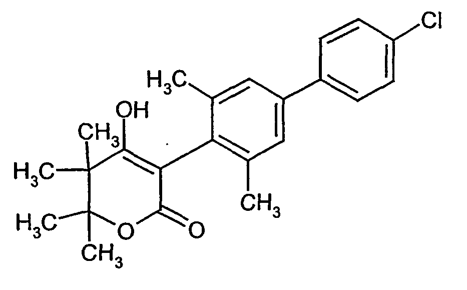

5,6 g (50 mmol) Kalium-tert-butylat werden in 30 ml absolutem DMF vorgelegt und bei 60°C mit 10,6 g der Verbindung gemäß Beispiel II-8 in 20 ml DMF versetzt. Man rührt 3 Stunden bei 60°C. Die Reaktionslösung wird in Eiswasser gegeben und mit konzentrierter Salzsäure angesäuert. Der Niederschlag wird abgesaugt, gewaschen und getrocknet.
Ausbeute: 7,4 g (77 % der Theorie), Fp. >220°C.

### Beispiel I-a-2

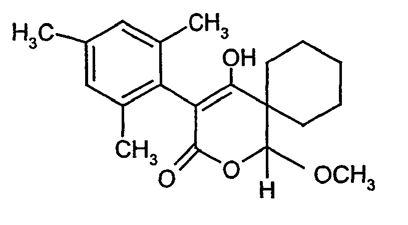

9,8 g (44 mmol) 2,4,5-Trimethylphenyl-chlorcarbonylketen legt man in 80 ml wasserfreiem Xylol vor und tropft bei 20°C 8,1 g (44mmol) Trimethylsilyloxymethylidencyclohexan in 30 ml wasserfreiem Xylol unter Ausschluss von Feuchtigkeit zu. Man erhitzt 8 Stunden unter Rückfluss, tropft dann 7,3 ml Methanol zu und erhitzt weitere 2 Stunden unter Rückfluss. Man wäscht mit Wasser, gesättigter Kochsalzlösung und trocknet über Natriumsulfat. Man dampft im Vakuum ein und chromatographiert den Rückstand an Kieselgel (35 bis 70 µm) mit Toluol/Aceton (20:1).
Ausbeute: 3,9 g (≙ 27 % der Theorie). Fp. 174 bis 175°C.

### Beispiel I-a-3

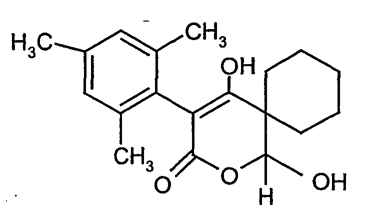

9,4 g (42,2 mmol) 2,4,5-Trimethylphenyl-chlorcarbonylketen legt man in 80 ml wasserfreiem Xylol vor und tropft bei 20°C 7,1 g (42,2 mmol) Trimethylsilyloxymethylidencyclopentan in 30 ml wasserfreiem Xylol unter Ausschluss von Feuchtigkeit zu. Man erhitzt 8 Stunden unter Rückfluss. Dann wird mit Wasser, gesättigter Kochsalzlösung gewaschen und die organische Phase über Natriumsulfat getrocknet. Man dampft ein und chromatographiert den Rückstand an Kieselgel (35 bis 70 µm) mit Toluol als Laufinittel.
Ausbeute: 3,2 g (≙ 25 % der Theorie). Fp. 122 bis 124°C.

In Analogie zu den Beispielen I-a-1, I-a-2 und I-a-3 und gemäß den allgemeinen Angaben zur Herstellung erhält man folgende Verbindungen der Formel I-a

| | | | | | | | | | |
|---|---|---|---|---|---|---|---|---|---|
| 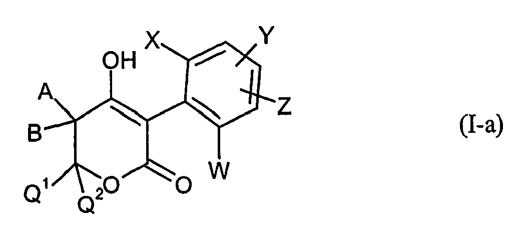 | | | | | | | | | |

| **Bsp.-Nr.** | **W** | **X** | **Y** | **Z** | **B** | **A** | **Q¹** | **Q²** | **Fp. °C** |
|---|---|---|---|---|---|---|---|---|---|
| I-a-4 | H | CH₃ | 4-Cl | H | CH₃ | CH₃ | OCH₃ | H | 164-166 |
| I-a-5 | H | CH₃ | 5-(4-Cl-C₆H₄) | H | CH₃ | CH₃ | H | H | Wachs |
| I-a-6 | H | CH₃ | 5-(4-Cl-C₆H₄) | H | CH₃ | CH₃ | CH₃ | CH₃ | 125 |
| I-a-7 | CH₃ | CH₃ | 4-CH₃ | H | CH₃ | CH₃ | CH₃ | CH₃ | 140 |
| I-a-8 | H | CH₃ | 5-CH₃ | H | CH₃ | CH₃ | OCH₃ | H | Öl |
| I-a-9 | CH₃ | CH₃ | 3-CH₃ | 4-CH₃ | CH₃ | CH₃ | OCH₃ | H | 121-122 |
| I-a-10 | CH₃ | CH₃ | 3-CH₃ | 4-CH₃ | CH₃ | CH₃ | OH | H | 184-186 |
| I-a-11 | CH₃ | CH₃ | 4-CH₃ | H | CH₃ | CH₃ | OCH₃ | H | 115-117 |
| I-a-12 | CH₃ | CH₃ | 4-CH₃ | H | 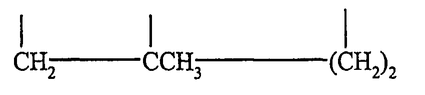 | | | H | 171 |
| I-a-13 | CH₃ | CH₃ | 4-(4-Cl-C₆H₄) | H | CH₃ | CH₃ | H | H | 174 |
| I-a-14 | CH₃ | CH₃ | 4-CH₃ | H | CH₃ | CH₃ | OC₂H₅ | H | 90-93 |
| I-a-15 | CH₃ | CH₃ | 4-CH₃ | H | -(CH₂)₅- | | OC₂H₅ | H | 148-150 |
| I-a-16 | CH₃ | CH₃ | 4-CH₃ | H | CH₃ | CH₃ | OC₃H₇ | H | 108-110 |
| I-a-17 | CH₃ | CH₃ | 4-CH₃ | H | CH₃ | C₂H₅ | OCH₃ | H | 111-113 |
| I-a-18 | CH₃ | CH₃ | 4-CH₃ | H | C₂H₅ | C₂H₅ | OCH₃ | H | 123-125 |
| I-a-19 | CH₃ | CH₃ | 4-CH₃ | H | -(CH₂)₅- | | OH | H | 154-156 |
| I-a-20 | H | CH₃ | 5-(3-Cl-C₆H₄) | H | CH₃ | CH₃ | CH₃ | CH₃ | Öl |
| I-a-21 | H | CH₃ | 5-(4-Cl-C₆H₄) | 4-CH₃ | CH₃ | CH₃ | CH₃ | CH₃ | 182-184°C |
| I-a-22 | CH₃ | CH₃ | 5-(4-Cl-C₆H₄) | 4-CH₃ | CH₃ | CH₃ | CH₃ | CH₃ | 233-234°C |
| I-a-23 | H | CH₃ | 5-Br | H | CH₃ | CH₃ | CH₃ | CH₃ | 184-186°C |
| I-a-24 | H | CH₃ | 5-(3,5-Cl₂-C₆H₃) | H | CH₃ | CH₃ | CH₃ | CH₃ | 192-193°C |
| I-a-25 | H | CH₃ | 5-(2-CH₃, 4-Cl-C₆H₃) | H | CH₃ | CH₃ | CH₃ | CH₃ | 202-203°C |
| I-a-26 | H | CF₃ | 4-Cl | H | CH₃ | CH₃ | CH₃ | CH₃ | 185-186°C |
| I-a-27 | Br | C₃H₇ | 4-Br | H | CH₃ | CH₃ | CH₃ | CH₃ | Öl |
| I-a-28 | C₂H₅ | C₂H₅ | 4-Br | H | CH₃ | CH₃ | CH₃ | CH₃ | 178-180°C |
| I-a-29 | Cl | Cl | 4-CF₃ | H | CH₃ | CH₃ | CH₃ | CH₃ | 233-234°C |
| I-a-30 | Cl | Cl | 4-Cl | H | CH₃ | CH₃ | CH₃ | CH₃ | >250°C |
| I-a-31 | H | Cl | 5-(4-Cl-C₆H₄) | H | CH₃ | CH₃ | CH₃ | CH₃ | 204-205°C |

### Beispiel I-A-b-1

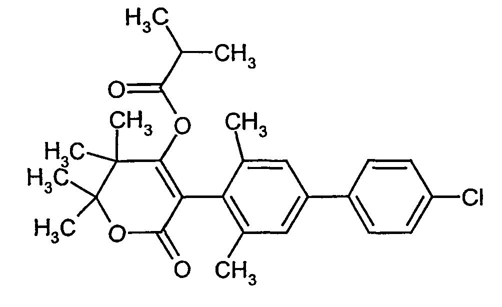

1,5 g (3,9 mmol) der Verbindung gemäß Beispiel I-a-1 werden in 15 ml wasserfreiem Dichlormethan vorgelegt und mit 0,78 ml (5,85 mmol) Triethylamin versetzt. Bei 0°C werden 0,57 g (5,07 mmol) Isobuttersäurechlorid zugegeben und 2 Stunden bei Raumtemperatur gerührt. Die Reaktionslösung wird mit 10 %iger Zitronensäure extrahiert und mit Dichlormethan gewaschen. Anschließend extrahiert man mit 1 N NaOH und wäscht nochmals mit Dichlormethan, trocknet und dampft das Lösungsmittel ein.
Ausbeute: 1,2 g (≙ 68 % der Theorie).
¹H-NMR (400 MHz, DMSO): δ = 0,69 (d, 6H, (CH₃)₂-CH); 1,17 (s, 6H, (CH₃)₂-C), 1,52 (s, 6H, (CH₃)₂-C-O), 2,11 (s, 6H, 2 Ar-CH₃) ppm.

In Analogie zu den Beispielen (I-A-b-1) und gemäß den allgemeinen Angaben zur Herstellung erhält man folgende Verbindungen der Formel (I-A-b) und (I-B-b)

| | | | | | | | | | | |
|---|---|---|---|---|---|---|---|---|---|---|
| 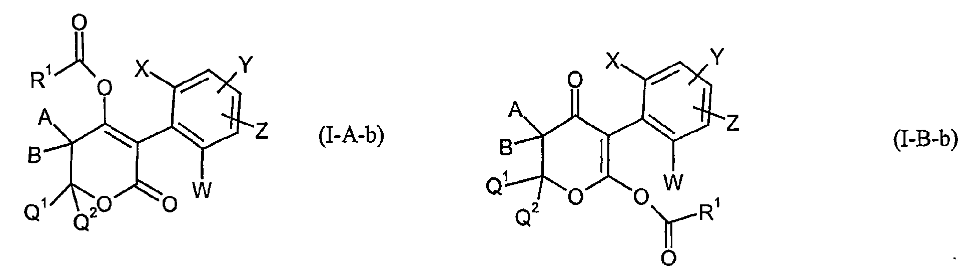 | | | | | | | | | | |

| **Bsp.-Nr.** | **W** | **X** | **Y** | **Z** | **B** | **A** | **Q¹** | **Q²** | **R¹** | **Fp. °C** |
|---|---|---|---|---|---|---|---|---|---|---|
| I-A-b-2 | H | CH₃ | 5-CH₃ | H | CH₃ | CH₃ | OCH₃ | H | CH₃ | Öl |
| I-A-b-3 | H | CH₃ | 4-Cl | H | CH₃ | CH₃ | OCH₃ | H | CH₃ | Öl |
| I-A-b-4 | CH₃ | CH₃ | 4-CH₃ | H | 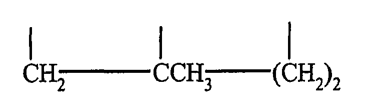 | | | H | CH(CH₃)₂ | 84-86 |
| I-A-b-5 | CH₃ | CH₃ | 4-CH₃ | H | CH₃ | CH₃ | OCH₃ | H | CH₃ | 118-120 |
| I-A-b-6 | CH₃ | CH₃ | 4-CH₃ | H | CH₃ | CH₃ | OCH₃ | H | 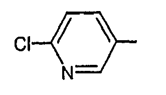 | 115-117 |
| I-A-b-7 | CH₃ | CH₃ | 4-CH₃ | H | CH₃ | CH₃ | OCH₃ | H | 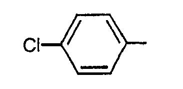 | Öl |
| I-A-b-8 | CH₃ | CH₃ | 4-CH₃ | H | CH₃ | CH₃ | O-COCH₃ | H | CH₃ | 125-127 |
| I-A-b-9 | CH₃ | CH₃ | 4-CH₃ | H | -(CH₂)₄- | | O-COCH₃ | H | CH₃ | 122-124 |
| I-A-b-10 | H | CH₃ | 5-(4-Cl-C₆H₄) | H | CH₃ | CH₃ | CH₃ | CH₃ | i-C₃H₇ | Öl |
| I-A-b-11 | CH₃ | CH₃ | 4-(4-Cl-C₆H₄) | H | CH₃ | CH₃ | H | H | i-C₃H₇ | Öl |
| I-B-b-12 | H | CH₃ | 5-CH₃ | H | CH₃ | CH₃ | OCH₃ | H | CH₃ | Öl |
| I-B-b-13 | H | CH₃ | 4-Cl | H | CH₃ | CH₃ | OCH₃ | H | CH₃ | Öl |
| I-B-b-14 | CH₃ | CH₃ | 4-CH₃ | H | CH₃ | CH₃ | OCH₃ | H | CH₃ | 110-113 |
| I-B-b-15 | CH₃ | CH₃ | 4-CH₃ | H | CH₃ | CH₃ | OCH₃ | H | 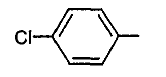 | 134-136 |
| I-B-b-16 | CH₃ | CH₃ | 4-CH₃ | H | CH₃ | CH₃ | CH₃ | CH₃ | i-C₃H₇ | Öl |
| I-B-b-17 | CH₃ | CH₃ | 4-CH₃ | H | CH₃ | CH₃ | H | H | i-C₃H7 | 88-91 |
| I-A-b-18 | H | CH₃ | 5-(4-Cl-C₆H₄) | H | CH₃ | CH₃ | H | H | i-C₃H₇ | Öl |

| | | | | | | | | | | |
|---|---|---|---|---|---|---|---|---|---|---|
| Isomerengemische der Formel I-A-b und I-B-b wurden durch Säulenchromatographie an Kieselgel getrennt. | | | | | | | | | | |

### Beispiel I-A-c-1

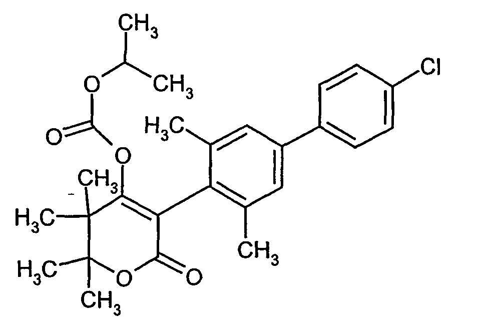

1,5 g (3,9 mmol) der Verbindung gemäß Beispiel I-a-1 werden in 15 ml wasserfreiem Dichlormethan vorgelegt und mit 0,78 ml (5,85 mmol) Triethylamin versetzt. Bei 0°C werden 0,63 g (5,07 mmol) Chlorameisensäureisopropylester zugegeben und 2 Stunden bei Raumtemperatur gerührt. Die Reaktionslösung wird mit 10 %iger Zitronensäure extrahiert und mit Dichlormethan gewaschen. Anschließend extrahiert man mit 1 N NaOH und wäscht nochmals mit Dichlormethan, trocknet und dampft das Lösungsmittel ein.
Ausbeute: 1,2 g (≙ 65 % der Theorie).
¹H-NMR (400 MHz, DMSO): δ = 1,08 (d, 6H, (CH₃)₂-CH); 1,16 (s, 6H, (CH₃)₂-C), 2,11 (s, 6H, 2 Ar-CH₃), 7,36 (s, 2H, 2 Ar-H) ppm.

In Analogie zu Beispiel I-A-c-1 und gemäß den allgemeinen Angaben zur Herstellung wurden folgende Verbindungen der Formeln (I-A-c) und (I-B-c) erhalten

| | | | | | | | | | | | |
|---|---|---|---|---|---|---|---|---|---|---|---|
| 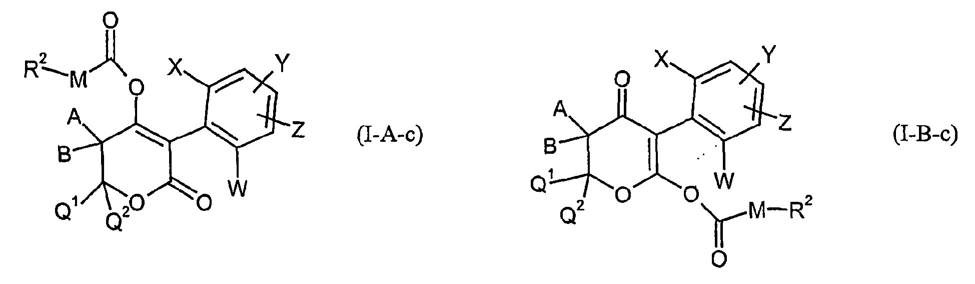 | | | | | | | | | | | |

| **Bsp.-Nr.** | **W** | **X** | **Y** | **Z** | **B** | **A** | **Q¹** | **Q²** | **M** | **R2** | **Fp.°C** |
|---|---|---|---|---|---|---|---|---|---|---|---|
| I-A-c-2 | CH₃ | CH₃ | 4-CH₃ | H | CH₃ | CH₃ | OCH₃ | H | O | CH₃ | 108-110 |
| I-A-c-3 | H | CH₃ | 5-(4-Cl-C₆H₄) | H | CH₃ | CH₃ | CH₃ | CH₃ | O | i-C₃H₇ | Öl |
| I-A-c-4 | CH₃ | CH₃ | 4-(4-Cl-C₆H₄) | H | CH₃ | CH₃ | H | H | O | i-C₃H₇ | Öl |
| I-B-c-1 | CH₃ | CH₃ | 4-CH₃ | H | CH₃ | CH₃ | H | H | O | i-C₃H₇ | Öl |
| I-B-c-2 | CH₃ | CH₃ | 4-CH₃ | H | 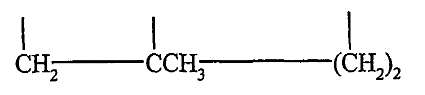 | | | H | O | i-C₃H₇ | Öl |
| I-B-c-7 | CH₃ | CH₃ | 4-CH₃ | H | CH₃ | CH₃ | H | H | O | i-C₃H₇ | Öl |
| I-A-c-8 | H | CH₃ | 5-(4-Cl-C₆H₄) | H | CH₃ | CH₃ | H | H | O | C₂H₅ | Öl |
| I-A-c-9 | H | CH₃ | 5-(4-Cl-C₆H₄) | H | CH₃ | CH₃ | CH₃ | CH₃ | O | C₂H₅ | 144 |
| I-A-c-10 | H | CH₃ | 5-(4-Cl-C₆H₄) | 4-CH₃ | CH₃ | CH₃ | CH₃ | CH₃ | O | C₂H₅ | Öl |
| I-A-c-11 | H | CH₃ | 5-(3-Cl-C₆H₄) | H | CH₃ | CH₃ | CH₃ | CH₃ | O | C₂H₅ | |
| I-A-c-12 | H | CH₃ | 5-Br | H | CH₃ | CH₃ | CH₃ | CH₃ | O | C₂H₅ | Öl |

### Beispiel II-1

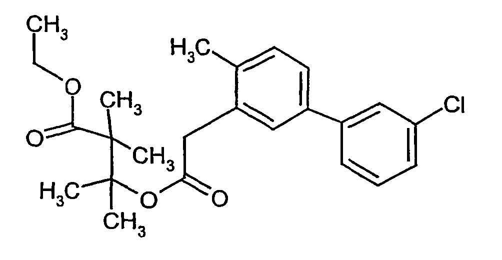

Herstellung des Säurechlorids:
9,0 g (34,5 mmol) 2-Methyl-5-(4-chlorphenyl)-phenylessigsäure werden in 50 ml wasserfreiem Toluol mit 2 Tropfen DMF vorgelegt und 6,15 g (51,8 mmol; 3,74 ml) Thionylchlorid zugegeben. Bei 100°C rührt man bis die Gasentwicklung beendet ist. Das Lösungsmittel wird abdestilliert.
6,00 g (34,5 mmol) 3-Hydroxy-2,2,3-trimethyl-buttersäure-ethylester und 10,3 g (34,5 mmol) Säurechlorid werden in 40 ml wasserfreiem Toluol über Nacht unter Rückfluss gekocht. Das Lösungsmittel wird dann abdestilliert und das Reaktionsgemisch säulenchromatographisch an Kieselgel gereinigt (Petrolether:Essigsäureethylester, 3:1→1:1).
Ausbeute: 11,6 g (≙ 80,6 % der Theorie).
¹H-NMR (DMSO, 400 MHz): δ = 1,06 (s, 6H, 2CH₃), 1,12 (t, 3H, CO₂CH₂CH₃), 1,50 (s, 6H, 2CH₃), 2,26 (s, 3H, Ar-CH₃), 3,66 (s, 2H, CH₂), 4,00 (q, 2H, CO₂CH₂CH₃) ppm.

In Analogie zu Beispiel II-1 und gemäß den allgemeinen Angaben zur Herstellung erhält man folgende Verbindungen der Formel (II)

| | | | | | | | | | | |
|---|---|---|---|---|---|---|---|---|---|---|
| 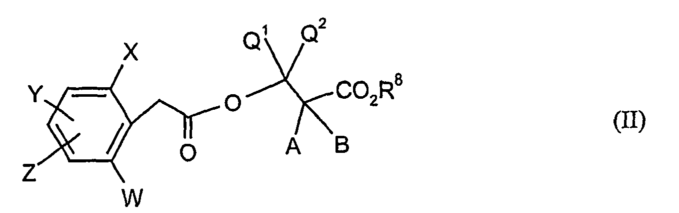 | | | | | | | | | | |

| **Bsp.-Nr.** | **W** | **X** | **Y** | **Z** | **B** | **A** | **Q¹** | **Q²** | **R⁸** | **Fp.°C** |
|---|---|---|---|---|---|---|---|---|---|---|
| II-2 | CH₃ | CH₃ | 4-CH₃ | H | 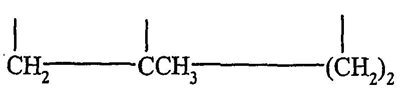 | | | H | C₂H₅ | Öl |
| II-3 | CH₃ | CH₃ | 4-CH₃ | H | CH₃ | CH₃ | CH₃ | CH₃ | C₂H₅ | Öl |
| II-4 | CH₃ | CH₃ | 4-CH₃ | H | CH₃ | CH₃ | H | H | CH₃ | Öl |
| II-5 | H | CH₃ | 5-(4-Cl-C₆H₄) | H | CH₃ | CH₃ | H | H | CH₃ | Öl |
| II-6 | H | CH₃ | 5-(4-Cl-C₆H₄) | H | CH₃ | CH₃ | CH₃ | CH₃ | C₂H₅ | Öl |
| II-7 | CH₃ | CH₃ | 4-(4-Cl-C₆H₄) | H | CH₃ | CH₃ | H | H | CH₃ | Öl |
| II-8 | CH₃ | CH₃ | 4-(4-Cl-C₆H₄) | H | CH₃ | CH₃ | CH₃ | CH₃ | C₂H₅ | Öl |
| II-9 | H | CH₃ | 5-(3-Cl-C₆H₄) | H | CH₃ | CH₃ | CH₃ | CH₃ | C₂H₅ | Öl |
| II-10 | H | CH₃ | 5-(4-Cl-C₆H₄) | 4-CH₃ | CH₃ | CH₃ | CH₃ | CH₃ | C₂H₅ | Öl |
| II-11 | CH₃ | CH₃ | 5-(4-Cl-C₆H₄) | 4-CH₃ | CH₃ | CH₃ | CH₃ | CH₃ | C₂H₅ | Öl |
| II-12 | H | CH₃ | 5-Br | H | CH₃ | CH₃ | CH₃ | CH₃ | C₂H₅ | Öl |
| II-13 | H | CH₃ | 5-(3,5-Cl₂-C₆H₃) | H | CH₃ | CH₃ | CH₃ | CH₃ | C₂H₅ | Öl |
| II-14 | H | CF₃ | 4-Cl | H | CH₃ | CH₃ | CH₃ | CH₃ | C₂H₅ | Öl |
| II-15 | Br | C₃H₇ | 4-Br | H | CH₃ | CH₃ | CH₃ | CH₃ | C₂H₅ | Öl |
| II-16 | C₂H₅ | C₂H₅ | 4-Br | H | CH₃ | CH₃ | CH₃ | CH₃ | C₂H₅ | Öl |
| II-17 | Cl | Cl | 4-CF₃ | H | CH₃ | CH₃ | CH₃ | CH₃ | C₂H₅ | Öl |
| II-18 | Cl | Cl | 4-Cl | H | CH₃ | CH₃ | CH₃ | CH₃ | C₂H₅ | Öl |
| II-19 | H | Cl | 5-(4-Cl-C₆H₄) | H | CH₃ | CH₃ | CH₃ | CH₃ | C₂H₅ | Öl |

Die Verbindungen fielen als Öle an und wurden ohne weitere Aufreinigung zur Herstellung von Verbindungen der Formel (I-a) verwendet.

### Anwendungsbeispiele

### Beispiel A

### Meloidogyne-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % Wirkung bedeutet, dass die Zahl der Gallen an den behandelten Pflanzen der unbehandelten Kontrolle entspricht.

Bei diesem Test zeigen z.B. die folgenden Verbindungen der Herstellungsbeispiele gute Wirksamkeit.

**Tabelle A**

| pflanzenschädigende Nematoden **Meloidogyne-Test** | | |
|---|---|---|
| Wirkstoffe | Wirkstoffkonzentration in ppm | Abtötungsgrad in % nach 14^{d} |
| Bsp. I-a-5 | 20 | 100 |
| Bsp. I-B-b-17 | 20 | 90 |
| Bsp. I-B-c-1 | 20 | 90 |
| Bsp. I-A-b-4 | 20 | 90 |
| Bsp. I-B-c-2 | 20 | 100 |
| Bsp. I-A-b-10 | 20 | 100 |
| Bsp. I-A-b-1 | 20 | 100 |

### Beispiel B

### Myzus-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele gute Wirksamkeit.

**Tabelle B**

| pflanzenschädigende Insekten **Myzus-Test** | | |
|---|---|---|
| Wirkstoffe | Wirkstoffkonzentration in ppm | Abtötungsgrad in % nach 6^{d} |
| Bsp. I-a-5 | 1 000 | 95 |
| Bsp. I-a-7 | 1 000 | 90 |
| Bsp. I-a-6 | 1 000 | 95 |

### Beispiel C

### Panonychus-Test

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Ca. 30 cm hohe Pflaumenbäumchen (Prunus domestica), die stark von allen Stadien der Obstbaumspinnmilbe (Panonychus Ulmi) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z.B. die folgende Verbindung der Herstellungsbeispiele gute Wirksamkeit.

**Tabelle C**

| pflanzenschädigende Milben **Panonychus-Test** | | |
|---|---|---|
| Wirkstoffe | Wirkstoffkonzentration in ppm | Abtötungsgrad in % nach 14^{d} |
| Bsp. I-a-6 | 200 | 100 |

### Beispiel D

### Phaedon Larven -Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele gute Wirksamkeit.

**Tabelle D**

| pflanzenschädigende Insekten **Phaedon Larven -Test** | | |
|---|---|---|
| Wirkstoffe | Wirkstoffkonzentration in ppm | Abtötungsgrad in % nach 7^{d} |
| Bsp. I-a-5 | 1 000 | 100 |
| Bsp. I-a-6 | 1 000 | 100 |
| Bsp. I-A-b-10 | 1 000 | 100 |
| Bsp. I-A-c-3 | 1 000 | 90 |
| Bsp. I-a-13 | 1 000 | 100 |
| Bsp. I-a-1 | 1 000 | 100 |
| Bsp. I-A-b-11 | 1 000 | 100 |

### Beispiel E

### Spodoptera frugiperda-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z.B. die folgenden Verbindungen der Herstellungsbeispiele gute Wirksamkeit.

**Tabelle E**

| pflanzenschädigende Insekten **Spodoptera frugiperda-Test** | | |
|---|---|---|
| Wirkstoffe | Wirkstoffkonzentration in ppm | Abtötungsgrad in % nach 7^{d} |
| Bsp. I-a-6 | 1 000 | 100 |
| Bsp. I-A-b-10 | 1 000 | 100 |

### Beispiel F

**Tetranychus-Test** (OP-resistent/Tauchbehandlung)

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z.B. die folgenden Verbindungen der Herstellungsbeispiele gute Wirksamkeit.

**Tabelle F**

| pflanzenschädigende Milben **Tetranychus-Test** (OP-resistent/Tauchbehandlung) | | |
|---|---|---|
| Wirkstoffe | Wirkstoffkonzentration in ppm | Abtötungsgrad in % nach 7^{d} |
| Bsp. I-a-6 | 100 | 100 |
| Bsp. I-B-b-17 | 100 | 100 |
| Bsp. I-B-c-1 | 100 | 100 |
| Bsp. I-B-c-2 | 100 | 99 |
| Bsp. I-B-b-16 | 100 | 100 |
| Bsp. I-A-c-3 | 0,01 | 100 |

### Beispiel G

### Meloidogyne-Test

| | |
|---|---|
| Lösungsmittel: | 8 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % Wirkung bedeutet, dass die Zahl der Gallen an den behandelten Pflanzen der unbehandelten Kontrolle entspricht.

Wirkstoffe, Aufwandmengen und Resultate gehen aus der nachstehenden Tabelle hervor:

**Tabelle G**

| Nematizide **Meloidogyne-incognita** | |
|---|---|
| Wirkstoff | Abtötungsgrad in % bei Wirkstoffkonzentration in ppm |
| Bsp. I-a-7 | 20 ppm = 100 % |

### Beispiel H

### Sphaerotheca-Test (Gurke) / protektiv

| | |
|---|---|
| Lösungsmittel: Emulgator: | 48,8 Gewichtsteile N,N-Dimethylformamid 1,2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von Spaerotheca fuliginea inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle H**

| **Sphaerotheca-Test** (Gurke) / protektiv | | |
|---|---|---|
| Wirkstoff | Aufwandmenge an Wirkstoff in g/ha | Wirkungsgrad in % |
| Bsp. I-B-b-13 | 750 | 70 |
| Bsp. I-A-b-3 | 750 | 80 |

### Beispiel I

### Grenzkonzentrations-Test / Bodeninsekten - Behandlung transgener Pflanzen

| | |
|---|---|
| Testinsekt: | Diabrotica balteata - Larven im Boden |
| | |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und läßt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel J

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 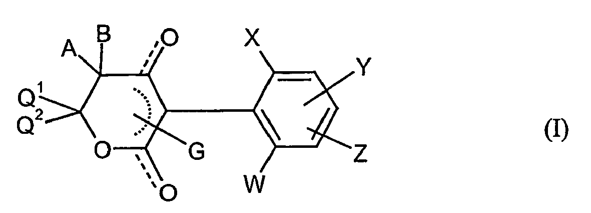 in welcher
W für Wasserstoff, Alkyl, Alkenyl, Alkinyl, Halogen, Halogenalkyl oder Alkoxy steht,
X für Halogen, Alkyl, Alkoxy, Alkenyl, Alkinyl, Halogenalkyl, Halogenalkoxy, Cyano oder jeweils gegebenenfalls substituiertes Phenyl, Phenoxy, Phenylthio, Phenylalkoxy oder Phenylalkylthio steht,
Y für Wasserstoff, Alkyl, Halogen, Halogenalkyl, Alkoxy, Alkenyl, Alkinyl oder gegebenenfalls substituiertes Aryl oder Hetaryl steht,
Z für Wasserstoff, Halogen, Alkyl, Alkoxy, Halogenalkyl, Halogen-alkoxy oder Cyano steht,
A für eine Bindung, Wasserstoff, jeweils gegebenenfalls durch Halogen substituiertes Alkyl, Alkenyl, Alkoxyalkyl, gegebenenfalls substituiertes Cycloalkyl oder Cycloalkylalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl, Hetaryl oder Hetarylalkyl steht,
B für Wasserstoff oder Alkyl steht, oder
A und B gemeinsam mit dem Kohlenstoffatom, an das sie gebunden sind, für einen gesättigten oder ungesättigten, gegebenenfalls mindestens ein Heteroatom enthaltenden unsubstituierten oder substituierten Cyclus stehen, oder
B und Q¹ gemeinsam für gegebenenfalls durch jeweils gegebenenfalls substituiertes Alkyl oder Alkoxy substituiertes Alkandiyl stehen, in welchem zwei nicht direkt benachbarte Kohlenstoffatome gegebenenfalls einen weiteren gegebenenfalls substituierten Cyclus bilden oder
Q¹ für Wasserstoff, Hydroxy, Alkyl, Alkoxy, Alkoxyalkyl, Alkylacyloxy, gegebenenfalls substituiertes Cycloalkyl (worin gegebenenfalls eine Methylengruppe durch Sauerstoff oder Schwefel ersetzt ist) oder gegebenenfalls substituiertes Phenyl steht,
Q² für Wasserstoff oder Alkyl steht, oder
Q¹ und Q² gemeinsam mit dem Kohlenstoffatom, an das sie gebunden sind, für einen gegebenenfalls ein Heteroatom enthaltenden unsubstituierten oder substituierten Cyclus stehen,
G für Wasserstoff (a) oder für eine der Gruppen 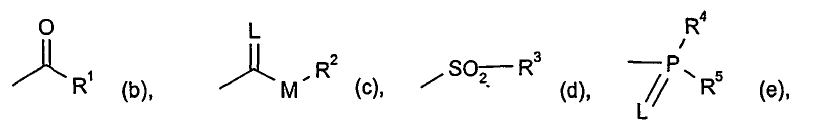 E (f) oder 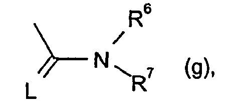 steht,
worin
E für ein Metallion oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, in welchem eine oder mehrere Methylen-gruppen durch Heteroatome ersetzt sein können, jeweils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamin, Dialkylamino, Alkylthio, Alkenylthio, Cycloalkylthio und für jeweils gegebenenfalls substituiertes Phenyl, Benzyl, Phenoxy oder Phenylthio stehen, und
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Ring stehen.

2. Verbindungen der Formel (I) gemäß Anspruch 1, in welcher
W für Wasserstoff, C₁-C₆-Alkyl, C₂-C₄-Alkenyl, Ethinyl, Fluor, Chlor, Brom, C₁-C₄-Halogenalkyl oder C₁-C₆-Alkoxy steht,
X für Fluor, Chlor, Brom, C₁-C₆-Alkyl, C₁-C₄-Halogenalkyl, C₁-C₆-Alkoxy, C₂-C₄-Alkenyl, Ethinyl, C₁-C₄-Halogenalkoxy, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder Benzyloxy steht,
Y für Wasserstoff, C₁-C₆-Alkyl, C₁-C₄-Halogenalkyl, Fluor, Chlor, Brom, C₁-C₆-Alkoxy, C₂-C₄-Alkenyl, Ethinyl oder für einen der Reste 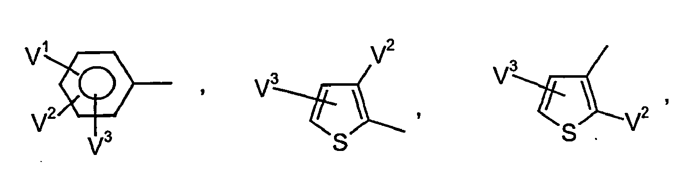 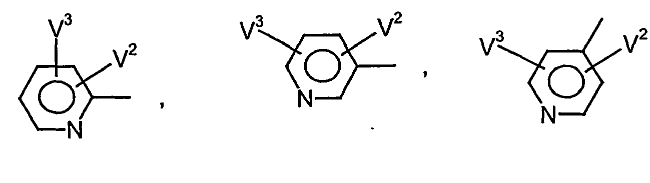 steht,
V¹ für Wasserstoff, Halogen, C₁-C₁₂-Alkyl, C₁-C₆-Alkoxy, C₁-C₆-Alkylthio, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² für Wasserstoff, Fluor, Chlor, C₁-C₆-Alkyl, C₁-C₆-Alkoxy, C₁-C₄-Halogenalkyl oder C₁-C₄-Halogenalkoxy steht,
V³ für Wasserstoff, Fluor, Chlor, Methyl oder Methoxy steht,
Z für Wasserstoff, Fluor, Chlor, Brom, C₁-C₆-Alkyl, C₁-C₄-Halogen-alkyl, C₁-C₆-Alkoxy, C₁-C₄-Halogenalkoxy oder Cyano steht,
mit der ersten Maßgabe, dass W, X und Z nicht für Brom, C₂-C₄-Alkenyl und Ethinyl stehen, wenn Y für durch V¹, V² und V³ substituiertes Phenyl oder Hetaryl steht, und dass zweitens nur maximal zwei der Reste W, X und Y für C₂-C₄-Alkenyl oder Ethinyl stehen dürfen, mit der Maßgabe, dass dann keiner der anderen Reste W, X, Y und Z für Brom stehen darf,
A für eine Bindung, Wasserstoff oder jeweils gegebenenfalls durch Halogen substituiertes C₁-C₁₂-Alkyl, C₃-C₈-Alkenyl, C₁-C₆-Alkoxy-C₁-C₄-alkyl, jeweils gegebenenfalls durch Halogen, C₁-C₄-Alkyl oder C₁-C₄-Alkoxy substituiertes C₃-C₈-Cycloalkyl oder C₃-C₆-Cycloalkyl-C₁-C₄-alkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenakyl, C₁-C₆-Alkoxy, C₁-C₆-Halogenalkoxy, Cyano oder Nitro substituiertes Phenyl, Benzyl, Hetaryl mit 5 bis 6 Ringatomen oder Hetaryl-C₁-C₄-alkyl mit 5 bis 6 Ringatomen steht,
B für Wasserstoff oder C₁-C₆-Alkyl steht, oder
A, B und das Kohlenstoffatom an das sie gebunden sind, für gesättigtes C₃-C₁₀-Cycloalkyl oder ungesättigtes C₅-C₁₀-Cycloalkyl stehen, worin gegebenenfalls ein Ringglied durch Sauerstoff oder Schwefel ersetzt ist und welche gegebenenfalls einfach oder zweifach durch C₁-C₆-Alkyl, C₃-C₈-Cycloalkyl, C₁-C₆-Halogenalkyl, C₁-C₆-Alkoxy, C₁-C₆-Alkylthio, Halogen oder Phenyl substituiert sind, mit der Maßgabe, dass Q¹ und Q² keinen weiteren Cyclus bilden, oder
B und Q¹ gemeinsam für gegebenenfalls einfach oder zweifach, gleich oder verschieden durch C₁-C₄-Alkyl substituiertes C₃-C₆-Alkandiyl stehen, in welchem zwei nicht direkt benachbarte Kohlenstoffatome gegebenenfalls einen weiteren 3- bis 6-gliedrigen Cyclus bilden, oder
Q¹ für Wasserstoff, Hydroxy, C₁-C₆-Alkyl, C₁-C₆-Alkoxy, C₁-C₆-Alkoxy-C₁-C₂-alkyl, C₁-C₆-Alkylacyloxy, gegebenenfalls durch Fluor, Chlor, C₁-C₄-Alkyl, C₁-C₂-Halogenalkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl steht,
Q² für Wasserstoff oder C₁-C₄-Alkyl steht, oder
Q¹ und Q² gemeinsam mit dem Kohlenstoffatom, an das sie gebunden sind, für gegebenenfalls durch C₁-C₆-Alkyl, C₁-C₆-Alkoxy oder C₁-C₂-Halogenalkyl substituiertes C₃-C₇-Cycloalkyl stehen, in welchem gegebenenfalls ein Ringglied durch Sauerstoff oder Schwefel ersetzt ist, mit der Maßgabe, dass A und B keinen weiteren Cyclus bilden,
G für Wasserstoff (a) oder für eine der Gruppen 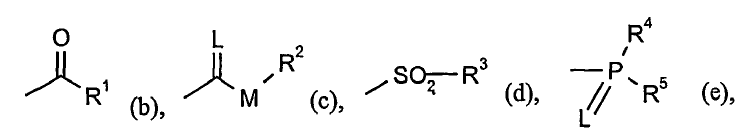 E (f) oder 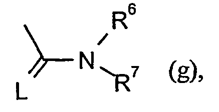 steht,
in welchen
E für ein Metallion oder ein Ammoniumion steht,
L für Sauerstoff oder Schwefel steht und
M für Sauerstoff oder Schwefel steht.
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind, oder
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl, oder
für gegebenenfalls durch Halogen, Nitro, Cyano, C₁-C₆-Alkyl, C₁-C₆-Alkoxy, C₁-C₆-Halogenakyl oder C₁-C₆-Halogenalkoxy substituiertes Phenyl-C₁-C₆-alkyl, oder
für gegebenenfalls durch Halogen, C₁-C₆-Alkyl oder Trifluormethyl substituiertes 5- oder 6-gliedriges Hetaryl oder
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂C₈-alkyl, oder
für gegebenenfalls durch Halogen, C₁-C₆-Alkyl oder C₁-C₆-Alkoxy substituiertes C₃-C₈-Cycoalklyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Benzyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest stehen, in welchem gegebenenfalls ein Kohlenstoffatom durch Sauerstoff oder Schwefel ersetzt ist.

3. Verbindungen der Formel (I) gemäß Anspruch 1, in welcher
W für Wasserstoff, C₁-C₄-Alkyl, Chlor oder Brom steht,
X für Chlor, Brom, C₁-C₄-Alkyl, C₁-C₄-Alkoxy, C₂-C₃-Alkenyl, Ethinyl, C₁-C₂-Halogenalkyl, C₁-C₂-Halogenalkoxy oder Cyano steht.
Y für Wasserstoff, C₁-C₄-Alkyl, C₁-C₂-Halogenalkyl, Fluor, Chlor, Brom, C₁-C₄-Alkoxy, C₂-C₃-Alkenyl, Ethinyl, 2-Thienyl, 3-Thienyl oder für den Rest 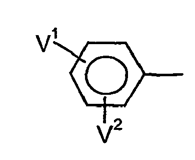 steht,
V¹ für Wasserstoff, Fluor, Chlor, Brom, C₁-C₆-Alkyl, C₁-C₄-Alkoxy, C₁-C₄-Alkylthio, C₁-C₂-Halogenalkyl, C₁-C₂-Halogenalkoxy, Nitro, Cyano oder Phenyl steht,
V² für Wasserstoff, Fluor, Chlor, C₁-C₄-Alkyl, C₁-C₄-Alkoxy oder C₁-C₂-Halogenalkyl steht,
Z für Wasserstoff, Fluor, Chlor, Brom, C₁-C₄-Alkyl, C₁-C₂-Halogenalkyl, C₁-C₄-Alkoxy oder C₁-C₂-Halogenalkoxy steht,
mit der ersten Maßgabe, dass W, X und Z nicht für Brom, C₂-C₃-Alkenyl und Ethinyl stehen, wenn Y für durch V¹ und V² substituiertes Phenyl, 2-Thienyl oder 3-Thienyl steht, und dass zweitens nur einer der Reste X und Y für C₂-C₃-Alkenyl und Ethinyl stehen darf, mit der Maßgabe, dass dann keiner der anderen Reste W, X, Y und Z für Brom stehen darf.
A für eine Bindung, Wasserstoff, jeweils gegebenenfalls durch Fluor substituiertes C₁-C₈-Alkyl, C₁-C₄-Alkoxy-C₁-C₂-alkyl, jeweils gegebenenfalls durch Fluor, Chlor, Methyl, Ethyl oder Methoxy substituiertes C₅-C₆-Cycloalkyl oder C₃-C₆-Cycloakyl-C₁-C₂-akyl, in welchem gegebenenfalls ein Ringglied durch Sauerstoff oder Schwefel ersetzt ist oder jeweils gegebenenfalls durch Fluor, Chlor, Brom, C₁-C₄-Alkyl, C₁-C₂-Halogenalkyl, C₁-C₄-Alkoxy oder C₁-C₂-Halogenalkoxy substituiertes Phenyl oder Benzyl steht.
B für Wasserstoff oder C₁-C₄-Alkyl steht, oder
A, B und das Kohlenstoffatom an das sie gebunden sind, für gesättigtes C₅-C₇-Cycloakyl stehen, worin gegebenenfalls ein Ringglied durch Sauerstoff ersetzt ist und welches gegebenenfalls einfach durch C₁-C₄-Alkyl, Trifluormethyl oder C₁-C₄-Alkoxy substituiert ist, mit der Maßgabe, dass Q¹ und Q² keinen weiteren Cyclus bilden oder
B und Q¹ gemeinsam für gegebenenfalls einfach durch C₁-C₂-Alkyl substituiertes C₃-C₄-Alkandiyl stehen, in welchem zwei nicht direkt benachbarte Kohlenstoffatome gegebenenfalls einen weiteren fünf- bis sechsgliedrigen Cyclus bilden, oder
Q¹ für Wasserstoff, Hydroxy, C₁-C₄-Alkyl, C₁-C₄-Alkoxy, C₁-C₄-Alkoxy-C₁-C₂-alkyl, C₁-C₄-Alkylacyloxy oder gegebenenfalls durch Methyl oder Methoxy substituiertes C₃-C₆-Cycloalkyl steht, worin gegebenenfalls eine Methylengruppe durch Sauerstoff ersetzt ist,
Q² für Wasserstoff, Methyl oder Ethyl steht, oder
Q¹ und Q² gemeinsam mit dem Kohlenstoff, an das sie gebunden sind, für gegebenenfalls durch C₁-C₄-Alkyl, Trifluormethyl oder C₁-C₄-Alkoxy substituiertes gesättigtes C₅-C₆-Cycloalkyl stehen, in welchem gegebenenfalls ein Ringglied durch Sauerstoff ersetzt ist, mit der Maßgabe, dass A und B keinen weiteren Cyclus bilden,
G für Wasserstoff (a) oder für eine der Gruppen 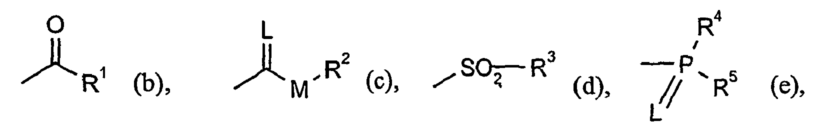 E (f) oder 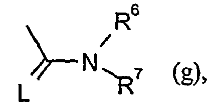 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, C₂-C₁₆-Alkenyl, C₁-C₄-Alkoxy-C₁-C₂-alkyl, C₁-C₄-Alkylthio-C₁-C₂-alkyl, oder gegebenenfalls durch Fluor, Chlor, C₁-C₂-Alkyl oder C₁-C₂-Alkoxy substituiertes C₃-C₇-Cycloalkyl, in welchem gegebenenfalls ein oder zwei nicht direkt benachbarte Ringglieder durch Sauerstoff und/oder Schwefel ersetzt sind, oder
für gegebenenfalls einfach bis zweifach durch Fluor, Chlor, Brom, Cyano, Nitro, C₁-C₄-Alkyl, C₁-C₄-Alkoxy, Trifluormethyl oder Trifluormethoxy substituiertes Phenyl, oder
für jeweils gegebenenfalls einfach durch Fluor, Chlor, Brom, Methyl, Ethyl oder Trifluormethyl substituiertes Pyridyl oder Thienyl steht,
R² für jeweils gegebenenfalls einfach bis dreifach durch Fluor substituiertes C₁-C₁₆-Alkyl, C₂-C₁₆-Alkenyl oder C₁-C₄-Alkoxy-C₂-C₄-alkyl, oder
für gegebenenfalls einfach durch Methyl, Ethyl oder Methoxy substituiertes C₃-C₇-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl steht,
R³ für gegebenenfalls einfach bis fünfach durch Fluor substituiertes C₁-C₆-Alkyl oder für gegebenenfalls einfach bis zweifach durch Fluor, Chlor, Brom, C₁-C₄-Alkyl, C₁-C₄-Alkoxy, Trifluormethyl, Trifluormethoxy, Cyano oder Nitro substituiertes Phenyl steht,
R⁴ für C₁-C₆-Alkyl, C₁-C₆-Alkoxy, C₁-C₆-Alkylamino, Di-(C₁-C₆-alkyl)amino, C₁-C₆-Alkylthio, oder für jeweils gegebenenfalls einfach bis zweifach durch Fluor, Chlor, Brom, Nitro, Cyano, C₁-C₃-Alkoxy, Trifluormethoxy, C₁-C₃-Alkyl oder Trifluormethyl substituiertes Phenyl, Benzyl, Phenoxy oder Phenylthio steht,
R⁵ für C₁-C₄-Alkyl, C₁-C₄-Alkoxy oder C₁-C₄-Alkylthio steht,
R⁶ für C₁-C₆-Alkyl, C₃-C₆-Cycloalkyl, C₁-C₆-Alkoxy, C₃-C₆-Alkenyl, C₁-C₆-Alkoxy-C₁-C₆-alkyl, oder für gegebenenfalls einfach bis zweifach durch Fluor, Chlor, Brom, Trifluormethyl, C₁-C₄-Alkyl oder C₁-C₄-Alkoxy substituiertes Phenyl, oder für gegebenenfalls einfach bis zweifach durch Fluor, Chlor, Brom, Methyl, Ethyl, Trifluormethyl oder Methoxy substituiertes Benzyl steht,
R⁷ für Wasserstoff, C₁-C₆-Alkyl oder C₃-C₆-Alkenyl steht, oder
R⁶ und R⁷ zusammen für einen gegebenenfalls einfach bis zweifach durch Methyl oder Ethyl substituierten C₄-C₅-Alkylenrest stehen, in welchem gegebenenfalls eine Methylengruppe durch Sauerstoff oder Schwefel ersetzt ist.

4. Verbindungen der Formel (I) gemäß Anspruch 1, in welcher
W für Wasserstoff, Chlor, Brom, Methyl oder Ethyl steht,
X für Chlor, Brom, Methyl, Ethyl, n-Propyl, Methoxy, Ethoxy, Trifluormethyl, Difluormethoxy, Trifluormethoxy oder Cyano steht,
Y für Wasserstoff, Methyl, Ethyl, Propyl, iso-Propyl, Fluor, Chlor, Brom, Methoxy oder für den Rest 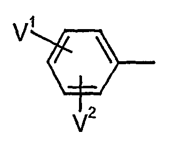 steht,
V¹ für Wasserstoff, Fluor, Chlor, Brom, Methyl, Ethyl, iso-Propyl, tert-Butyl, Methoxy, Trifluormethyl oder Trifluormethoxy, Cyano oder Phenyl steht,
V² für Wasserstoff, Fluor, Chlor, Methyl, Methoxy oder Trifluormethyl steht,
Z für Wasserstoff, Fluor, Chlor, Brom, Methyl, Methoxy oder Trifluormethyl steht, mit der Maßgabe, dass W, X und Z nicht für Brom stehen, wenn Y für durch V¹ und V² substituiertes Phenyl steht,
A für eine Bindung, Wasserstoff, Methyl, Ethyl, n-Propyl, iso-Propyl, n-Butyl oder iso-Butyl steht,
B für Wasserstoff, Methyl oder Ethyl steht, oder
A, B und das Kohlenstoffatom, an das sie gebunden sind, für gesättigtes C₅-C₆-Cycloalkyl stehen, in welchem gegebenenfalls ein Ringglied durch Sauerstoff ersetzt ist und welches gegebenenfalls einfach durch Methyl, Ethyl, Methoxy oder Ethoxy substituiert ist, mit der Maßgabe, dass Q¹ und Q² keinen weiteren Cyclus bilden oder
B und Q¹ gemeinsam für gegebenenfalls einfach durch Methyl substituiertes C₃-C₄-Alkandiyl stehen, in welchem zwei nicht direkt benachbarte Kohlenstoffatome gegebenenfalls einen weiteren drei- bis sechsgliedrigen Cyclus bilden, oder
Q¹ für Wasserstoff, Hydroxy, Methyl, Ethyl, n-Propyl, iso-Propyl, Methoxy, Ethoxy, Propoxy, Acetyloxy oder Propionyloxy steht,
Q² für Wasserstoff, Methyl oder Ethyl steht, oder
Q¹ und Q² gemeinsam mit dem Kohlenstoff, an den sie gebunden sind, für gegebenenfalls durch Methyl, Ethyl, Methoxy, Ethoxy, Propoxy oder Butoxy substituiertes gesättigtes C₆-Cycloalkyl stehen, in welchem gegebenenfalls ein Ringglied durch Sauerstoff ersetzt ist, mit der Maßgabe, dass A und B keinen weiteren Cyclus bilden,
G für Wasserstoff (a) oder für eine der Gruppen 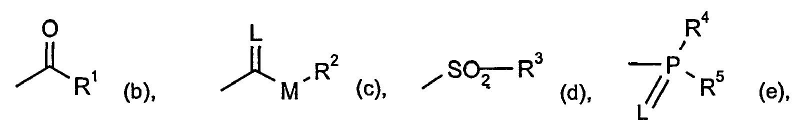 E (f) oder 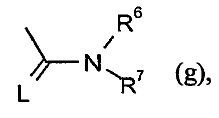 steht,
in welchen
E für ein Metallion oder ein Ammoniumion steht,
L für Sauerstoff oder Schwefel steht und
M für Sauerstoff oder Schwefel steht.
R¹ für jeweils gegebenenfalls durch Fluor oder Chlor substituiertes C₁-C₈-Alkyl, C₂-C₈-Alkenyl, C₁-C₂-Alkoxy-C₁-alkyl, C₁-Alkylthio-C₁-alkyl, Cyclopropyl, Cyclopentyl oder Cyclohexyl oder
für gegebenenfalls einfach durch Fluor, Chlor, Brom, Cyano, Nitro, Methyl, Ethyl, iso-Propyl, tert-Butyl, Methoxy, Trifluormethyl oder Trifluormethoxy substituiertes Phenyl, oder
für jeweils gegebenenfalls einfach durch Chlor, Brom oder Methyl substituiertes Thienyl oder Pyridyl steht,
R² für C₁-C₈-Alkyl, C₂-C₈-Alkenyl oder C₁-C₄-Alkoxy-C₂-alkyl, oder Cyclohexyl
oder für jeweils gegebenenfalls einfach durch Fluor, Brom, Chlor, Cyano, Nitro, Methyl, tert-Butyl, Methoxy, Trifluormethyl oder Trifluormethoxy substituiertes Phenyl oder Benzyl steht,
R³ für Methyl, Ethyl, n-Propyl, oder gegebenenfalls einfach durch Fluor, Chlor, Brom, Methyl, tert-Butyl, Methoxy, Trifluormethyl, Trifluormethoxy, Cyano oder Nitro substituiertes Phenyl steht,
R⁴ für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls einfach durch Fluor, Chlor, Brom, Nitro, Cyano, C₁-C₂-Alkoxy, Trifluormethoxy oder C₁-C₃-Alkyl substituiertes Phenyl, Phenoxy oder Phenylthio steht,
R⁵ für Methyl, Ethyl, Methoxy, Ethoxy, Methylthio oder Ethylthio steht,
R⁶ für C₁-C₄-Alkyl, C₃-C₆-Cycloalkyl, C₁-C₄-Alkoxy, C₃-C₄-Alkenyl, C₁-C₄-Alkoxy-C₁-C₄-alkyl steht,
R⁷ für Wasserstoff, C₁-C₄-Alkyl oder C₃-C₄-Alkenyl steht, oder
R⁶ und R⁷ zusammen für einen C₆-Alkylenrest stehen, in welchem gegebenenfalls eine Methylengruppe durch Sauerstoff oder Schwefel ersetzt ist.

5. Verbindungen der Formel (I) gemäß Anspruch 1, in welcher
W für Wasserstoff, Methyl, Ethyl, Chlor oder Brom steht,
X für Methyl, Ethyl, n-Propyl, Trifluormethyl oder Chlor steht
Y für Methyl, Trifluormethyl, Chlor, Brom, für gegebenenfalls einfach oder zweifach durch Chlor und/oder Methyl substituiertes Phenyl steht,
Z für Wasserstoff oder Methyl steht, mit der Maßgabe, dass W nicht für Brom steht, wenn Y für substituiertes Phenyl steht,
A für Methyl, Ethyl oder eine Bindung steht,
B für Methyl oder Ethyl steht, oder
A und B und das Kohlenstoffatom, an das sie gebunden sind für Cyclopropyl oder Cyclohexyl stehen, oder
B und Q¹ gemeinsam für gegebenenfalls einfach durch Methyl substituiertes C₄-Alkandiyl stehen,
Q¹ für Wasserstoff, Methyl, Methoxy, Ethoxy, Propoxy, Hydroxy oder Acetyloxy steht,
Q² für Wasserstoff oder Methyl steht,
G für Wasserstoff (a) oder für eine der Gruppen 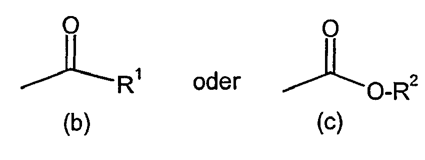 steht, wobei
R¹ für C₁-C₄-Alkyl oder für jeweils gegebenenfalls einfach durch Chlor substituiertes Phenyl oder Pyridyl steht,
R² für C₁-C₄-Alkyl steht.

6. Verfahren zur Herstellung von Verbindungen der Formel (I) gemäß Anspruch 1, **dadurch gekennzeichnet, dass** man zum Erhalt von
(A) Verbindungen der Formel (I-a) 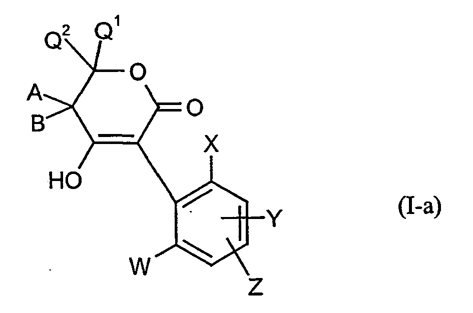 in welcher
A, B, Q¹, Q², W, X, Y und Z die oben angegebenen Bedeutungen haben,
Verbindungen der Formel (II) 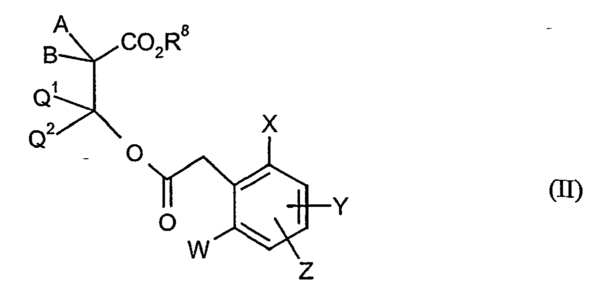 in welcher
A, B, Q¹, Q², W, X, Y und Z die oben angegebenen Bedeutungen haben,
und
R⁸ für Alkyl steht,
in Gegenwart eines Verdünnungsmittels und in Gegenwart einer Base intramolekular kondensiert;
(B) Verbindungen der Formeln (I-a) bis (I-g) 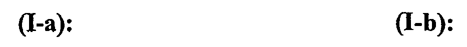 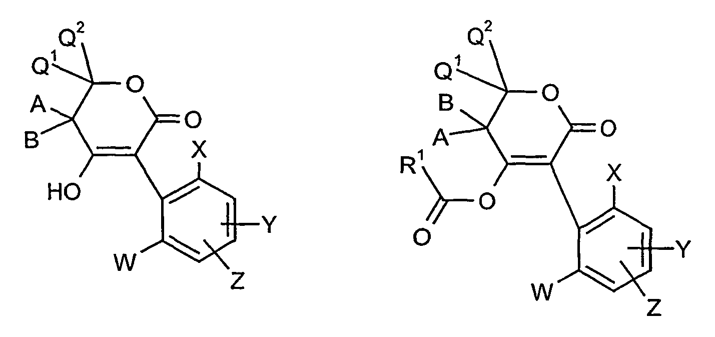 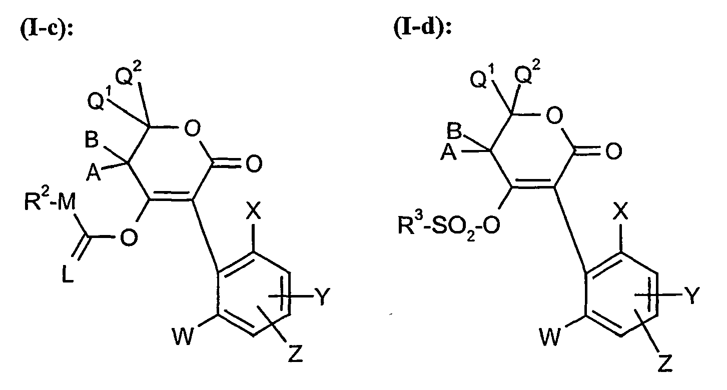 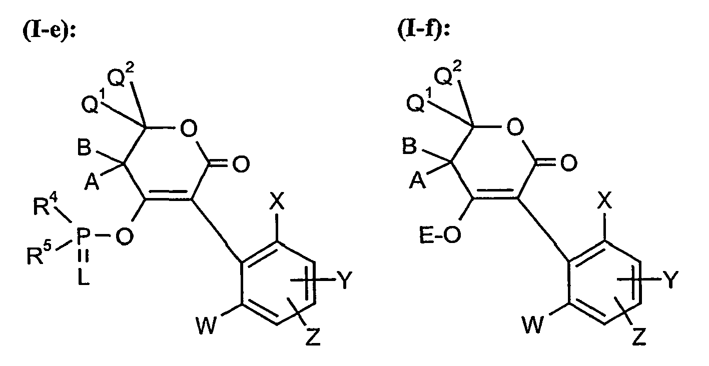 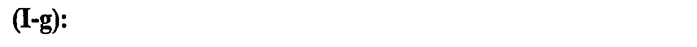 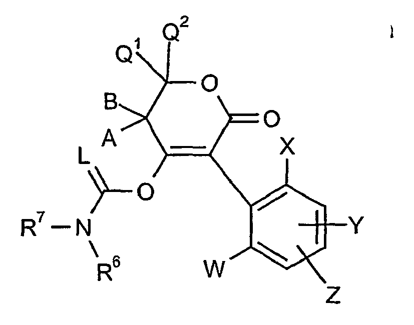 in welchen A, B, G, Q¹, Q², W, X, Y und Z die oben angegebenen Bedeutungen haben,
Verbindungen der Formel (I-a') bis (I-g'), 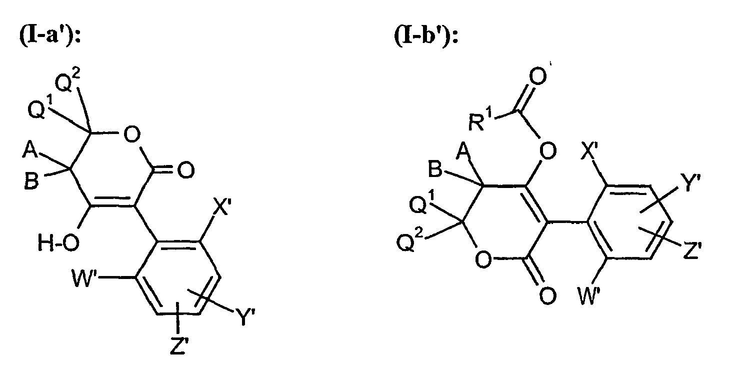 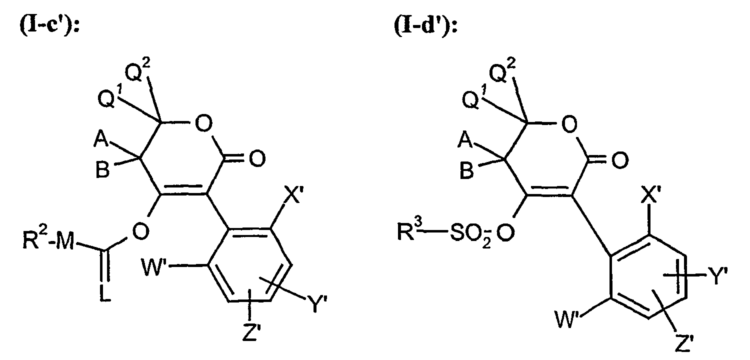 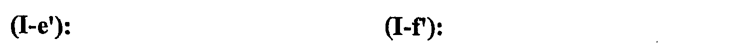 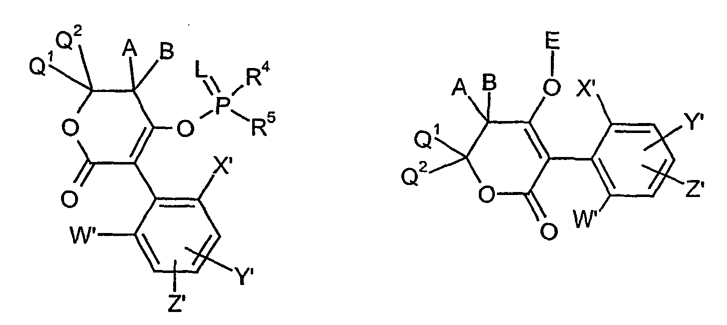 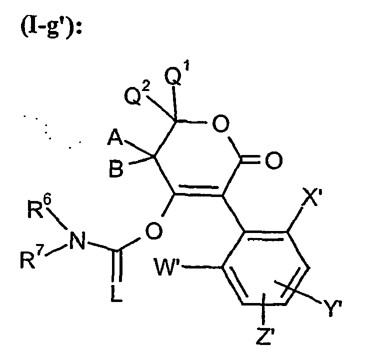 in welchen
A, B, R¹, R², R³, R⁴, R⁵, R⁶, R⁷, E, L, M, Q¹, Q², W', X', Y' und Z' die oben angegebene Bedeutung von W, X, Y und Z haben und wobei mindestens einer der Reste
W', X', Y' für Chlor, Brom oder Jod,
und Z' nicht für Brom oder Jod steht,
α) mit Verbindungen der Formel (III) 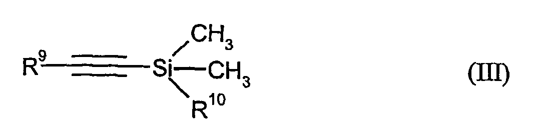 in welcher
R9 für Wasserstoff und
R¹⁰ für C₁-C₄-Alkyl oder Phenyl steht,
zunächst in Gegenwart eines Lösungsmittels, einer Base und eines Katalysators umsetzt und anschließend die Silylgruppe abspaltet,
oder
β) mit Verbindungen der Formel (IV) 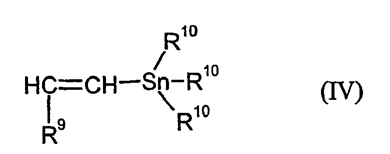 in welcher
R⁹ für Wasserstoff, Methyl oder Ethyl und
R¹⁰ für C₁-C₄-Alkyl steht,
in Gegenwart eines Lösungsmittels, gegebenenfalls in Gegenwart einer Base und in Gegenwart eines Katalysators umsetzt,
oder
γ) im speziellen Fall, wenn Y' für Chlor, Brom oder Jod und W', X' und Z' nicht für Brom oder Jod steht, mit Boronsäuren der Formel (V) 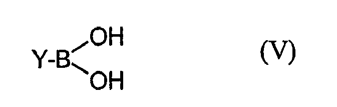 in welcher
Y für gegebenenfalls substituiertes Phenyl oder Hetaryl steht,
in Gegenwart eines Lösungsmittels, einer Base und eines Katalysators umsetzt;
(C) Verbindungen der oben gezeigten Formel (I-b), in welcher A, B, Q¹, Q², R¹, W, X, Y und Z die oben angebenen Bedeutungen haben,
Verbindungen der oben gezeigten Formel (I-a), in welchen A, B, Q¹, Q², W, X, Y und Z die oben angegebenen Bedeutungen haben, jeweils
(α) mit Verbindungen der Formel (VI) 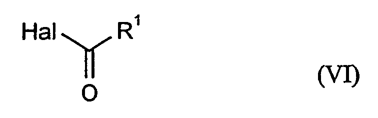 in welcher
R¹ die oben angegebene Bedeutung hat und
Hal für Halogen steht
oder
(β) mit Verbindungen der Formel (VII)
R¹-CO-O-CO-R¹ (VII)
in welcher
R¹ die oben angegebene Bedeutung hat,
gegebenenfalls in Gegenwart eines Verdünnungsmittels und gegebenenfalls in Gegenwart eines Säurebindemittels umsetzt;
(D) Verbindungen der oben gezeigten Formel (I-c), in welcher A, B, Q¹, Q², R², M, W, X, Y und Z die oben angegebenen Bedeutungen haben und L für Sauerstoff steht,
Verbindungen der oben gezeigten Formel (I-a), in welcher A, B, Q¹, Q², W, X, Y und Z die oben angegebenen Bedeutungen haben, jeweils
mit Verbindungen der Formel (VIII)
R²-M-CO-Cl (VIII)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(E) Verbindungen der oben gezeigten Formel (I-c), in welcher A, B, Q¹, Q², R², M, W, X, Y und Z die oben angegebenen Bedeutungen haben und L für Schwefel steht,
Verbindungen der oben gezeigten Formel (I-a), in welcher A, B, Q¹, Q², W, X, Y und Z die oben angegebenen Bedeutungen haben, jeweils
mit Verbindungen der Formel (IX) 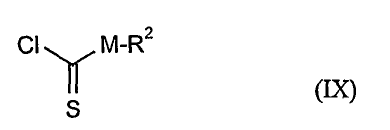 in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(F) Verbindungen der oben gezeigten Formel (I-d), in welcher A, B, Q¹, Q², R³, W, X, Y und Z die oben angegebenen Bedeutungen haben,
Verbindungen der oben gezeigten Formel (I-a), in welcher A, B, Q¹, Q², W, X, Y und Z die oben angegebenen Bedeutungen haben, jeweils
mit Verbindungen der Formel (X)
R³-SO₂-Cl (X)
in welcher
R³ die oben angegebene Bedeutung hat,
gegebenenfalls in Gegenwart eines Verdünnungsmittels und gegebenenfalls in Gegenwart eines Säurebindemittels umsetzt,
(G) Verbindungen der oben gezeigten Formel (I-e), in welcher A, B, L, Q¹, Q², R⁴, R⁵, W, X, Y und Z die oben angegebenen Bedeutungen haben,
Verbindungen der oben gezeigten Formel (I-a), in welcher A, B, Q¹, Q², W, X, Y und Z die oben angegebenen Bedeutungen haben, jeweils
mit Verbindungen der Formel (XI) 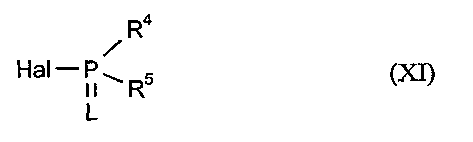 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(H) Verbindungen der oben gezeigten Formel (I-f), in welcher A, B, E, Q¹, Q², W, X, Y und Z die oben angegebenen Bedeutungen haben,
Verbindungen der Formel (I-a), in welchen A, B, Q¹, Q², W, X, Y und Z die oben angegebenen Bedeutungen haben, jeweils
mit Metallverbindungen oder Aminen der Formel (XII) oder (XIII)
Me(OR¹¹)ₜ (XII) 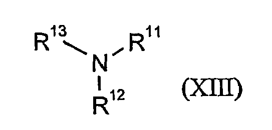
in welchen
Me für ein ein- oder zweiwertiges Metall,
t für die Zahl 1 oder 2 und
R¹¹, R¹², R¹³ unabhängig voneinander für Wasserstoff oder Alkyl stehen,
gegebenenfalls in Gegenwart eines Verdünnungsmittels umsetzt,
(I) Verbindungen der oben gezeigten Formel (I-g), in welcher A, B, L, Q¹, Q², R⁶, R⁷, W, X, Y und Z die oben angegebenen Bedeutungen haben,
Verbindungen der oben gezeigten Formel (I-a), in welcher A, B, Q¹, Q², W, X, Y und Z die oben angegebenen Bedeutungen haben, jeweils
(α) mit Verbindungen der Formel (XIV)
R⁶-N=C=L (XIV)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Verbindungen der Formel (XV) 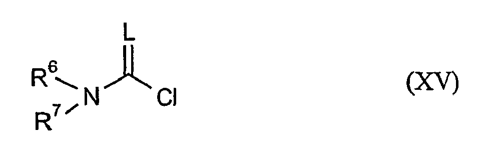
in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt;
J) Verbindungen der Formel (XVI) 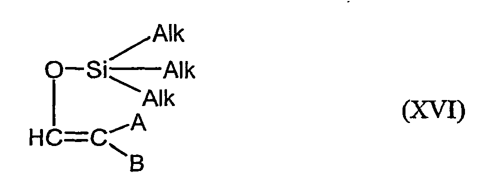 in welcher
A und B die oben angegebenen Bedeutungen haben und
Alk für Alkyl mit 1 bis 4 Kohlenstoffatomen steht,
jeweils mit Verbindungen der Formel (XVII) 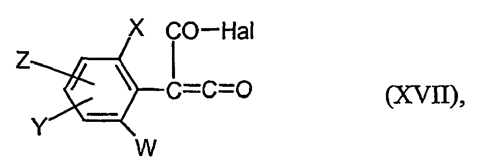 in welcher
W, X, Y und Z die oben angegebenen Bedeutungen haben und
Hal für Chlor oder Brom steht,
gegebenenfalls in Gegenwart eines Verdünnungsmittels oder eines Verdünnungsmittelgemisches und gegebenenfalls in Gegenwart eines Säurebindemittels umsetzt.

7. Verbindungen der Formel (II) 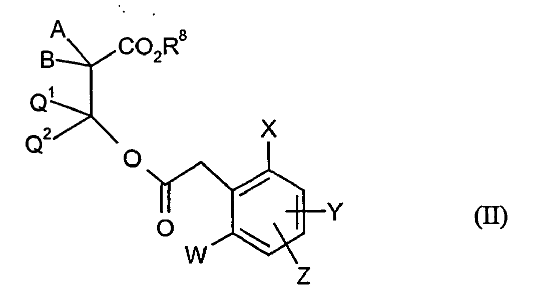 in welcher
A, B, Q¹, Q², W X, Y, Z und R⁸ die oben angegebenen Bedeutungen haben.

8. Schädlingsbekämpfungsmittel und Herbizide, **gekennzeichnet durch** einen Gehalt an mindestens einer Verbindung der Formel (I) gemäß Anspruch 1.

9. Verfahren zur Bekämpfung von tierischen Schädlingen und unerwünschten Pflanzenbewuchs, **dadurch gekennzeichnet, daß** man Verbindungen der Formel (I) gemäß Anspruch 1 auf Schädlinge und/oder ihren Lebensraum einwirken läßt.

10. Verwendung von Verbindungen der Formel (I) gemäß Anspruch 1 zur Bekämpfung von tierischen Schädlingen und unerwünschtem Pflanzenbewuchs.

11. Verfahren zur Herstellung von Schädlingsbekämpfungsmitteln und Herbiziden, **dadurch gekennzeichnet, daß** man Verbindungen der Formel (I) gemäß Anspruch 1 mit Streckmitteln und/oder oberflächenaktiven Stoffen vermischt.

12. Verwendung von Verbindungen der Formel (I) gemäß Anspruch 1 zur Herstellung von Schädlingsbekämpfungsmitteln und.Herbiziden.

## Claims

1. Compounds of the formula (I) 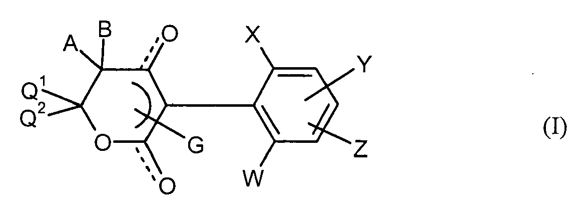 in which
W represents hydrogen, alkyl, alkenyl, alkinyl, halogen, halogenoalkyl or alkoxy,
X represents halogen, alkyl, alkoxy, alkenyl, alkinyl, halogenoalkyl, halogenoalkoxy, cyano or in each case optionally substituted phenyl, phenoxy, phenylthio, phenylalkoxy or phenylalkylthio,
Y represents hydrogen, alkyl, halogen, halogenoalkyl, alkoxy, alkenyl, alkinyl or optionally substituted aryl or hetaryl,
Z represents hydrogen, halogen, alkyl, alkoxy, halogenoalkyl, halogenoalkoxy or cyano,
A represents a bond, hydrogen, in each case optionally halogen-substituted alkyl, alkenyl, alkoxyalkyl, optionally substituted cycloalkyl or cycloalkylalkyl in which optionally at least one ring atom is replaced by a hetero atom, or in each case optionally halogen-, alkyl-, halogenoalkyl-, alkoxy-, halogenoalkoxy-, cyano- or nitrosubstituted aryl, arylalkyl, hetaryl or hetarylalkyl,
B represents hydrogen or alkyl, or
A and B together with the carbon atom to which they are attached represent a saturated or unsaturated unsubstituted or substituted cycle which optionally contains at least one hetero atom, or
B and Q¹ together represent alkanediyl which is optionally substituted by in each case optionally substituted alkyl or alkoxy and in which two not directly adjacent carbon atoms optionally form a further optionally substituted cycle or
Q¹ represents hydrogen, hydroxyl, alkyl, alkoxy, alkoxyalkyl, alkylacyloxy, optionally substituted cycloalkyl (in which optionally one methylene group is replaced by oxygen or sulphur) or optionally substituted phenyl,
Q² represents hydrogen or alkyl, or
Q¹ and Q² together with the carbon atom to which they are attached represent an unsubstituted or substituted cycle which optionally contains a hetero atom,
G represents hydrogen (a) or represents one of the groups 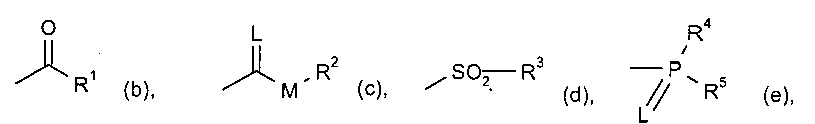 E (f) or 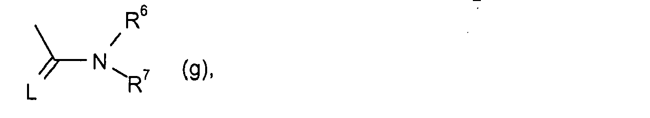 in which
E represents a metal ion or an ammonium ion,
L represents oxygen or sulphur,
M represents oxygen or sulphur,
R¹ represents in each case optionally halogen-substituted alkyl, alkenyl, alkoxyalkyl, alkylthioalkyl, polyalkoxyalkyl or optionally halogen-, alkyl- or alkoxy-substituted cycloalkyl in which one or more methylene groups may be replaced by hetero atoms, in each case optionally substituted phenyl, phenylalkyl, hetaryl, phenoxyalkyl or hetaryloxyalkyl,
R² represents in each case optionally halogen-substituted alkyl, alkenyl, alkoxyalkyl, polyalkoxyalkyl or represents in each case optionally substituted cycloalkyl, phenyl or benzyl,
R³, R⁴ and R⁵ independently of one another each represent in each case optionally halogen-substituted alkyl, alkoxy, alkylamino, dialkylamino, alkylthio, alkenylthio, cycloalkylthio and represent in each case optionally substituted phenyl, benzyl, phenoxy or phenylthio, and
R⁶ and R⁷ independently of one another each represent hydrogen, in each case optionally halogen-substituted alkyl, cycloalkyl, alkenyl, alkoxy, alkoxyalkyl, represent optionally substituted phenyl, represent optionally substituted benzyl, or together with the N atom to which they are attached represent a ring which is optionally interrupted by oxygen or sulphur.

2. Compounds of the formula (I) according to Claim 1 in which
W represents hydrogen, C₁-C₆-alkyl, C₂-C₄-alkenyl, ethinyl, fluorine, chlorine, bromine, C₁-C₄-halogenoalkyl or C₁-C₆-alkoxy,
X represents fluorine, chlorine, bromine, C₁-C₆-alkyl, C₁-C₄-halogenoalkyl, C₁-C₆-alkoxy, C₂-C₄-alkenyl, ethinyl, C₁-C₄-halogenoalkoxy, cyano or in each case optionally halogen-, C₁-C₆-alkyl-, C₁-C₆-alkoxy-, C₁-C₄-halogenoalkyl-, C₁-C₄-halogenoalkoxy-, nitro-or cyano-substituted phenyl or benzyloxy,
Y represents hydrogen, C₁-C₆-alkyl, C₁-C₄-halogenoalkyl, fluorine, chlorine, bromine, C₁-C₆-alkoxy, C₂-C₄-alkenyl, ethinyl or represents one of the radicals 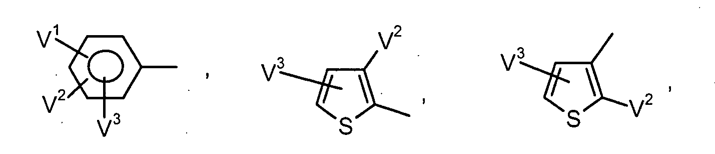 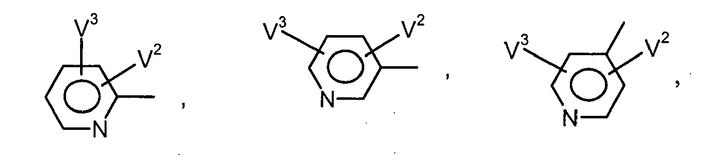
V¹ represents hydrogen, halogen, C₁-C₁₂-alkyl, C₁-C₆-alkoxy, C₁-C₆-alkylthio, C₁-C₄-halogenoalkyl, C₁-C₄-halogenoalkoxy, nitro, cyano or represents phenyl, phenoxy, phenoxy-C₁-C₄-alkyl, phenylC₁-C₄-alkoxy, phenylthio-C₁-C₄-alkyl or phenyl-C₁-C₄-alkylthio, each of which is optionally mono- or polysubstituted by halogen, C₁-C₆-alkyl, C₁-C₆-alkoxy, C₁-C₄-halogenoalkyl, C₁-C₄-halogenoalkoxy, nitro or cyano,
V² represents hydrogen, fluorine, chlorine, C₁-C₆-alkyl, C₁-C₆-alkoxy, C₁-C₄-halogenoalkyl or C₁-C₄-halogenoalkoxy,
V³ represents hydrogen, fluorine, chlorine, methyl or methoxy,
Z represents hydrogen, fluorine, chlorine, bromine, C₁-C₆-alkyl, C₁-C₄-halogenoalkyl, C₁-C₆-alkoxy, C₁-C₄-halogenoalkoxy or cyano,
with the first proviso, that W, X and Z do not represent bromine, C₂-C₄-alkenyl and ethinyl if Y represents V¹-, V²- and V³-substituted phenyl or hetaryl and that secondly only at most two of the radicals W, X and Y represent C₂-C₄-alkenyl or ethinyl, with the proviso that none of the other radicals W, X, Y and Z may represent bromine,
A represents a bond, hydrogen or in each case optionally halogen-substituted C₁-C₁₂-alkyl, C₃-C₈-alkenyl, C₁-C₆-alkoxy-C₁-C₄-alkyl, in each case optionally halogen-, C₁-C₄-alkyl- or C₁-C₄-alkoxy-substituted C₃-C₈-cycloalkyl or C₃-C₆-cycloalkyl-C₁-C₄-alkyl in which optionally one or two not directly adjacent ring members are replaced by oxygen and/or sulphur or represents in each case optionally halogen-, C₁-C₆-alkyl-, C₁-C₆-halogenoalkyl-, C₁-C₆-alkoxy-, C₁-C₆-halogenoalkoxy-, cyano- or nitro-substituted phenyl, benzyl, hetaryl having 5 or 6 ring atoms or hetaryl-C₁-C₄-alkyl having 5 or 6 ring atoms,
B represents hydrogen or C₁-C₆-alkyl, or
A, B and the carbon atom to which they are attached represent saturated C₃-C₁₀-cycloalkyl or unsaturated C₅-C₁₀-cycloalkyl in which optionally one ring member is replaced by oxygen or sulphur and which are optionally mono- or disubstituted by C₁-C₆-alkyl, C₃-C₈-cycloalkyl, C₁-C₆-halogenoalkyl, C₁-C₆-alkoxy, C₁-C₆-alkylthio, halogen or phenyl, with the proviso that Q¹ and Q² do not form a further cycle, or
B and Q¹ together represent C₃-C₆-alkanediyl which is optionally mono- or disubstituted by identical or different C₁-C₄-alkyl and in which two not directly adjacent carbon atoms optionally form a further 3- to 6-membered cycle, or
Q¹ represents hydrogen, hydroxyl, C₁-C₆-alkyl, C₁-C₆-alkoxy, C₁-C₆-alkoxy-C₁-C₂-alkyl, C₁-C₆-alkylacyloxy, optionally fluorine-, chlorine-, C₁-C₄-alkyl-, C₁-C₂-halogenoalkyl- or C₁-C₄-alkoxysubstituted C₃-C₈-cycloalkyl in which optionally one methylene group is replaced by oxygen or sulphur or optionally halogen, C₁-C₄-alkyl-, C₁-C₄-alkoxy-, C₁-C₂-halogenoalkyl-, C₁-C₂-halogenoalkoxy-, cyano- or nitro-substituted phenyl,
Q² represents hydrogen or C₁-C₄-alkyl, or
Q¹ and Q² together with the carbon atom to which they are attached represent optionally C₁-C₆-alkyl-, C₁-C₆-alkoxy- or C₁-C₂-halogenoalkylsubstituted C₃-C₇-cycloalkyl in which optionally one ring member is replaced by oxygen or sulphur, with the proviso that A and B do not form a further cycle,
G represents hydrogen (a) or represents one of the groups 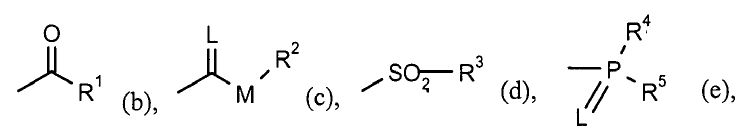 E (f) or 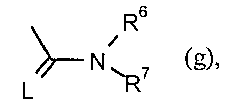 in which
E represents a metal ion or an ammonium ion,
L represents oxygen or sulphur and
M represents oxygen or sulphur,
R¹ represents in each case optionally halogen-substituted C₁-C₂₀-alkyl, C₂-C₂₀-alkenyl, C₁-C₈-alkoxy-C₁-C₈-alkyl, C₁-C₈-alkylthio-C₁-C₈-alkyl, ploy-C₁-C₈-alkoxy-C₁-C₈-alkyl or optionally halogen-, C₁-C₆-alkyl- or C₁-C₆-alkoxy-substituted C₃-C₈-cycloalkyl in which optionally one or more not directly adjacent ring members are replaced by oxygen and/or sulphur, or
represents optionally halogen-, cyano-, nitro-, C₁-C₆-alkyl-, C₁-C₆-alkoxy-, C₁-C₆-halogenoalkyl-, C₁-C₆-halogenoalkoxy-, C₁-C₆-alkylthio- or C₁-C₆-alkylsulphonyl-substituted phenyl, or
represents optionally halogen-, nitro-, cyano-, C₁-C₆-alkyl-, C₁-C₆-alkoxy-, C₁-C₆-halogenoalkyl- or C₁-C₆-halogenoalkoxy-substituted phenyl-C₁-C₆-alkyl, or
represents optionally halogen-, C₁-C₆-alkyl- or trifluoromethyl-substituted 5- or 6-membered hetaryl or
represents optionally halogen- or C₁-C₆-alkyl-substituted phenoxy-C₁-C₆-alkyl or
represents optionally halogen-, amino- or C₁-C₆-alkyl-substituted 5-or 6-membered hetaryloxy-C₁-C₆-alkyl,
R² represents in each case optionally halogen-substituted C₁-C₂₀-alkyl, C₂-C₂₀-alkenyl, C₁-C₈-alkoxy-C₂-C₈-alkyl, poly-C₁-C₈-alkoxy-C₂-C₈-alkyl, or
represents optionally halogen-, C₁-C₆-alkyl- or C₁-C₆-alkoxy-substituted C₃-C₈-cycloalkyl or
represents in each case optionally halogen-, cyano-, nitro-, C₁-C₆-alkyl-, C₁-C₆-alkoxy-, C₁-C₆-halogenoalkyl- or C₁-C₆-halogenoalkoxy-substituted phenyl or benzyl,
R³ represents optionally halogen-substituted C₁-C₈-alkyl or represents in each case optionally halogen-, C₁-C₆-alkyl-, C₁-C₆-alkoxy-, C₁-C₄-halogenoalkyl-, C₁-C₄₋halogenoalkoxy-, cyano- or nitro-substituted phenyl or benzyl,
R⁴ and R⁵ independently of one another each represent in each case optionally halogen-substituted C₁-C₈-alkyl, C₁-C₈-alkoxy, C₁-C₈-alkylamino, di-(C₁-C₈-alkyl)amino, C₁-C₈-alkylthio, C₂-C₈-alkenylthio, C₃-C₇-cycloalkylthio or represent in each case optionally halogen-, nitro-, cyano-, C₁-C₄-alkoxy-, C₁-C₄-halogenoalkoxy-, C₁-C₄-alkylthio-, C₁-C₄-halogenoalkylthio-, C₁-C₄-alkyl- or C₁-C₄-halogenoalkyl-substituted phenyl, benzyl, phenoxy or phenylthio,
R⁶ and R⁷ independently of one another each represent hydrogen, represent in each case optionally halogen-substituted C₁-C₈-alkyl, C₃-C₈-cycloalkyl, C₁-C₈-alkoxy, C₃-C₈-alkenyl, C₁-C₈-alkoxy-C₁-C₈-alkyl, represent optionally halogen-, C₁-C₈-halogenoalkyl-, C₁-C₈-alkyl- or C₁-C₈-alkoxy-substituted phenyl, optionally halogen-, C₁-C₈-alkyl-, C₁-C₈-halogenoalkyl- or C₁-C₈-alkoxy-substituted benzyl or together represent an optionally C₁-C₄-alkyl-substituted C₃-C₆-alkylene radical in which optionally one carbon atom is replaced by oxygen or sulphur.

3. Compounds of the formula (I) according to Claim 1 in which
W represents hydrogen, C₁-C₄-alkyl, chlorine or bromine,
X represents chlorine, bromine, C₁-C₄-alkyl, C₁-C₄-alkoxy, C₂-C₃-alkenyl, ethinyl, C₁-C₂-halogenoalkyl, C₁-C₂-halogenoalkoxy or cyano,
Y represents hydrogen, C₁-C₄-alkyl, C₁-C₂-halogenoalkyl, fluorine, chlorine, bromine, C₁-C₄-alkoxy, C₂-C₃-alkenyl, ethinyl, 2-thienyl, 3-thienyl or represents the radical 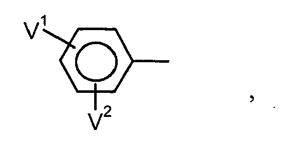
V¹ represents hydrogen, fluorine, chlorine, bromine, C₁-C₆-alkyl, C₁-C₄-alkoxy, C₁-C₄-alkylthio, C₁-C₂-halogenoalkyl, C₁-C₂-halogenoalkoxy, nitro, cyano or phenyl,
V² represents hydrogen, fluorine, chlorine, C₁-C₄-alkyl, C₁-C₄-alkoxy or C₁-C₂-halogenoalkyl,
Z represents hydrogen, fluorine, chlorine, bromine, C₁-C₄-alkyl, C₁-C₂-halogenoalkyl, C₁-C₄-alkoxy or C₁-C₂-halogenoalkoxy,
with the first proviso that W, X and Z do not represent bromine, C₂-C₃-alkenyl and ethinyl if Y represents V¹- and V²-substituted phenyl, 2-thienyl or 3-thienyl and that secondly only one of the radicals X and Y represents C₂-C₃-alkenyl and ethinyl, with the proviso that in this case none of the other radicals W, X, Y and Z may represent bromine,
A represents a bond, hydrogen, in each case optionally fluorine-substituted C₁-C₈-alkyl, C₁-C₄-alkoxy-C₁-C₂-alkyl, in each case optionally fluorine-, chlorine-, methyl-, ethyl- or methoxy-substituted C₅-C₆-cycloalkyl or C₃-C₆-cycloalkyl-C₁-C₂-alkyl in which optionally one ring member is replaced by oxygen or sulphur or in each case optionally fluorine-, chlorine-, bromine-, C₁-C₄-alkyl-, C₁-C₂-halogenoalkyl-, C₁-C₄-alkoxy- or C₁-C₂-halogenoalkoxy-substituted phenyl or benzyl,
B represents hydrogen or C₁-C₄-alkyl, or
A, B and the carbon atom to which they are attached represent saturated C₅-C₇-cycloalkyl in which optionally one ring member is replaced by oxygen and which is optionally monosubstituted by C₁-C₄-alkyl, trifluoromethyl or C₁-C₄-alkoxy, with the proviso that Q¹ and Q² do not form a further cycle, or
B and Q¹ together represent C₃-C₄-alkanediyl which is optionally monosubstituted by C₁-C₂-alkyl and in which two not directly adjacent carbon atoms optionally form a further five- or six-membered cycle, or
Q¹ represents hydrogen, hydroxyl, C₁-C₄-alkyl, C₁-C₄-alkoxy, C₁-C₄-alkoxy-C₁-C₂-alkyl, C₁-C₄-alkylacyloxy or optionally methyl- or methoxy-substituted C₃-C₆-cycloalkyl in which optionally one methylene group is replaced by oxygen,
Q² represents hydrogen, methyl or ethyl, or
Q¹ and Q² together with the carbon to which they are attached represent optionally C₁-C₄-alkyl-, trifluoromethyl- or C₁-C₄-alkoxy-substituted saturated C₅-C₆-cycloalkyl in which optionally one ring member is replaced by oxygen, with the proviso that A and B do not form a further cycle,
G represents hydrogen (a) or represents one of the groups 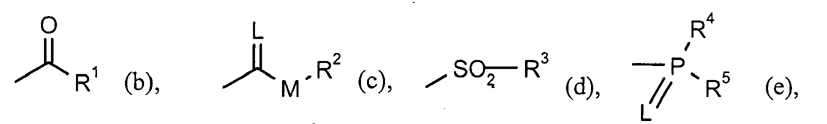 E (f) or 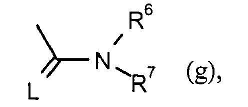 in which
E represents a metal ion or an ammonium ion,
L represents oxygen or sulphur and
M represents oxygen or sulphur,
R¹ represents in each case optionally fluorine- or chlorine-substituted C₁-C₁₆-alkyl, C₂-C₁₆-alkenyl, C₁-C₄-alkoxy-C₁-C₂-alkyl, C₁-C₄-alkylthio-C₁-C₂-alkyl, or optionally fluorine-, chlorine-, C₁-C₂-alkyl-, or C₁-C₂-alkoxy-substituted C₃-C₇-cycloalkyl in which optionally one or two not directly adjacent ring members are replaced by oxygen and/or sulphur, or
represents phenyl which is optionally mono- or disubstituted by fluorine, chlorine, bromine, cyano, nitro, C₁-C₄-alkyl, C₁-C₄-alkoxy, trifluoromethyl or trifluoromethoxy, or
represents pyridyl or thienyl, each of which is optionally monosubstituted by fluorine, chlorine, bromine, methyl, ethyl or trifluoromethyl,
R² represents C₁-C₁₆-alkyl, C₂-C₁₆-alkenyl or C₁-C₄-alkoxy-C₂-C₄-alkyl, each of which is optionally mono- to trisubstituted by fluorine, or
represents C₃-C₇-cycloalkyl which is optionally monosubstituted by methyl, ethyl or methoxy, or
represents phenyl or benzyl, each of which is optionally mono- or disubstituted by fluorine, chlorine bromine, cyano, nitro, C₁-C₄-alkyl, C₁-C₃-alkoxy, trifluoromethyl or trifluoromethoxy,
R³ represents C₁-C₆-alkyl which is optionally mono- to pentasubstituted by fluorine or represents phenyl which is optionally mono- or disubstituted by fluorine, chlorine, bromine, C₁-C₄-alkyl, C₁-C₄-alkoxy, trifluoromethyl, trifluoromethoxy, cyano or nitro,
R⁴ represents C₁-C₆-alkyl, C₁-C₆-alkoxy, C₁-C₆-alkylamino, di-(C₁-C₆-alkyl)amino, C₁-C₆-alkylthio, or represents phenyl, benzyl, phenoxy or phenylthio, each of which is optionally mono- or disubstituted by fluorine, chlorine, bromine, nitro, cyano, C₁-C₃-alkoxy, trifluoromethoxy, C₁-C₃-alkyl or trifluoromethyl,
R⁵ represents C₁-C₄-alkyl, C₁-C₄-alkoxy or C₁-C₄-alkylthio,
R⁶ represents C₁-C₆-alkyl, C₃-C₆-cycloalkyl, C₁-C₆-alkoxy, C₃-C₆-alkenyl, C₁-C₆-alkoxy-C₁-C₆-alkyl, or represents phenyl which is optionally mono- or disubstituted by fluorine, chlorine, bromine, trifluoromethyl, C₁-C₄-alkyl or C₁-C₄-alkoxy, or represents benzyl which is optionally mono- or disubstituted by fluorine, chlorine, bromine, methyl, ethyl, trifluoromethyl or methoxy,
R⁷ represents hydrogen, C₁-C₆-alkyl or C₃-C₆-alkenyl, or
R⁶ and R⁷ together represent a C₄-C₅-alkylene radical which is optionally mono- or disubstituted by methyl or ethyl and in which optionally one methylene group is replaced by oxygen or sulphur.

4. Compounds of the formula (I) according to Claim 1, in which
W represents hydrogen, chlorine, bromine, methyl or ethyl,
X represents chlorine, bromine, methyl, ethyl, n-propyl, methoxy, ethoxy, trifluoromethyl, difluoromethoxy, trifluoromethoxy or cyano,
Y represents hydrogen, methyl, ethyl, propyl, iso-propyl, fluorine, chlorine, bromine, methoxy or represents the radical 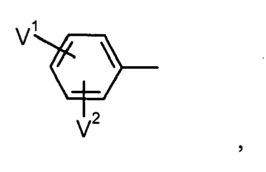
V¹ represents hydrogen, fluorine, chlorine, bromine, methyl, ethyl, isopropyl, tert-butyl, methoxy, trifluoromethyl or trifluoromethoxy, cyano or phenyl,
V² represents hydrogen, fluorine, chlorine, methyl, methoxy or trifluoromethyl,
Z represents hydrogen, fluorine, chlorine, bromine, methyl, methoxy or trifluoromethyl, with the proviso that W, X and Z do not represent bromine if Y represents V¹- and V²-substituted phenyl,
A represents a bond, hydrogen, methyl, ethyl, n-propyl, iso-propyl, n-butyl or iso-butyl,
B represents hydrogen, methyl or ethyl, or
A, B and the carbon atoms to which they are attached represent saturated C₅-C₆-cycloalkyl in which optionally one ring member is replaced by oxygen and which is optionally monosubstituted by methyl, ethyl, methoxy or ethoxy, with the proviso that Q¹ and Q² do not form a further cycle or
B and Q¹ together represent C₃-C₄-alkanediyl which is optionally monosubstituted by methyl and in which two not directly adjacent carbon atoms optionally form a further three- to six-membered cycle, or
Q¹ represents hydrogen, hydroxyl, methyl, ethyl, n-propyl, iso-propyl, methoxy, ethoxy, propoxy, acetyloxy or propionyloxy,
Q² represents hydrogen, methyl or ethyl, or
Q¹ and Q² together with the carbon to which they are attached represent saturated C₆-cycloalkyl which is optionally substituted by methyl, ethyl, methoxy, ethoxy, propoxy or butoxy and in which optionally one ring member is replaced by oxygen, with the proviso that A and B do not form a further cycle,
G represents hydrogen (a) or represents one of the groups 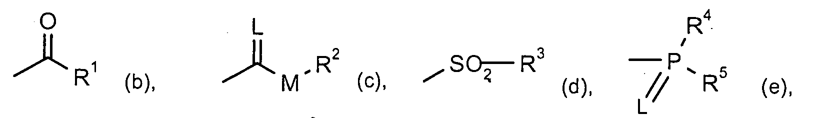 E (f) or 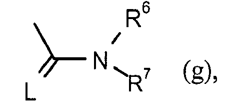 in which
E represents a metal ion or an ammonium ion,
L represents oxygen or sulphur and
M represents oxygen or sulphur,
R¹ represents in each case optionally fluorine- or chlorine-substituted C₁-C₈-alkyl, C₂-C₈-alkenyl, C₁-C₂-alkoxy-C₁-alkyl, C₁-alkylthio-C₁-alkyl, cyclopropyl, cyclopentyl or cyclohexyl or
represents phenyl which is optionally monosubstituted by fluorine, chlorine, bromine, cyano, nitro, methyl, ethyl, iso-propyl, tert-butyl, methoxy, trifluoromethyl or trifluoromethoxy, or
represents thienyl or pyridyl, each of which is optionally monosubstituted by chlorine, bromine or methyl,
R² represents C₁-C₈-alkyl, C₂-C₈-alkenyl or C₁-C₄-alkoxy-C₂-alkyl, or cyclohexyl
or represents phenyl or benzyl, each of which is optionally monosubstituted by fluorine, bromine, chlorine, cyano, nitro, methyl, tert-butyl, methoxy, trifluoromethyl or trifluoromethoxy,
R³ represents methyl, ethyl, n-propyl, or phenyl which is optionally monosubstituted by fluorine, chlorine, bromine, methyl, tert-butyl, methoxy, trifluoromethyl, trifluoromethoxy, cyano or nitro,
R⁴ represents C₁-C₄-alkyl, C₁-C₄-alkoxy, C₁-C₄-alkylamino, di-(C₁-C₄-alkyl)amino, C₁-C₄-alkylthio or represents phenyl, phenoxy or phenylthio, each of which is optionally monosubstituted by fluorine, chlorine, bromine, nitro, cyano, C₁-C₂-alkoxy, trifluoromethoxy or C₁-C₃-alkyl,
R⁵ represents methyl, ethyl, methoxy, ethoxy, methylthio or ethylthio,
R⁶ represents C₁-C₄-alkyl, C₃-C₆-cycloalkyl, C₁-C₄-alkoxy, C₃-C₄-alkenyl, C₁-C₄-alkoxy-C₁-C₄-alkyl,
R⁷ represents hydrogen, C₁-C₄-alkyl or C₃-C₄-alkenyl, or
R⁶ and R⁷ together represent a C₆-alkylene radical in which optionally one methylene group is replaced by oxygen or sulphur.

5. Compounds of the formula (I) according to Claim 1, in which
W represents hydrogen, methyl, ethyl, chlorine or bromine,
X represents methyl, ethyl, n-propyl, trifluoromethyl or chlorine,
Y represents methyl, trifluoromethyl, chlorine, bromine, represents phenyl which is optionally mono- or disubstituted by chlorine and/or methyl,
Z represents hydrogen or methyl, with the proviso that W does not represent bromine if Y represents substituted phenyl,
A represents methyl, ethyl or a bond,
B represents methyl or ethyl, or
A and B and the carbon atom to which they are attached represent cyclopropyl or cyclohexyl, or
B and Q¹ together represent C₄-alkanediyl which is optionally monosubstituted by methyl,
Q¹ represents hydrogen, methyl, methoxy, ethoxy, propoxy, hydroxyl or acetyloxy,
Q² represents hydrogen or methyl,
G represents hydrogen (a) or represents one of the groups 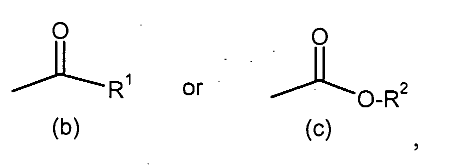 where
R¹ represents C₁-C₄-alkyl or represents phenyl or pyridyl, each of which is optionally monosubstituted by chlorine,
R² represents C₁-C₄-alkyl.

6. Process for preparing compounds of the formula (I) according to Claim 1, **characterized in that** to obtain
(A) compounds of the formula (I-a) 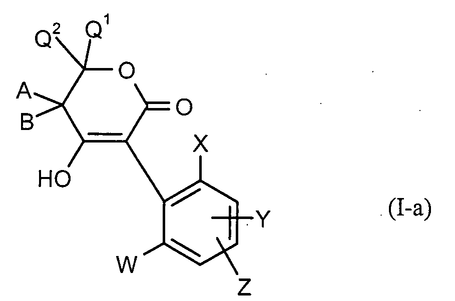 in which
A, B, Q¹, Q², W, X, Y and Z are each as defined above,
compounds of the formula (II) 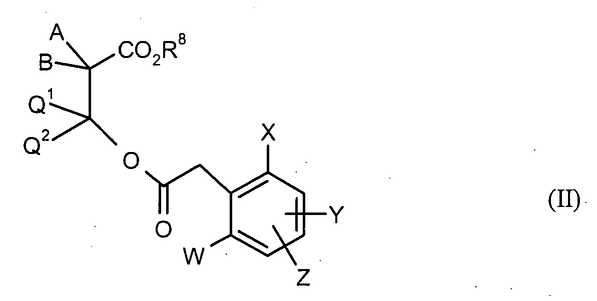 in which
A, B, Q¹, Q², W, X, Y and Z are each as defined above,
and
R⁸ represents alkyl,
are condensed intramolecularly in the presence of a diluent and in the presence of a base;
(B) compounds of the formulae (I-a) to (I-g) 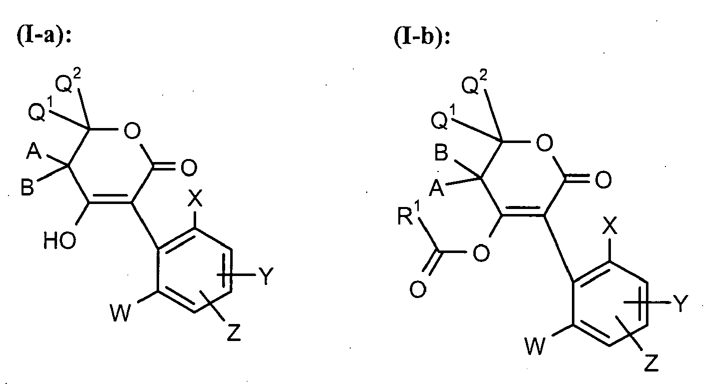 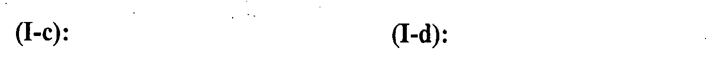 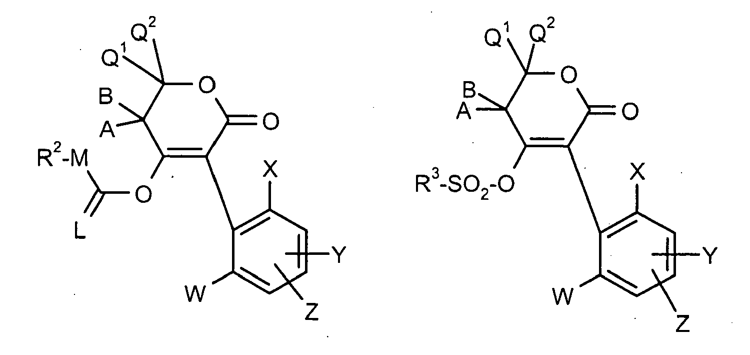 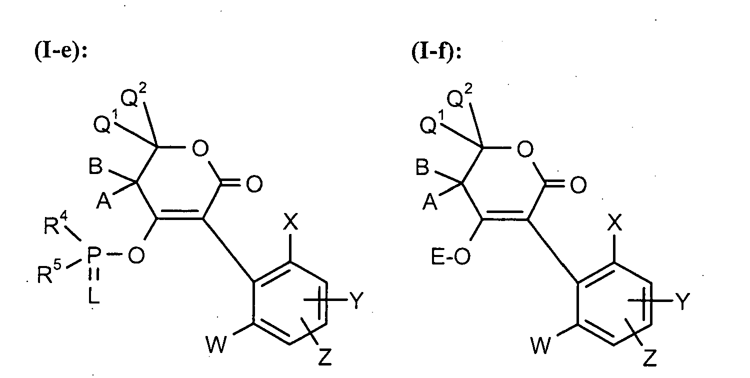 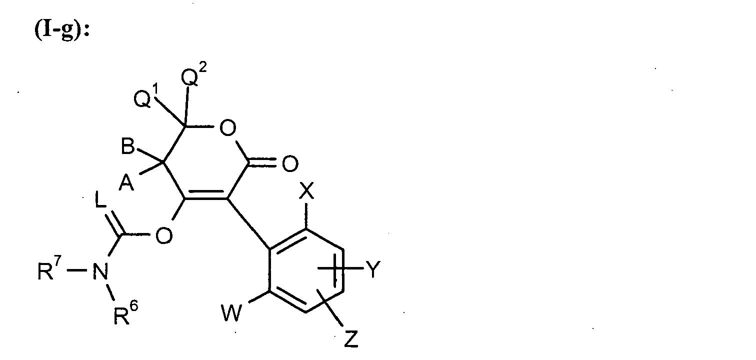 in which A, B, G, Q¹, Q², W, X, Y and Z are each as defined above,
compounds of the formulae (I-a') to (I-g'), 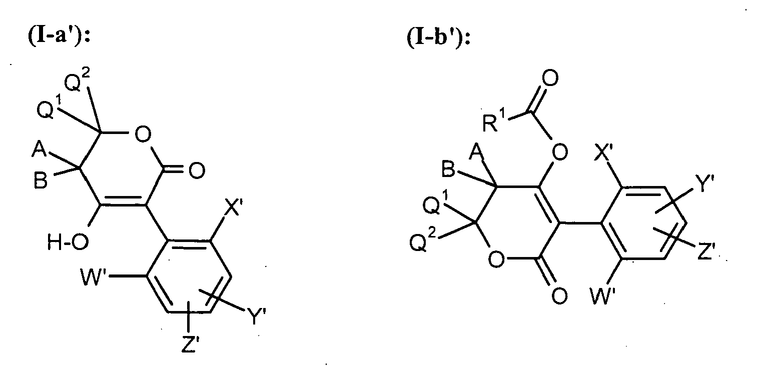 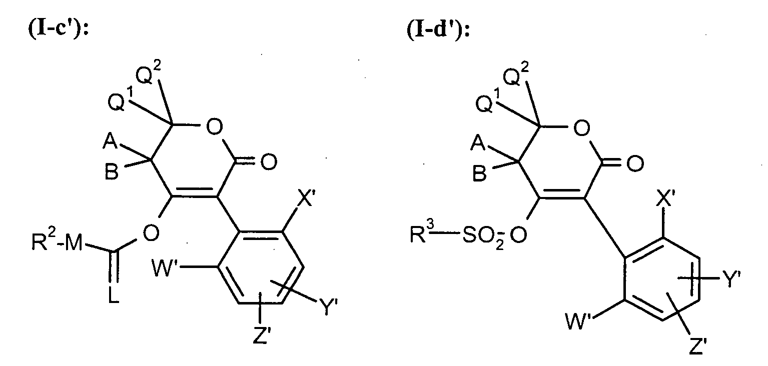 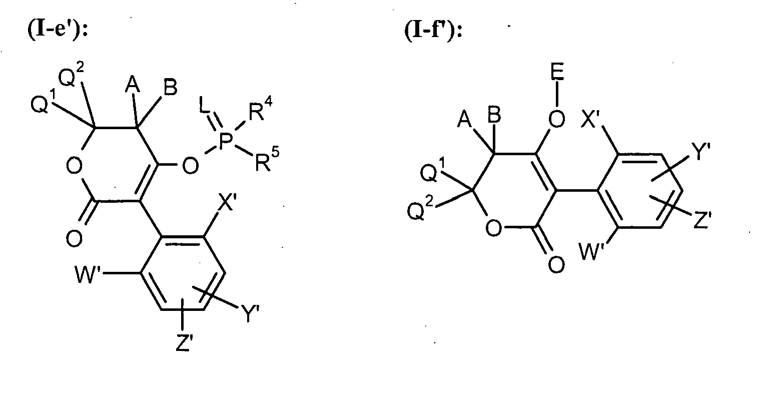 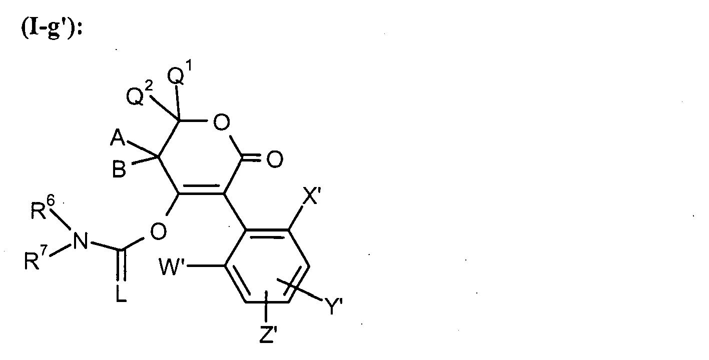 in which
A, B, R¹, R², R³, R⁴, R⁵, R⁶, R⁷, E, L, M, Q¹, Q², W', X', Y' and Z' each have the meanings of W, X, Y and Z given above and where at least one of the radicals
W', X', Y' represents chlorine, bromine or iodine,
and Z' does not represent bromine or iodine,
α) are initially reacted with compounds of the formula (III) 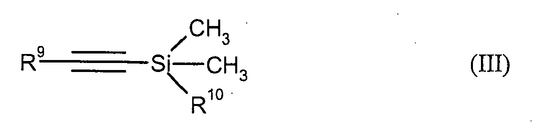 in which
R⁹ represents hydrogen and
R¹⁰ represents C₁-C₄-alkyl or phenyl,
in the presence of a solvent, a base and a catalyst, and the silyl group is then removed,
or
β) are reacted with compounds of the formula (IV) 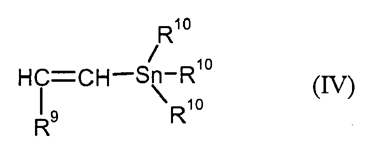 in which
R⁹ represents hydrogen, methyl or ethyl and
R¹⁰ represents C₁-C₄-alkyl,
in the presence of a solvent, if appropriate in the presence of a base and in the presence of a catalyst,
or
y) in the specific case where Y' represents chlorine, bromine or iodine and W', X' and Z' do not represent bromine or iodine, are reacted with boronic acids of the formula (V) 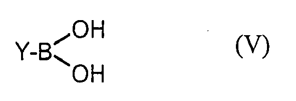 in which
Y represents optionally substituted phenyl or hetaryl,
in the presence of a solvent, a base and a catalyst;
(C) compounds of the formula (I-b) shown above in which A, B, Q¹, Q², R¹, W, X, Y and Z are each as defined above,
compounds of the formula (I-a) shown above in which A, B, Q¹, Q², W, X, Y and Z are each as defined above are in each case reacted
(α) with compounds of the formula (VI) 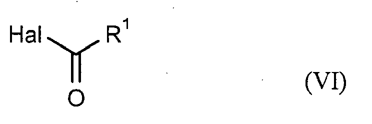 in which
R¹ is as defined above and
Hal represents halogen
or
(β) with compounds of the formula (VII)
R¹-CO-O-CO-R¹ (VII)
in which
R¹ is as defined above,
if appropriate in the presence of a diluent and if appropriate in the presence of an acid binder;
(D) compounds of the formula (I-c) shown above in which A, B, Q¹, Q², R², M, W, X, Y and Z are each as defined above and L represents oxygen,
compounds of the formula (I-a) shown above in which A, B, Q¹, Q², W, X, Y and Z are each as defined above are in each case reacted
with compounds of the formula (VIII)
R²-M-CO-Cl (VIII)
in which
R² and M are each as defined above,
if appropriate in the presence of a diluent and if appropriate in the presence of an acid binder;
(E) compounds of the formula (I-c) shown above in which A, B, Q¹, Q², R², M, W, X, Y and Z are each as defined above and L represents sulphur,
compounds of the formula (I-a) shown above in which A, B, Q¹, Q², W, X, Y and Z are each as defined above, are in each case reacted
with compounds of the formula (IX) 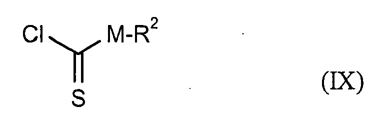 in which
M and R² are each as defined above,
if appropriate in the presence of a diluent and if appropriate in the presence of an acid binder
and
(F) compounds of the formula (I-d) shown above in which A, B, Q¹, Q², R³, W, X, Y and Z are each as defined above,
compounds of the formula (I-a) shown above in which A, B, Q¹, Q², W, X, Y and Z are each as defined above are in each case reacted
with compounds of the formula (X)
R³-SO₂-Cl (X)
in which
R³ is as defined above,
if appropriate in the presence of a diluent and if appropriate in the presence of an acid binder,
(G) compounds of the formula (I-e) shown above in which A, B, L, Q¹, Q², R⁴, R⁵, W, X, Y and Z are each as defined above,
compounds of the formula (I-a) shown above in which A, B, Q¹, Q², W, X, Y and Z are each as defined above are in each case reacted
with compounds of the formula (XI) 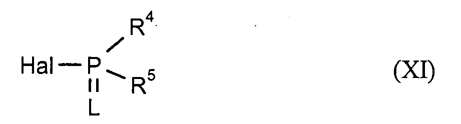 in which
L, R⁴ and R⁵ are each as defined above and
Hal represents halogen,
if appropriate in the presence of a diluent and if appropriate in the presence of an acid binder,
(H) compounds of the formula (I-f) shown above in which A, B, E, Q¹, Q², W, X, Y and Z are each as defined above,
compounds of the formula (I-a) shown above in which A, B, Q¹, Q², W, X, Y and Z are each as defined above are in each case reacted
with metal compounds or amines of the formula (XII) or (XIII)
Me(OR¹¹)ₜ (XII) 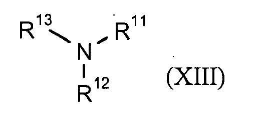
in which
Me represents a mono- or divalent metal,
t represents the number 1 or 2 and
R¹¹, R¹², R¹³ independently of one another each represents hydrogen or alkyl,
if appropriate in the presence of a diluent,
(I) compounds of the formula (I-g) shown above in which A, B, L, Q¹, Q², R⁶, R⁷, W, X, Y and Z are each as defined above,
compounds of the formula (I-a) shown above in which A, B, Q¹, Q², W, X, Y and Z are each as defined above are in each case reacted
(α) with compounds of the formula (XIV)
R⁶-N=C=L (XIV)
in which
R⁶ and L are each as defined above,
if appropriate in the presence of a diluent and if appropriate in the presence of a catalyst or
(β) with compounds of the formula (XV) 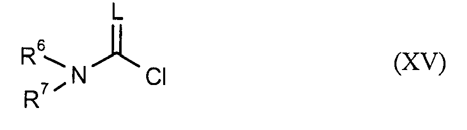 in which
L, R⁶ and R⁷ are each as defined above,
if appropriate in the presence of a diluent and if appropriate in the presence of an acid binder;
J) compounds of the formula (XVI) 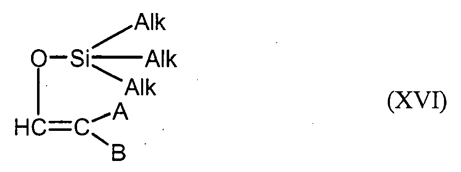 in which
A and B are each as defined above and
Alk represents alkyl having 1 to 4 carbon atoms,
are in each case reacted with compounds of the formula (XVII) 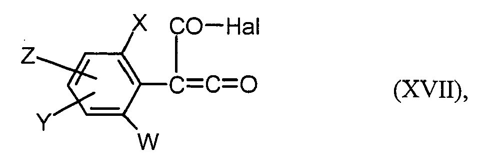 in which
W, X, Y and Z are each as defined above and
Hal represents chlorine or bromine,
if appropriate in the presence of a diluent or a diluent mixture and if appropriate in the presence of an acid binder.

7. Compounds of the formula (II) 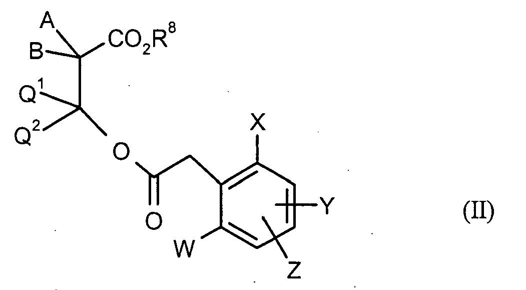 in which
A, B, Q¹, Q², W X, Y, Z and R⁸ are each as defined above.

8. Pesticides and herbicides, **characterized in that** they comprise at least one compound of the formula (I) according to Claim 1.

9. Method for controlling animal pests and undesirable vegetation, **characterized in that** compounds of the formula (I) according to Claim 1 are allowed to act on pests and/or their habitat.

10. Use of compounds of the formula (I) according to Claim 1 for controlling animal pests and undesirable vegetation.

11. Process for preparing pesticides and herbicides, **characterized in that** compounds of the formula (I) according to Claim 1 are mixed with extenders and/or surfactants.

12. Use of compounds of the formula (I) according to Claim 1 for preparing pesticides and herbicides.

## Revendications

1. Composés de formule (I) 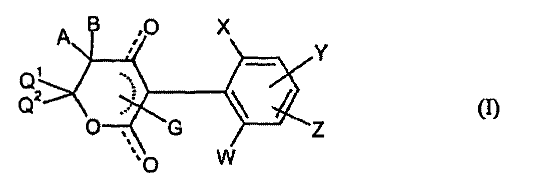 dans laquelle
W représente l'hydrogène, un reste alkyle, alcényle, alcynyle, halogéno, halogénalkyle ou alkoxy,
X représente un reste halogéno, alkyle, alkoxy, alcényle, alcynyle, halogénalkyle, halogénalkoxy, cyano, ou un reste phényle, phénoxy, phénylthio, phénylalkoxy ou phénylalkylthio, chacun éventuellement substitué,
Y représente l'hydrogène, un reste alkyle, halogéno, halogénalkyle, alkoxy, alcényle, alcynyle ou un reste aryle ou hétaryle éventuellement substitué,
Z représente l'hydrogène, un halogène, un reste alkyle, alkoxy, halogénalkyle, halogénalkoxy ou cyano,
A représente une liaison, l'hydrogène, un reste alkyle, alcényle, alkoxyalkyle, chacun éventuellement substitué par un halogène, un reste cycloalkyle éventuellement substitué ou un reste cycloalkylalkyle, dans lequel, le cas échéant, au moins un atome du noyau est remplacé par un hétéroatome, ou bien un reste aryle, arylalkyle, hétaryle ou hétarylalkyle, chacun éventuellement substitué par un radical halogéno, alkyle, halogénalkyle, alkoxy, halogénalkoxy, cyano ou nitro,
B représente l'hydrogène ou un reste alkyle, ou bien
A et B forment, conjointement avec l'atome de carbone auquel ils sont liés, un cycle saturé ou non saturé, non substitué ou substitué, contenant au moins un hétéroatome, ou bien
B et Q¹ forment ensemble un reste alcanediyle, éventuellement substitué par un radical alkyle ou alkoxy, chacun éventuellement substitué, dans lequel deux atomes de carbone non directement voisins forment, le cas échéant, un autre cycle éventuellement substitué, ou bien
Q¹ représente l'hydrogène, un reste hydroxy, alkyle, alkoxy, alkoxyalkyle, alkylacyloxy, un reste cycloalkyle éventuellement substitué (dans lequel, le cas échéant, un groupe méthylène est remplacé par de l'oxygène ou par du soufre) ou un reste phényle éventuellement substitué,
Q² représente l'hydrogène ou un reste alkyle, ou bien
Q¹ et Q² forment, conjointement avec l'atome de carbone auquel ils sont liés, un cycle non substitué ou substitué contenant éventuellement un hétéroatome,
G représente l'hydrogène (a) ou l'un des groupes 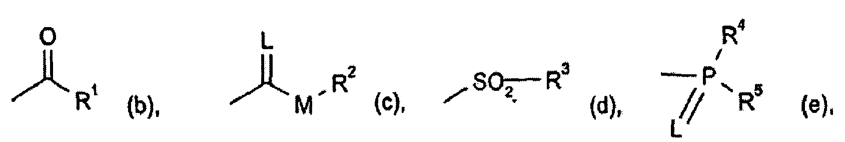 E (f) ou 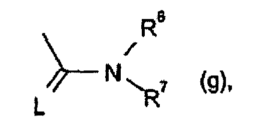 où
E désigne un ion métallique ou un ion ammonium,
L représente l'oxygène ou le soufre,
M représente l'oxygène ou le soufre,
R¹ est un reste alkyle, alcényle, alkoxyalkyle, alkylthioalkyle, polyalkoxyalkyle, chacun éventuellement substitué par un halogène, ou un reste cycloalkyle, éventuellement substitué par un radical halogéno, alkyle ou alkoxy, dans lequel un ou plusieurs groupes méthylène peuvent être remplacés par des hétéroatomes, un reste phényle, phénylalkyle, hétaryle, phénoxyalkyle ou hétaryloxyalkyle, chacun éventuellement substitué,
R² représente un reste alkyle, alcényle, alkoxyalkyle, polyalkoxyalkyle, chacun éventuellement substitué par un halogène, ou bien un reste cycloalkyle, phényle ou benzyle, chacun éventuellement substitué,
R³, R⁴ et R⁵ représentent, indépendamment les uns des autres, un reste alkyle, alkoxy, alkylamino, dialkylamino, alkylthio, alcénylthio, cycloalkylthio, chacun éventuellement substitué par un halogène, et un reste phényle, benzyle, phénoxy ou phénylthio, chacun éventuellement substitué, et
R⁶ et R⁷ représentent, indépendamment l'un de l'autre, l'hydrogène, un reste alkyle, cycloalkyle, alcényle, alkoxy, alkoxyalkyle, chacun éventuellement substitué par un halogène, un reste phényle éventuellement substitué, un reste benzyle éventuellement substitué,
ou bien ils forment, conjointement avec l'atome d'azote auquel ils sont liés, un noyau éventuellement interrompu par de l'oxygène ou par du soufre.

2. Composés de formule (I) suivant la revendication 1, formule dans laquelle
W représente l'hydrogène, un reste alkyle en C₁ à C₆, alcényle en C₂ à C₄, éthynyle, le fluor, le chlore, le brome, un reste halogénalkyle en C₁ à C₄ ou alkoxy en C₁ à C₆,
X représente le fluor, le chlore, le brome, un reste alkyle en C₁ à C₆, halogénalkyle en C₁ à C₄, alkoxy en C₁ à C₆, alcényle en C₂ à C₄, éthynyle, halogénalkoxy en C₁ à C₄, cyano ou un reste phényle ou benzyloxy, chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
Y représente l'hydrogène, un reste alkyle en C₁ à C₆, halogénalkyle en C₁ à C₄, le fluor, le chlore, le brome, un reste alkoxy en C₁ à C₆, alcényle en C₂ à C₄, éthynyle ou l'un des restes 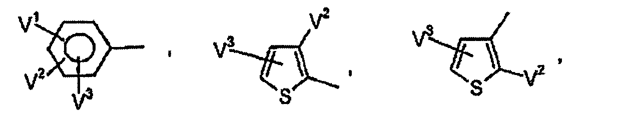 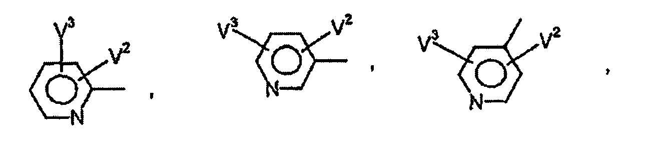
V¹ représente l'hydrogène, un halogène, un reste alkyle en C₁ à C₁₂, alkoxy en C₁ à C₆, alkylthio en C₁ à C₆, halogénalkyle en C₁ à C₄, halogénalkoxy en C₁ à C₄, nitro, cyano ou un reste phényle, phénoxy, phénoxy- (alkyle en C₁ à C₄), phényl-(alkoxy en C₁ à C₄), phénylthio-(alkyle en C₁ à C₄) ou phényl-(alkylthio en C₁ à C₄), chacun éventuellement substitué une ou plusieurs fois par un radical halogéno, alkyle en C₁ à C₆, alkoxy en C₁ à C₆, halogénalkyle en C₁ à C₄, halogénalkoxy en C₁ à C₄, nitro ou cyano,
V² représente l'hydrogène, le fluor, le chlore, un reste alkyle en C₁ à C₆, alkoxy en C₁ à C₆, halogénalkyle en C₁ à C₄ ou halogénalkoxy en C₁ à C₄,
V³ représente l'hydrogène, le fluor, le chlore, un reste méthyle ou méthoxy,
Z représente l'hydrogène, le fluor, le chlore, le brome, un reste alkyle en C₁ à C₆, halogénalkyle en C₁ à C₄, alkoxy en C₁ à C₆, halogénalkoxy en C₁ à C₄ ou cyano, sous réserve, premièrement, que W, X et Z ne représentent pas le brome, un reste alcényle, en C₂ à C₄ ou éthynyle, lorsque Y est un reste phényle ou hétaryle substitué par V¹, V² et V³, et que, deuxièmement, seul un maximal de deux des restes W, X et Y puissent représenter un reste alcényle en C₂ à C₄ ou éthynyle, à condition qu'aucun des autres restes W, X, Y et Z ne puissent représenter le brome,
A représente une liaison, l'hydrogène ou un reste alkyle en C₁ à C₁₂, alcényle en C₃ à C₈, (alkoxy en C₁ à C₆)- (alkyle en C₁ à C₄), chacun éventuellement substitué par un halogène, un reste cycloalkyle en C₃ à C₈ ou (cycloalkyle en C₃ à C₆)-(alkyle en C₁ à C₄), chacun éventuellement substitué par un radical halogéno, alkyle en C₁ à C₄ ou alkoxy en C₁ à C₄, dans lequel, le cas échéant, un ou deux chaînons du noyau non directement voisins sont remplacés par l'oxygène et/ou le soufre, ou bien un reste phényle, benzyle, hétaryle à noyau de 5 ou 6 atomes ou hétaryl-(alkyle en C₁ à C₄) à noyau de 5 ou 6 atomes, chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro,
B représente l'hydrogène ou un reste alkyle en C₁ à C₆, ou bien
A, B et l'atome de carbone auquel ils sont liés représentent un reste cycloalkyle en C₃ à C₁₀ saturé ou cycloalkyle en C₅ à C₁₀ non saturé où, le cas échéant, un chaînon du noyau est remplacé par l'oxygène ou le soufre et qui sont éventuellement substitués une ou deux fois par un radical alkyle en C₁ à C₆, cycloalkyle en C₃ à C₈, halogénalkyle en C₁ à C₆, alkoxy en C₁ à C₆, alkylthio en C₁ à C₆, halogéno ou phényle, sous réserve que Q¹ et Q² ne forment aucun autre cycle, ou bien
B et Q¹ représentent ensemble un reste alcanediyle en C₃ à C₆, éventuellement substitué une ou deux fois identiques ou différentes par un radical alkyle en C₁ à C₄, dans lequel deux atomes de carbone non directement voisins forment éventuellement un autre cycle trigonal à hexagonal, ou bien
Q¹ représente l'hydrogène, un reste hydroxy, alkyle en C₁ à C₆, alkoxy en C₁ à C₆, (alkoxy en C₁ à C₆)-(alkyle en C₁ ou C₂), (alkyle en C₁ à C₆)acyloxy, un reste cycloalkyle en C₃ à C₈, éventuellement substitué par un radical fluoro, chloro, alkyle en C₁ à C₄, halogénalkyle en C₁ ou C₂ ou alkoxy en C₁ à C₄, dans lequel, le cas échéant, un groupe méthylène est remplacé par l'oxygène ou le soufre, ou un reste phényle éventuellement substitué par un radical halogéno, alkyle en C₁ à C₄, alkoxy en C₁ à C₄, halogénalkyle en C₁ ou C₂, halogénalkoxy en C₁ ou C₂, cyano ou nitro,
Q² représente l'hydrogène ou un reste alkyle en C₁ à C₄, ou bien
Q¹ et Q² forment, conjointement avec l'atome de carbone auquel ils sont liés, un reste cycloalkyle en C₃ à C₇ éventuellement substitué par un radical alkyle en C₁ à C₆, alkoxy en C₁ à C₆ ou halogénalkyle en C₁ ou C₂, et dans lequel, le cas échéant, un chaînon du noyau est remplacé par l'oxygène ou le soufre, sous réserve que A et B ne forment aucun autre cycle,
G représente l'hydrogène (a) ou l'un des groupes 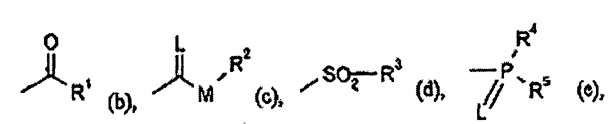 **E (f)** ou 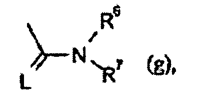 dans lesquels
E représente un ion métallique ou un ion ammonium,
L représente l'oxygène ou le soufre et
M représente l'oxygène ou le soufre,
R¹ représente un reste alkyle en C₁ à C₂₀, alcényle en C₂ à C₂₀, (alkoxy en C₁ à C₈) - (alkyle en C₁ à C₈), (alkylthio en C₁ à C₈) - (alkyle en C₁ à c₈), poly (alkoxy en C₁ à C₈) - (alkyle en C₁ à C₈), chacun éventuellement substitué par un halogène, ou un reste cycloalkyle en C₃ à C₈, éventuellement substitué par un radical halogéno, alkyle en C₁ à C₆ ou alkoxy en C₁ à C₆, dans lequel, le cas échéant, un ou plusieurs chaînons du noyau non directement voisins sont remplacés par l'oxygène et/ou le soufre, ou bien
un reste phényle éventuellement substitué par un radical halogéno, cyano, nitro, alkyle en C₁ à C₆, alkoxy en C₁ à C₆, halogénalkyle en C₁ à C₆, halogénalkoxy en C₁ à C₆, alkylthio en C₁ à C₆ ou alkylsulfonyle en C₁ à C₆, ou bien
un reste phényl-(alkyle en C₁ à C₆) éventuellement substitué par un radical halogéno, nitro, cyano, alkyle en C₁ à C₆, alkoxy en C₁ à C₆, halogénalkyle en C₁ à C₆ ou halogénalkoxy en C₁ à C₆, ou bien
un reste hétaryle pentagonal ou hexagonal éventuellement substitué par un radical halogéno, alkyle en C₁ à C₆ ou trifluorométhyle, ou bien
un reste phénoxy-(alkyle en C₁ à C₆) éventuellement substitué par un radical halogéno ou alkyle en C₁ à C₆, ou bien
un reste hétaryloxy- (alkyle en C₁ à C₆) pentagonal ou hexagonal éventuellement substitué par un radical halogéno, amino ou alkyle en C₁ à C₆,
R² représente un reste alkyle en C₁ à C₂₀, alcényle en C₂ à C₂₀, (alkoxy en C₁ à C₈)-(alkyle en C₂ à C₈), poly(alkoxy en C₁ à C₈)-(alkyle en C₂ à C₈), chacun éventuellement substitué par un halogène, ou bien
un reste cycloalkyle en C₃ à C₈ éventuellement substitué par un radical halogéno, alkyle en C₁ à C₆ ou alkoxy en C₁ à C₆, ou bien
un reste phényle ou benzyle, chacun éventuellement substitué par un radical halogéno, cyano, nitro, alkyle en C₁ à C₆, alkoxy en C₁ à C₆, halogénalkyle en C₁ à C₆ ou halogénalkoxy en C₁ à C₆,
R³ représente un reste alkyle en C₁ à C₈ éventuellement substitué par un halogène, ou un reste phényle ou benzyle, chacun éventuellement substitué par un radical halogéno, alkyle en C₁ à C₆, alkoxy en C₁ à C₆, halogénalkyle en C₁ à C₄, halogénalkoxy en C₁ à C₄, cyano ou nitro,
R⁴ et R⁵ représentent, indépendamment l'un de l'autre, un reste alkyle en C₁ à C₈, alkoxy en C₁ à C₈, alkylamino en C₁ à C₈, di(alkyle en C₁ à C₈)amino, alkylthio en C₁ à C₈, alcénylthio en C₂ à C₈, cycloalkylthio en C₃ à C₇, chacun éventuellement substitué par un halogène, ou un reste phényle, benzyle, phénoxy ou phénylthio, chacun éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄,
R⁶ et R⁷ représentent, indépendamment l'un de l'autre, l'hydrogène, un reste alkyle en C₁ à C₈, cycloalkyle en C₃ à C₈, alkoxy en C₁ à C₈, alcényle en C₃ à C₈, (alkoxy en C₁ à C₈)-(alkyle en C₁ à C₈), chacun éventuellement substitué par un halogène, un reste phényle éventuellement substitué par un radical halogéno, halogénalkyle en C₁ à C₈, alkyle en C₁ à C₈ ou alkoxy en C₁ à C₈, un reste benzyle éventuellement substitué par un radical halogéno, alkyle en C₁ à C₈, halogénalkyle en C₁ à C₈ ou alkoxy en C₁ à C₈, ou forment ensemble un reste alkylène en C₃ à C₆ éventuellement substitué par un radical alkyle en C₁ à C₄, dans lequel, le cas échéant, un atome de carbone est remplacé par l'oxygène ou le soufre.

3. Composés de formule (I) suivant la revendication 1, formule dans laquelle
W représente l'hydrogène, un reste alkyle en C₁ à C₄, le chlore ou le brome,
X représente le chlore, le brome, un reste alkyle en C₁ à C₄, alkoxy en C₁ à C₄, alcényle en C₂ ou C₃, éthynyle, halogénalkyle en C₁ ou C₂, halogénalkoxy en C₁ ou C₂ ou cyano,
Y représente l'hydrogène, un reste alkyle en C₁ à C₄, halogénalkyle en C₁ ou C₂, le fluor, le chlore, le brome, un reste alkoxy en C₁ à C₄, alcényle en C₂ ou C₃, éthynyle, 2-thiényle, 3-thiényle ou le reste 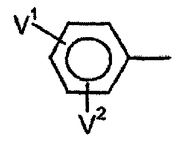
V¹ représente l'hydrogène, le fluor, le chlore, le brome, un reste alkyle en C₁ à C₆, alkoxy en C₁ à C₄, alkylthio en C₁ à C₄, halogénalkyle en C₁ ou C₂, halogénalkoxy en C₁ ou C₂, nitro, cyano ou phényle,
V² représente l'hydrogène, le fluor, le chlore, un reste alkyle en C₁ à C₄, alkoxy en C₁ à C₄ ou halogénalkyle en C₁ ou C₂,
Z représente l'hydrogène, le fluor, le chlore, le brome, un reste alkyle en C₁ à C₄, halogénalkyle en C₁ ou C₂, alkoxy en C₁ à C₄ ou halogénalkoxy en C₁ ou C₂,
sous réserve, premièrement, que W, X et Z ne représentent pas le brome, un reste alcényle en C₂ ou C₃ ou un reste éthynyle lorsque Y est un reste phényle, 2-thiényle ou 3-thiényle substitué par V¹ et V² et, deuxièmement, qu'un seul des restes X et Y puisse représenter un reste alcényle en C₂ ou C₃ ou éthynyle, à condition qu'aucun des autres restes W, X, Y et Z ne puissent alors représenter le brome,
A représente une liaison, l'hydrogène, un reste alkyle en C₁ à C₈, (alkoxy en C₁ à C₈)-(alkyle en C₁ ou C₂), chacun éventuellement substitué par du fluor, un reste cycloalkyle en C₅ ou C₆ ou (cycloalkyle en C₃ à C₆) - (alkyle en C₁ ou C₂), chacun éventuellement substitué par un radical fluoro, chloro, méthyle, éthyle ou méthoxy et dans lequel, le cas échéant, un chaînon du noyau est remplacé par l'oxygène ou le soufre, ou bien un reste phényle ou benzyle, chacun éventuellement substitué par un radical fluoro, chloro, bromo, alkyle en C₁ à C₄, halogénalkyle en C₁ ou C₂, alkoxy en C₁ à C₄ ou halogénalkoxy en C₁ ou C₂,
B représente l'hydrogène ou un reste alkyle en C₁ à C₄, ou bien
A, B et l'atome de carbone auquel ils sont liés forment un reste cycloalkyle saturé en C₅ à C₇ dans lequel, le cas échéant, un chaînon du noyau est remplacé par l'oxygène et qui est éventuellement substitué une fois par un radical alkyle en C₁ à C₄, trifluorométhyle ou alkoxy en C₁ à C₄, sous réserve que Q¹ et Q² ne puissent former aucun autre cycle, ou bien
B et Q¹ représentent ensemble un reste alcanediyle en C₃ ou C₄, éventuellement substitué une fois par un radical alkyle en C₁ ou C₂, dans lequel deux atomes de carbone non directement voisins forment éventuellement un autre cycle pentagonal ou hexagonal, ou bien
Q¹ représente l'hydrogène, un reste hydroxy, alkyle en C₁ à C₄, alkoxy en C₁ à C₄, (alkoxy en C₁ à C₄)-(alkyle en C₁ ou C₂), (alkyle en C₁ à C₄)acyloxy ou un reste cycloalkyle en C₃ à C₆, éventuellement substitué par un radical méthyle ou méthoxy, dans lequel, le cas échéant, un groupe méthylène est remplacé par l'oxygène,
Q² représente l'hydrogène, un reste méthyle ou éthyle, ou bien
Q¹ et Q² forment, conjointement avec l'atome de carbone auquel ils sont liés, un reste cycloalkyle en C₅ ou C₆ éventuellement substitué par un radical alkyle en C₁ à C₄, trifluorométhyle ou alkoxy en C₁ à C₄, dans lequel, le cas échéant, un chaînon du noyau est remplacé par l'oxygène, sous réserve que A et B ne forment aucun autre cycle,
G représente l'hydrogène (a) ou l'un des groupes 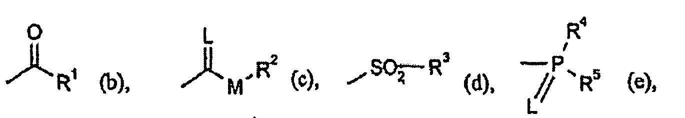 **E (f)** ou 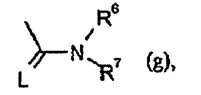 dans lesquels
E représente un ion métallique ou un ion ammonium,
L représente l'oxygène ou le soufre, et
M représente l'oxygène ou le soufre,
R¹ représente un reste alkyle en C₁ à C₁₆, alcényle en C₂ à C₁₆, (alkoxy en C₁ à C₄) - (alkyle en C₁ ou C₂), (alkylthio en C₁ à C₄) - (alkyle en C₁ ou C₂), chacun éventuellement substitué par du fluor ou par du chlore, ou bien un reste cycloalkyle en C₃ à C₇ éventuellement substitué par un radical fluoro, chloro, alkyle en C₁ ou C₂ ou alkoxy en C₁ ou C₂, dans lequel, le cas échéant, un ou deux chaînons non directement voisins sont remplacés par l'oxygène et/ou le soufre, ou bien
un reste phényle éventuellement substitué une ou deux fois par un radical fluoro, chloro, bromo, cyano, nitro, alkyle en C₁ à C₄, alkoxy en C₁ à C₄, trifluorométhyle ou trifluorométhoxy, ou bien
un reste pyridyle ou thiényle, chacun éventuellement substitué une fois par un radical fluoro, chloro, bromo, méthyle, éthyle ou trifluorométhyle,
R² représente un reste alkyle en C₁ à C₁₆, alcényle en C₂ à C₁₆ ou (alkoxy en C₁ à C₄)-(alkyle en C₂ à C₄), chacun éventuellement substitué une à trois fois par du fluor, ou bien
un reste cycloalkyle en C₃ à C₇ éventuellement substitué une fois par un radical méthyle, éthyle ou méthoxy, ou bien
un reste phényle ou benzyle éventuellement substitué une ou deux fois par un radical fluoro, chloro, bromo, cyano, nitro, alkyle en C₁ à C₄, alkoxy en C₁ à C₃, trifluorométhyle ou trifluorométhoxy,
R³ représente un reste alkyle en C₁ à C₆ éventuellement substitué une à cinq fois par du fluor, ou un reste phényle éventuellement substitué une ou deux fois par un radical fluoro, chloro, bromo, alkyle en C₁ à C₄, alkoxy en C₁ à C₄, trifluorométhyle, trifluorométhoxy, cyano ou nitro,
R⁴ représente un reste alkyle en C₁ à C₆, alkoxy en C₁ à C₆, alkylamino en C₁ à C₆, di(alkyle en C₁ à C₆)amino, alkylthio en C₁ à C₆, ou un reste phényle, benzyle, phénoxy ou phénylthio, chacun éventuellement substitué une ou deux fois par un radical fluoro, chloro, bromo, nitro, cyano, alkoxy en C₁ à C₃, trifluorométhoxy, alkyle en C₁ à C₃ ou trifluorométhyle,
R⁵ est un reste alkyle en C₁ à C₄, alkoxy en C₁ à C₄ ou alkylthio en C₁ à C₄,
R⁶ est un reste alkyle en C₁ à C₆, cycloalkyle en C₃ à C₆, alkoxy en C₁ à C₆, alcényle en C₃ à C₆, (alkoxy en C₁ à C₆) - (alkyle en C₁ à C₆), ou un reste phényle éventuellement substitué une ou deux fois par un radical fluoro, chloro, bromo, trifluorométhyle, alkyle en C₁ à C₄ ou alkoxy en C₁ à C₄, ou bien un reste benzyle éventuellement substitué une ou deux fois par un radical fluoro, chloro, bromo, méthyle, éthyle, trifluorométhyle ou méthoxy,
R⁷ représente l'hydrogène, un reste alkyle en C₁ à C₆ ou alcényle en C₃ à C₆, ou bien
R⁶ et R⁷ forment ensemble un reste alkylène en C₄ ou C₅ éventuellement substitué une ou deux fois par un radical méthyle ou éthyle, dans lequel, le cas échéant, un groupe méthylène est remplacé par l'oxygène ou le soufre.

4. Composés de formule (I) suivant la revendication 1, formule dans laquelle
W représente l'hydrogène, le chlore, le brome, un reste méthyle ou éthyle,
X représente le chlore, le brome, un reste méthyle, éthyle, n-propyle, méthoxy, éthoxy, trifluorométhyle, difluorométhoxy, trifluorométhoxy ou cyano,
Y représente l'hydrogène, un reste méthyle, éthyle, propyle, isopropyle, le fluor, le chlore, le brome, un reste méthoxy, ou le reste 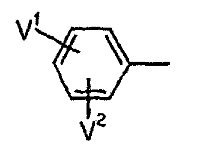
V¹ représente l'hydrogène, le fluor, le chlore, le brome, un reste méthyle, éthyle, isopropyle, tertiobutyle, méthoxy, trifluorométhyle, trifluorométhoxy, cyano ou phényle,
V² représente l'hydrogène, le fluor, le chlore, un reste méthyle, méthoxy ou trifluorométhyle,
Z représente l'hydrogène, le fluor, le chlore, le brome, un reste méthyle, méthoxy ou trifluorométhyle, sous réserve que W, X et Z ne représentent pas le brome lorsque Y est un reste phényle substitué par V¹ et V²,
A représente une liaison, l'hydrogène, un reste méthyle, éthyle, n-propyle, isopropyle, n-butyle ou isobutyle,
B représente l'hydrogène, un reste méthyle ou éthyle, ou bien
A, B et l'atome de carbone auquel ils sont liés forment un reste cycloalkyle saturé en C₅ ou C₆ dans lequel, le cas échéant, un chaînon du noyau est remplacé par l'oxygène et qui est éventuellement substitué une fois par un radical méthyle, éthyle, méthoxy ou éthoxy, sous réserve que Q¹ et Q² ne forment aucun autre cycle, ou bien
B et Q¹ forment ensemble un reste alcanediyle en C₃ ou C₄ éventuellement substitué une fois par un radical méthyle, dans lequel deux atomes de carbone non directement voisins forment éventuellement un autre cycle trigonal à hexagonal, ou bien
Q¹ représente l'hydrogène, un reste hydroxy, méthyle, éthyle, n-propyle, isopropyle, méthoxy, éthoxy, propoxy, acétyloxy ou propionyloxy,
Q² représente l'hydrogène, un reste méthyle ou éthyle, ou bien
Q¹ et Q² forment, conjointement avec l'atome de carbone auquel ils sont liés, un reste cycloalkyle en C₆ éventuellement substitué par un radical méthyle, éthyle, méthoxy, éthoxy, propoxy ou butoxy, dans lequel, le cas échéant, un chaînon du noyau est remplacé par l'oxygène, sous réserve que A et B ne forment aucun autre cycle,
G représente l'hydrogène (a) ou l'un des groupes 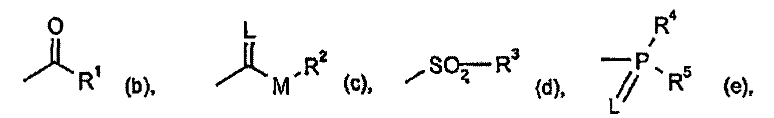 **E (f)** ou 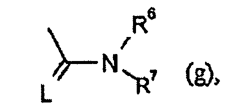 dans lesquels
E représente un ion métallique ou un ion ammonium,
L représente l'oxygène ou le soufre et
M représente l'oxygène ou le soufre,
R¹ représente un reste alkyle en C₁ à C₈, alcényle en C₂ à C₈, (alkoxy en C₁ ou C₂)-(alkyle en C₁), (alkylthio en C₁)-(alkyle en C₁), cyclopropyle, cyclopentyle ou cyclohexyle, chacun éventuellement substitué par du fluor ou par du chlore, ou bien
un reste phényle éventuellement substitué une fois par du fluor, du chlore, du brome, un groupe cyano, nitro, un reste méthyle, éthyle, isopropyle, tertiobutyle, méthoxy, trifluorométhyle ou trifluorométhoxy, ou bien un reste thiényle ou pyridyle, chacun éventuellement substitué par un radical chloro, bromo ou méthyle,
R² représente un reste alkyle en C₁ à C₈, alcényle en C₂ à C₈ ou (alkoxy en C₁ à C₄) - (alkyle en C₂) ou un reste cyclohexyle,
ou bien un reste phényle ou benzyle, chacun éventuellement substitué une fois par un radical fluoro, bromo, chloro, cyano, nitro, méthyle, tertiobutyle, méthoxy, trifluorométhyle ou trifluorométhoxy,
R³ représente un reste méthyle, éthyle, n-propyle ou un reste phényle éventuellement substitué une fois par un radical fluoro, chloro, bromo, méthyle, tertiobutyle, méthoxy, trifluorométhyle, trifluorométhoxy, cyano ou nitro,
R⁴ représente un reste alkyle en C₁ à C₄, alkoxy en C₁ à C₄, alkylamino en C₁ à C₄, di(alkyle en C₁ à C₄) amino, alkylthio en C₁ à C₄ ou un reste phényle, phénoxy ou phénylthio, chacun éventuellement substitué une fois par un radical fluoro, chloro, bromo, nitro, cyano, alkoxy en C₁ ou C₂, trifluorométhoxy ou alkyle en C₁ à C₃,
R⁵ représente un reste méthyle, éthyle, méthoxy, éthoxy, méthylthio ou éthylthio,
R⁶ représente un reste alkyle en C₁ à C₄, cycloalkyle en C₃ à C₆, alkoxy en C₁ à C₄, alcényle en C₃ ou C₄, (alkoxy en C₁ à C₄) - (alkyle en C₁ à C₄),
R⁷ représente l'hydrogène, un reste alkyle en C₁ à C₄ ou alcényle en C₃ ou C₄, ou bien
R⁶ et R⁷ forment ensemble un reste alkylène en C₆ dans lequel, le cas échéant, un groupe méthylène est remplacé par l'oxygène ou par le soufre.

5. Composés de formule (I) suivant la revendication 1, formule dans laquelle
W représente l'hydrogène, un reste méthyle, éthyle, le chlore ou le brome,
X représente un reste méthyle, éthyle, n-propyle, trifluorométhyle ou le chlore,
Y représente un reste méthyle, trifluorométhyle, le chlore, le brome, un reste phényle éventuellement substitué une ou deux fois par un radical chloro et/ou méthyle,
Z représente l'hydrogène ou un reste méthyle, sous réserve que W ne représente pas le brome lorsque Y est un reste phényle substitué,
A représente un reste méthyle, éthyle ou une liaison,
B est un reste méthyle ou éthyle, ou bien
A et B et l'atome de carbone auquel ils sont liés forment un reste cyclopropyle ou cyclohexyle, ou bien
B et Q¹ forment ensemble un reste alcanediyle en C₄ éventuellement substitué une fois par un radical méthyle,
Q¹ représente l'hydrogène, un reste méthyle, méthoxy, éthoxy, propoxy, hydroxy ou acétyloxy,
Q² représente l'hydrogène ou un reste méthyle,
G représente l'hydrogène (a) ou l'un des groupes 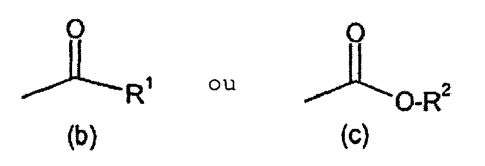 où
R¹ est un reste alkyle en C₁ à C₄ ou un reste phényle ou pyridyle, chacun éventuellement substitué une fois par du chlore,
R² représente un reste alkyle en C₁ à C₄.

6. Procédé de production de composés de formule (I) suivant la revendication 1, **caractérisé en ce que**, pour l'obtention
(A) de composés de formule (Ia) 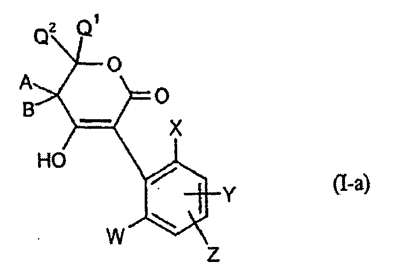 dans laquelle
A, B, Q¹, Q², W, X, Y et Z ont les définitions indiquées ci-dessus,
des composés de formule (II) 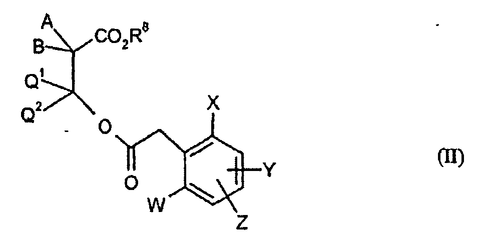 dans laquelle
A, B, Q¹, Q², W, X, Y et Z ont les définitions indiquées ci-dessus,
et
R⁸ est un reste alkyle,
sont soumis à une condensation intramoléculaire en présence d'un diluant et en présence d'une base ;
(B) de composés de formules (I-a) à (I-g) : 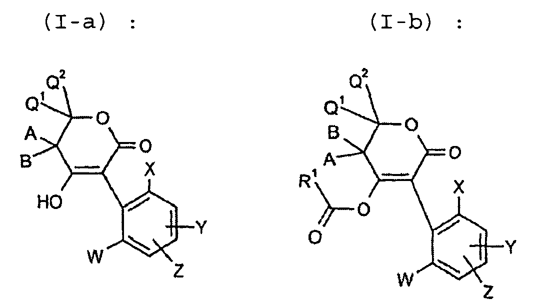 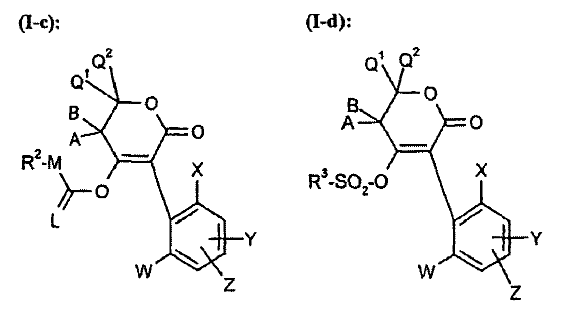 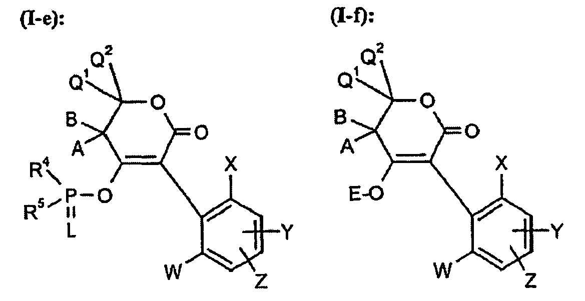 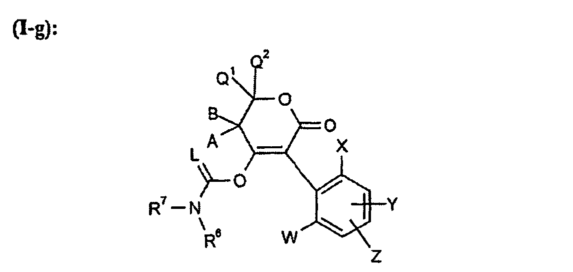 dans lesquelles A, B, G, Q¹, Q², W, X, Y et Z ont les définitions indiquées ci-dessus,
des composés de formules (I-a') à (I-g') : 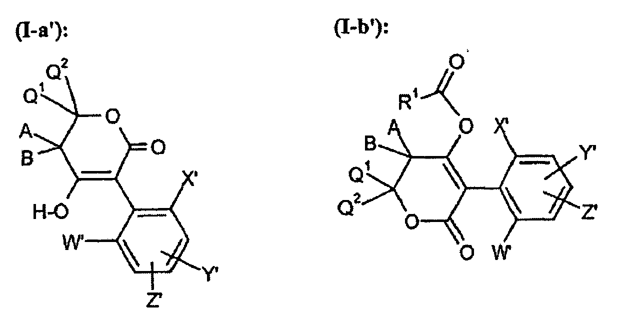 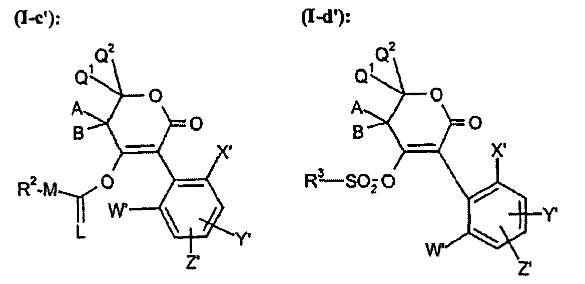 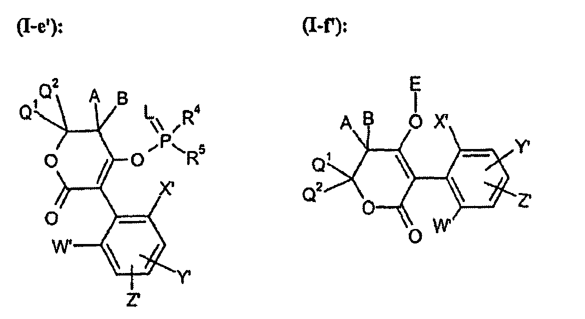 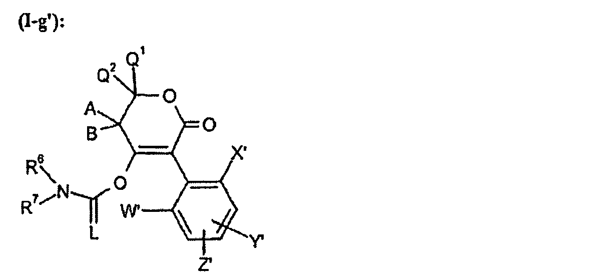 dans lesquelles
A, B, R¹, R², R³, R⁴, R⁵, R⁶, R⁷, E, L, M, Q¹, Q², W', X', Y' et Z' ont la définition indiquée pour W, X, Y et Z ci-dessus, et l'un au moins des restes
W', X', Y' représentent le chlore, le brome ou l'iode, et Z' ne représente pas le brome ou l'iode,
α) sont amenés à réagir avec des composés de formule (III) 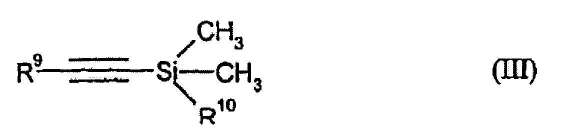 dans laquelle
R⁹ représente l'hydrogène et
R¹⁰ est un reste alkyle en C₁ à C₄ ou phényle,
tout d'abord en présence d'un solvant, d'une base et d'un catalyseur, puis le groupe silyle est éliminé,
ou bien
β) sont amenés à réagir avec des composés de formule (IV) : 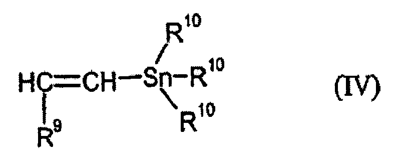 dans laquelle
R⁹ représente l'hydrogène, un reste méthyle ou éthyle, et
R¹⁰ est un reste alkyle en C₁ à C₄,
en présence d'un solvant, éventuellement en présence d'une base et, le cas échéant, en présence d'un catalyseur,
ou bien
γ) dans le cas spécial où Y' représente le chlore, le brome ou l'iode et W', X' et Z' ne représentent pas le brome ou l'iode, sont amenés à réagir avec des acides boroniques de formule (V) 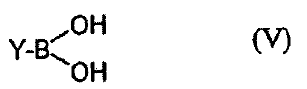 dans laquelle
Y est un reste phényle ou hétaryle éventuellement substitué,
en présence d'un solvant, d'une base et d'un catalyseur ;
(C) de composés de formule générale (I-b) indiquée ci-dessus dans laquelle A, B, Q¹, Q², R¹, W, X, Y et Z ont les définitions indiquées ci-dessus,
des composés de formule (I-a) indiquée ci-dessus, dans lesquels A, B, Q¹, Q², W, X, Y et Z ont les définitions indiquées ci-dessus,
sont amenés à réagir dans chaque cas,
(α) avec des composés de formule (VI) 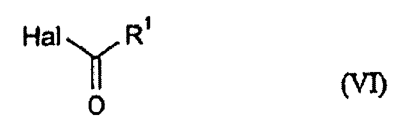 dans laquelle
R¹ a la définition indiquée ci-dessus et
Hal représente un halogène
ou bien
(β) avec des composés de formule (VII)
**R¹-CO-O-CO-R¹** **(VII)**
dans laquelle
R¹ a la définition indiquée ci-dessus,
éventuellement en présence d'un diluant et, le cas échéant, en présence d'un accepteur d'acide ;
(D) de composés de formule (I-c) indiquée ci-dessus dans laquelle A, B, Q¹, Q², R², M, W, X, Y et Z ont les définitions indiquées ci-dessus et L représente l'oxygène,
des composés de formule (I-a) indiquée ci-dessus dans laquelle A, B, Q¹, Q², W, X, Y et Z ont les définitions indiquées ci-dessus, sont amenés à réagir dans chaque cas avec des composés de formule (VIII)
**R²-M-CO-Cl** **(VIII)**
dans laquelle
R² et M ont les définitions indiquées ci-dessus,
éventuellement en présence d'un diluant et, le cas échéant, en présence d'un accepteur d'acide ;
(E) de composés de formule (I-c) indiquée ci-dessus dans laquelle A, B, Q¹, Q², R², M, W, X, Y et Z ont les définitions indiquées ci-dessus et L représente le soufre,
des composés de formule (I-a) indiquée ci-dessus dans laquelle A, B, Q¹, Q², W, X, Y et Z ont les définitions indiquées ci-dessus, sont amenés à réagir dans chaque cas avec des composés de formule (IX) 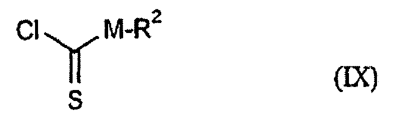 dans laquelle
M et R² ont les définitions indiquées ci-dessus,
éventuellement en présence d'un diluant et, le cas échéant, en présence d'un accepteur d'acide, et
pour l'obtention
(F) de composés de formule (I-d) indiquée ci-dessus dans laquelle A, B, Q¹, Q², R³, W, X, Y et Z ont les définitions indiquées ci-dessus,
des composés de formule (I-a) indiquée ci-dessus dans laquelle A, B, Q¹, Q², W, X, Y et Z ont les définitions indiquées ci-dessus, sont amenés à réagir dans chaque cas avec des composés de formule (X)
**R³-SO₂-Cl** **(X)**
dans laquelle
R³ a la définition indiquée ci-dessus,
éventuellement en présence d'un diluant et, le cas échéant, en présence d'un accepteur d'acide,
(G) de composés de formule (I-e) indiquée ci-dessus dans laquelle A, B, L, Q¹, Q², R⁴, R⁵, W, X, Y et Z ont les définitions indiquées ci-dessus,
des composés de formule (I-a) indiquée ci-dessus dans laquelle A, B, Q¹, Q², W, X, Y et Z ont les définitions indiquées ci-dessus, sont amenés à réagir dans chaque cas avec des composés de formule (XI) 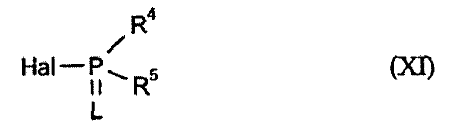 dans laquelle
L, R⁴ et R⁵ ont les définitions indiquées ci-dessus et
Hal représente un halogène,
éventuellement en présence d'un diluant et, le cas échéant, en présence d'un accepteur d'acide,
(H) de composés de formule (I-f) indiquée ci-dessus dans laquelle A, B, E, Q¹, Q², W, X, Y et Z ont les définitions indiquées ci-dessus,
des composés de formule (I-a) dans lesquels A, B, Q¹, Q², W, X, Y et Z ont les définitions indiquées ci-dessus,
sont amenés à réagir dans chaque cas avec des composés métalliques ou avec des amines de formule (XII) ou (XIII)
**Me(OR¹¹)ₜ** **(XII)** 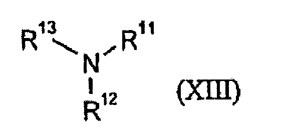
dans lesquelles
Me est un métal monovalent ou divalent,
t représente le nombre 1 ou 2 et
R¹¹, R¹², R¹³ représentent, indépendamment les uns des autres, l'hydrogène ou un reste alkyle,
le cas échéant, en présence d'un diluant,
(I) de composés de formule (I-g) indiquée ci-dessus, dans laquelle, A, B, L, Q¹, Q², R⁶, R⁷, W, X, Y et Z ont les définitions indiquées ci-dessus,
des composés de formule (I-a) indiquée ci-dessus, dans laquelle A, B, Q¹, Q², W, X, Y et Z ont les définitions indiquées ci-dessus, sont amenés à réagir dans chaque cas
(α) avec des composés de formule (XIV)
**R⁶-N=C=L** **(XIV)**
dans laquelle
R⁶ et L ont les définitions indiquées ci-dessus,
éventuellement en présence d'un diluant et, le cas échéant, en présence d'un catalyseur, ou bien
(β) avec des composés de formule (XV) 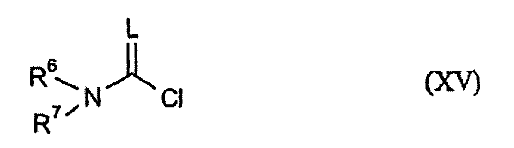
dans laquelle
L, R⁶ et R⁷ ont les définitions indiquées ci-dessus,
éventuellement en présence d'un diluant et, le cas échéant, en présence d'un accepteur d'acide ;
J) des composés de formule (XVI) 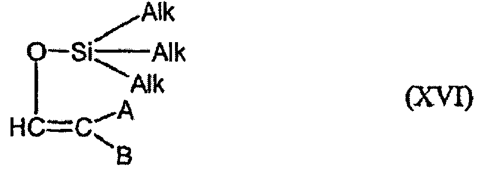 dans laquelle
A et B ont les définitions indiquées ci-dessus et Alk est un reste alkyle ayant 1 à 4 atomes de carbone, sont amenés à réagir dans chaque cas avec des composés de formule (XVII) 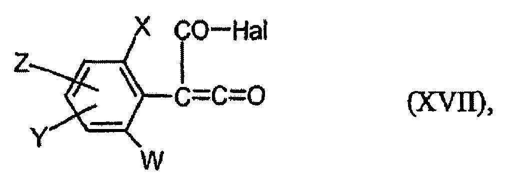 dans laquelle
W, X, Y et Z ont les définitions indiquées ci-dessus et Hal représente le chlore ou le brome,
éventuellement en présence d'un diluant ou d'un mélange de diluants et, le cas échéant, en présence d'un accepteur d'acide.

7. Composés de formule (II) 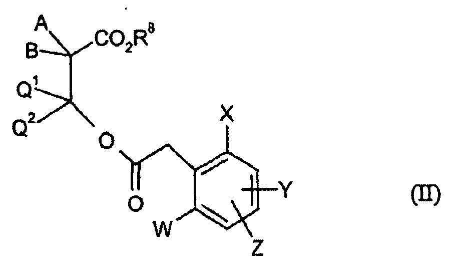 dans laquelle
A, B, Q¹, Q², W, X, Y, Z et R⁸ ont les définitions indiquées ci-dessus.

8. Pesticides et herbicides, **caractérisés par** une teneur en au moins un composé de formule (I) suivant la revendication 1.

9. Procédé pour combattre des parasites animaux et une végétation non souhaitée, **caractérisé en ce qu'**on fait agir des composés de formule (I) suivant la revendication 1 sur les parasites et/ou sur leur milieu.

10. Utilisation de composés de formule (I) suivant la revendication 1, pour combattre des parasites animaux et une végétation non souhaitée.

11. Procédé de préparation de pesticides et d'herbicides, **caractérisé en ce qu'**on mélange des composés de formule (I) suivant la revendication 1 avec des diluants et/ou des agents tensioactifs.

12. Utilisation de composés de formule (I) suivant la revendication 1, pour la préparation de pesticides et d'herbicides.
